# EUROPEAN PATENT APPLICATION

(11) **EP 4 238 990 A1**
(43) Date of publication of application: **06.09.2023**
(21) Application number: 21885370.3
(22) Date of filing: 01.11.2021
(51) Int. Cl.: C07K 16/28, C07K 16/30, C12N 5/10, C12N 15/13, C12N 15/62, A61K 39/395, A61P 35/00, A61P 35/02

(54) **FULLY HUMAN ANTIBODY TARGETING CD5, AND FULLY HUMAN CHIMERIC ANTIGEN RECEPTOR (CAR) AND APPLICATION THEREOF**

(30) Priority: 01.11.2020 CN 202011200628
(71) Applicant: Nanjing Iaso Biotechnology Co., Ltd., Nanjing, Jiangsu 210061 (CN)
(72) Inventor: TAN, Taochao, Nanjing, Jiangsu 210061 (CN); DAI, Zhenyu, Nanjing, Jiangsu 210061 (CN); ZHOU, Jianfeng, Nanjing, Jiangsu 210061 (CN); WEI, Qiaoe, Nanjing, Jiangsu 210061 (CN); JIA, Xiangyin, Nanjing, Jiangsu 210061 (CN); ZHAO, Ya, Nanjing, Jiangsu 210061 (CN); XIE, Meng, Nanjing, Jiangsu 210061 (CN)
(74) Representative: Symbiosis IP Limited
(86) International application number: PCT/CN2021/127995
(87) International publication number: WO 2022/089644

(57) **Abstract**

The present application provides an anti-CD5 antibody. The present application further provides a chimeric antigen receptor (CAR) that specifically binds to a CD5 protein, which comprises a CD5 binding domain, a transmembrane domain, a co-stimulatory domain and an intracellular signaling domain. Further provided are engineered immune effector cells (such as T-cells) comprising the chimeric antigen receptor. The present application further provides use of the CAR and the engineered immune effector cells in the treatment of diseases or conditions associated with the expression of CD5.

## Description

### Related Application

The present application claims priority to the Chinese patent application No. 202011200628.8 filed on November 1, 2020 in the CNIPA, which is incorporated herein by reference in its entirety.

### Sequence Listing

The present application includes a sequence listing in .txt format submitted electronically. This .txt file contains a sequence listing named "CD5 CAR_ST25.txt" created on November 1, 2020, and is approximately 126 kilobytes in size. The sequence listing contained in the .txt file is part of the specification and is incorporated herein by reference in its entirety.

### FIELD OF THE INVENTION

The present application relates to the field of biomedicine, in particular to a fully human antibody capable of specifically binding to CD5 protein, a fully human chimeric antigen receptor and applications thereof.

### BACKGROUND OF THE INVENTION

In recent years, chimeric antigen receptor T-cell (CAR-T) technology has made breakthroughs, especially great success has been made in targets for B-cell malignancies. As represented by anti-CD19 CAR-T, so far, three products, Kymriah, Yescarta and Tecartus, have been approved by the FDA (US) for marketing^{[1]}. Clinical trials have confirmed that CAR-T cells specifically targeting CD19 molecules can effectively target and treat B-cell malignancies, including relapsed/refractory B-ALL, CLL, and B-cell lymphoma^{[2-9]}. According to literature reports, the effective rate of CD19 CAR-T in treatment of relapsed/refractory ALL can be as high as 90%, and the effective rate for CLL and some B-cell lymphomas is greater than 50%. Although CAR-T therapy has achieved great success in the field of treatment of B-cell malignancies, research on CAR-T therapy and application thereof in T-cell malignancies are very limited.

T-cell malignancies include acute T-lymphocytic leukemia (T-ALL) and T-cell lymphoma (TCL). T-ALL is a blood disease caused by abnormal proliferation of T lymphocytes, which is aggressive and progresses rapidly. T-cell lymphoma is a malignancy of T-cells that can develop in lymphoid tissues (such as lymph nodes and spleen) or outside lymphoid tissues (such as gastrointestinal tract, liver, nasal cavity, and skin), it accounts for about 10-15% of non-Hodgkin's lymphoma, and this proportion is higher in China. CD5 is constitutively expressed on lymphocyte precursors, mature T-cells and a portion of mature B-cells (B1 cells)^{[10] [11]}. CD5 is highly expressed in about 85% of T-ALLs and about 75% of peripheral T-cell lymphomas. In addition, CD5 is also frequently expressed in mantle cell lymphoma, chronic lymphocytic leukemia (B-CLL) and hairy cell leukemia cells (HCL). At present, T-cell malignancies have a high recurrence rate and poor prognosis after radiotherapy and chemotherapy. They are clinically intractable hematological malignancies, and there is an urgent need to develop cell therapy drugs for this disease. In normal cells, the expression of CD5 is limited to mature T-cells and some subtypes of B-cells, and the biological characteristics of CD5 antigen allow CD5 CAR T cells to produce effective anti-tumor activity against T-ALL and T lymphoma cells in vitro and in vivo^{[12]}. Therefore, CD5 can serve as a safe and reliable target for T-cell tumors.

Researchers such as Mamonkin M developed a second-generation CD5 CAR using the scFv of murine monoclonal antibody H65. The results of clinical trials show that the CD5 CAR-T cells are safe in patients with r/r CD5+T-ALL and T-cell non-Hodgkin's lymphoma (T-NHL) who have been treated by multiple lines in the past, and can produce clinical efficacy without completely eliminating T-cells. More importantly, depletion of malignant T-cells by CD5 CAR-T cells may allow patients who were previously ineligible for transplantation to undergo HSCT (hematopoietic stem cell transplantation). However, according to the existing clinical follow-up data, the murine scFv CAR-T may suffer relapse of CD5+ malignant diseases after infusion, and the limited duration of CAR-T in patients may be related to the production of anti-murine antibodies, while fully human scFv may be able to solve the problem of short duration of CD5 CAR-T in patients. The development of a fully human CD5 antibody is of great significance for the development of the next generation of CAR-T products that last longer in vivo and have better long-term efficacy.

### SUMMARY OF THE INVENTION

The present application provides a fully human antibody that can specifically bind to CD5, a fully human chimeric antigen receptor and applications thereof.

In one aspect, the present application provides a CD5-targeting fully human antibody or a single chain antibody or fragment thereof, wherein the fully human antibody comprises a heavy chain variable region (HCVR) and/or a light chain variable region (LCVR), and the heavy chain variable region comprises HCDR1, HCDR2 and HCDR3, the light chain variable region comprises LCDR1, LCDR2 and LCDR3, and the HCDR1, HCDR2, HCDR3 and/or LCDR1, LCDR2 and LCDR3 are selected from one of the following combinations:
(1) LCDR1 having an amino acid sequence of SGSIARNY (SEQ ID NO: 64);
   LCDR2 having an amino acid sequence of EDN (SEQ ID NO: 65);
   LCDR3 having an amino acid sequence of QSYDDNTSWV (SEQ ID NO: 66);
   HCDR1 having an amino acid sequence of GGTFSSNA (SEQ ID NO: 61);
   HCDR2 having an amino acid sequence of IIPMFGTT (SEQ ID NO: 62);
   HCDR3 having an amino acid sequence of ARDGGGRHPYYYYGMDV (SEQ ID NO: 63);
(2) LCDR1 having an amino acid sequence of SSNIGNND (SEQ ID NO: 71);
   LCDR2 having an amino acid sequence of DND (SEQ ID NO: 72);
   LCDR3 having an amino acid sequence of AVWDSSLSAAV (SEQ ID NO: 73);
   HCDR1 having an amino acid sequence of GYSFTSYW (SEQ ID NO: 67);
   HCDR2 having an amino acid sequence of IYPDDSDT (SEQ ID NO: 68);
   HCDR3 having an amino acid sequence of ARMSLTSYLATDG (SEQ ID NO: 69);
(3) LCDR1 having an amino acid sequence of SDDIGNYKR (SEQ ID NO: 76);
   LCDR2 having an amino acid sequence of EVT (SEQ ID NO: 77);
   LCDR3 having an amino acid sequence of SSYASGDTYV (SEQ ID NO: 78);
   HCDR1 having an amino acid sequence of TYTFTNYY (SEQ ID NO: 73);
   HCDR2 having an amino acid sequence of INPSGDTT (SEQ ID NO: 74);
   HCDR3 having an amino acid sequence of ARLSWYWGGSFDD (SEQ ID NO: 75);
(4) LCDR1 having an amino acid sequence of SSNIGAGYD (SEQ ID NO: 46);
   LCDR2 having an amino acid sequence of GNI (SEQ ID NO: 47);
   LCDR3 having an amino acid sequence of GTWDNSLSAHYV (SEQ ID NO: 48);
   HCDR1 having an amino acid sequence of GFTFNNYT (SEQ ID NO: 43);
   HCDR2 having an amino acid sequence of ISSSSSYI (SEQ ID NO: 44);
   HCDR3 having an amino acid sequence of ARYFSGSAGDY (SEQ ID NO: 45);
(5) LCDR1 having an amino acid sequence of NSNIGNNY (SEQ ID NO: 52);
   LCDR2 having an amino acid sequence of DNN (SEQ ID NO: 53);
   LCDR3 having an amino acid sequence of GIWDSSLDAYV (SEQ ID NO: 54);
   HCDR1 having an amino acid sequence of GFTFSSYS (SEQ ID NO: 49);
   HCDR2 having an amino acid sequence of ISSSSSYI (SEQ ID NO: 50);
   HCDR3 having an amino acid sequence of ARGNPWYGVDY (SEQ ID NO: 51);
(6) LCDR1 having an amino acid sequence of SSNIGNNY (SEQ ID NO: 58);
   LCDR2 having an amino acid sequence of DNN (SEQ ID NO: 59);
   LCDR3 having an amino acid sequence of GTWDSSLSAVV (SEQ ID NO: 60);
   HCDR1 having an amino acid sequence of GFTFSSYA (SEQ ID NO: 55);
   HCDR2 having an amino acid sequence of ITDSGDIT (SEQ ID NO: 56);
   HCDR3 having an amino acid sequence of ARMSSHWYFSADY (SEQ ID NO: 57); or
   the fully human antibody comprises a variant of the CDR sequence combination in any one of (1)-(6), wherein compared with the CDR sequence in any one of (1)-(6), the variant has at least 90% sequence identity, or comprises at least 1 and no more than 10 amino acid changes, or no more than 5, 4, 3, 2 or 1 amino acid changes in total in the CDR sequences.

In some embodiments, the amino acid sequence of the heavy chain variable region and/or the light chain variable region is selected from any one of the following combinations:
(1) a sequence as set forth in SEQ ID NO: 30 or a heavy chain variable region sequence having at least 90% sequence identity therewith, and
   a sequence as set forth in SEQ ID NO: 29 or a light chain variable region sequence having at least 90% sequence identity therewith;
(2) a sequence as set forth in SEQ ID NO: 36 or a heavy chain variable region sequence having at least 90% sequence identity therewith, and
   a sequence as set forth in SEQ ID NO: 35 or a light chain variable region sequence having at least 90% sequence identity therewith;
(3) a sequence as set forth in SEQ ID NO: 42 or a heavy chain variable region sequence having at least 90% sequence identity therewith, and
   a sequence as set forth in SEQ ID NO: 41 or a light chain variable region sequence having at least 90% sequence identity therewith;
(4) a sequence as set forth in SEQ ID NO: 90 or a heavy chain variable region sequence having at least 90% sequence identity therewith, and
   a sequence as set forth in SEQ ID NO: 89 or a light chain variable region sequence having at least 90% sequence identity therewith;
(5) a sequence as set forth in SEQ ID NO: 93 or a heavy chain variable region sequence having at least 90% sequence identity therewith, and
   a sequence as set forth in SEQ ID NO: 92 or a light chain variable region sequence having at least 90% sequence identity therewith;
(6) a sequence as set forth in SEQ ID NO: 96 or a heavy chain variable region sequence having at least 90% sequence identity therewith, and a sequence as set forth in SEQ ID NO: 95 or a light chain variable region sequence having at least 90% sequence identity therewith.

In some embodiments, the amino acid sequence of the heavy chain variable region and/or the light chain variable region is selected from any one of the following combinations:
(1) a heavy chain variable region sequence as set forth in SEQ ID NO: 30, and a light chain variable region sequence as set forth in SEQ ID NO: 29;
(2) a heavy chain variable region sequence as set forth in SEQ ID NO: 36, and a light chain variable region sequence as set forth in SEQ ID NO: 35;
(3) a heavy chain variable region sequence as set forth in SEQ ID NO: 42, and a light chain variable region sequence as set forth in SEQ ID NO: 41;
(4) a heavy chain variable region sequence as set forth in SEQ ID NO: 90, and a light chain variable region sequence as set forth in SEQ ID NO: 89;
(5) a heavy chain variable region sequence as set forth in SEQ ID NO: 93, and a light chain variable region sequence as set forth in SEQ ID NO: 92;
(6) a heavy chain variable region sequence as set forth in SEQ ID NO: 96, and a light chain variable region sequence as set forth in SEQ ID NO: 95.

In certain embodiments, the fully human antibody comprises an amino acid sequence as set forth in SEQ ID NO: 28, 34, 40, 88, 91 or 94.

In another aspect, the present application provides a nucleic acid molecule encoding the above-mentioned fully human antibody or single-chain antibody or fragment thereof.

In some embodiments, the nucleic acid molecule comprises a nucleotide sequence as set forth in any one of SEQ ID NOs: 25-27, 31-33, 37-39, and 79-87_{∘}

In another aspect, the present application provides an expression vector comprising the above-mentioned nucleic acid molecule.

In another aspect, the present application provides a host cell comprising the above-mentioned expression vector.

In another aspect, the present application provides a pharmaceutical composition comprising the above-mentioned fully human antibody or single chain antibody or fragment thereof, and a pharmaceutically acceptable carrier or diluent.

In another aspect, the present application provides a method for treating a disease or condition, comprising administering a therapeutically effective amount of the above-mentioned fully human antibody or single-chain antibody or fragment thereof, host cell, or pharmaceutical composition to a patient in need thereof, to eliminate, inhibit or reduce CD5 activity, thereby preventing, alleviating, improving or inhibiting the disease or condition.

In some embodiments, the disease or condition is a cancer.

In some embodiments, the cancer is a T-cell malignancy.

In another aspect, the present application provides an antibody or fragment competing for the same epitope with the above-mentioned fully human antibody or single-chain antibody or fragment thereof.

In another aspect, the present application provides a kit for detecting CD5 protein in a sample, wherein the kit comprises the above-mentioned fully human antibody or single-chain antibody or fragment thereof.

In another aspect, the present application provides use of the above-mentioned fully human antibody or single chain antibody or fragment thereof or host cell in the preparation of a drug for eliminating, inhibiting or reducing CD5 activity, thereby preventing, alleviating, improving or inhibiting a disease or condition.

In some embodiments, the disease or condition is a cancer.

In some embodiments, the cancer is a T-cell malignancy or a B-cell malignancy.

In some embodiments, the T-cell malignancy is selected from T-cell acute lymphocytic leukemia (T-ALL) and T-cell lymphoma (TCL), and the B-cell malignancy is selected from chronic lymphocytic leukemia (B-CLL) and mantle cell lymphoma (B-MCL).

In another aspect, the present application includes a chimeric antigen receptor (CAR), wherein the CAR comprises a CD5 binding domain, a transmembrane domain, a co-stimulatory domain and an intracellular signaling domain, and the amino acid sequences of the HCDR1, HCDR2, and HCDR3 are selected from the following combinations: (1) HCDR1 having an amino acid sequence as set forth in SEQ ID NO: 43, HCDR2 having an amino acid sequence as set forth in SEQ ID NO: 44, and HCDR3 having an amino acid sequence as set forth in SEQ ID NO: 45; (2) HCDR1 having an amino acid sequence as set forth in SEQ ID NO: 49, HCDR2 having an amino acid sequence as set forth in SEQ ID NO: 50, and HCDR3 having an amino acid sequence as set forth in SEQ ID NO: 51; (3) HCDR1 having an amino acid sequence as set forth in SEQ ID NO: 55, HCDR2 having an amino acid sequence as set forth in SEQ ID NO: 56, and HCDR3 having an amino acid sequence as set forth in SEQ ID NO: 57; (4) HCDR1 having an amino acid sequence as set forth in SEQ ID NO: 61, HCDR2 having an amino acid sequence as set forth in SEQ ID NO: 62, and HCDR3 having an amino acid sequence as set forth in SEQ ID NO: 63; (5) HCDR1 having an amino acid sequence as set forth in SEQ ID NO: 67, HCDR2 having an amino acid sequence as set forth in SEQ ID NO: 68, and HCDR3 having an amino acid sequence as set forth in SEQ ID NO: 69; or (6) HCDR1 having an amino acid sequence as set forth in SEQ ID NO: 73, HCDR2 having an amino acid sequence as set forth in SEQ ID NO: 74, and HCDR3 having an amino acid sequence as set forth in SEQ ID NO: 75.

In some embodiments, the antibody further comprises a light chain complementarity determining region 1 (LCDR1), a light chain complementarity determining region 2 (LCDR2) and a light chain complementarity determining region 3 (LCDR3), the amino acid sequences of the LCDR1, LCDR2, LCDR3 are selected from the following combinations: (1) LCDR1 having an amino acid sequence as set forth in SEQ ID NO: 46, LCDR2 having an amino acid sequence as set forth in SEQ ID NO: 47, and LCDR3 having an amino acid sequence as set forth in SEQ ID NO: 48; (2) LCDR1 having an amino acid sequence as set forth in SEQ ID NO: 52, LCDR2 having an amino acid sequence as set forth in SEQ ID NO: 53, and LCDR3 having an amino acid sequence as set forth in SEQ ID NO: 54; (3) LCDR1 having an amino acid sequence as set forth in SEQ ID NO: 58, LCDR2 having an amino acid sequence as set forth in SEQ ID NO: 59, and LCDR3 having an amino acid sequence as set forth in SEQ ID NO: 60; (4) LCDR1 having an amino acid sequence as set forth in SEQ ID NO: 64, LCDR2 having an amino acid sequence as set forth in SEQ ID NO: 65, and LCDR3 having an amino acid sequence as set forth in SEQ ID NO: 66; (5) LCDR1 having an amino acid sequence as set forth in SEQ ID NO: 70, LCDR2 having an amino acid sequence as set forth in SEQ ID NO: 71, and LCDR3 having an amino acid sequence as set forth in SEQ ID NO: 72; or (6) LCDR1 having an amino acid sequence as set forth in SEQ ID NO: 76, LCDR2 having an amino acid sequence as set forth in SEQ ID NO: 77, and LCDR3 having an amino acid sequence as set forth in SEQ ID NO: 78.

In some embodiments, the HCDR1, HCDR2, HCDR3 and LCDR1, LCDR2, LCDR3 comprised in the antibody are selected from any one of the following combinations: (1) HCDR1 having an amino acid sequence as set forth in SEQ ID NO: 43, HCDR2 having an amino acid sequence as set forth in SEQ ID NO: 44, and HCDR3 having an amino acid sequence as set forth in SEQ ID NO: 45, LCDR1 having an amino acid sequence as set forth in SEQ ID NO: 46, LCDR2 having an amino acid sequence as set forth in SEQ ID NO: 47, and LCDR3 having an amino acid sequence as set forth in SEQ ID NO: 48; (2) HCDR1 having an amino acid sequence as set forth in SEQ ID NO: 49, HCDR2 having an amino acid sequence as set forth in SEQ ID NO: 50, and HCDR3 having an amino acid sequence as set forth in SEQ ID NO: 51, LCDR1 having an amino acid sequence as set forth in SEQ ID NO: 52, LCDR2 having an amino acid sequence as set forth in SEQ ID NO: 53, and LCDR3 having an amino acid sequence as set forth in SEQ ID NO: 54; (3) HCDR1 having an amino acid sequence as set forth in SEQ ID NO: 55 , HCDR2 having an amino acid sequence as set forth in SEQ ID NO: 56, and HCDR3 having an amino acid sequence as set forth in SEQ ID NO: 57, LCDR1 having an amino acid sequence as set forth in SEQ ID NO: 58, LCDR2 having an amino acid sequence as set forth in SEQ ID NO: 59, and LCDR3 having an amino acid sequence as set forth in SEQ ID NO: 60; (4) HCDR1 having an amino acid sequence as set forth in SEQ ID NO: 61, HCDR2 having an amino acid sequence as set forth in SEQ ID NO: 62, and HCDR3 having an amino acid sequence as set forth in SEQ ID NO: 63, LCDR1 having an amino acid sequence as set forth in SEQ ID NO: 64, LCDR2 having an amino acid sequence as set forth in SEQ ID NO: 65, and LCDR3 having an amino acid sequence as set forth in SEQ ID NO: 66; (5) HCDR1 having an amino acid sequence as set forth in SEQ ID NO: 67, HCDR2 having an amino acid sequence as set forth in SEQ ID NO: 68, and HCDR3 having an amino acid sequence as set forth in SEQ ID NO: 69, LCDR1 having an amino acid sequence as set forth in SEQ ID NO: 70, LCDR2 having an amino acid sequence as set forth in SEQ ID NO: 71, and LCDR3 having an amino acid sequence as set forth in SEQ ID NO: 72; or (6) HCDR1 having an amino acid sequence as set forth in SEQ ID NO: 73, HCDR2 having an amino acid sequence as set forth in SEQ ID NO: 74, and HCDR3 having an amino acid sequence as set forth in SEQ ID NO: 75, LCDR1 having an amino acid sequence as set forth in SEQ ID NO: 76, LCDR2 having an amino acid sequence as set forth in SEQ ID NO: 77, and LCDR3 having an amino acid sequence as set forth in SEQ ID NO: 78.

In some embodiments, the antibody comprises a heavy chain variable region, and the amino acid sequence of the heavy chain variable region is set forth in SEQ ID NO: 30, 36, 42, 90, 93 or 96. In some embodiments, the antibody comprises a light chain variable region, and the amino acid sequence of the light chain variable region is set forth in SEQ ID NO: 29, 35, 41, 89, 92 or 95. In some embodiments, the antibody is a single chain antibody. In some embodiments, the antibody comprises an amino acid sequence as set forth in SEQ ID NO: 28, 34, 40, 88, 91 or 94 or a functional variant thereof.

In some embodiments, the transmembrane domain of the CAR comprises a polypeptide selected from the following proteins: α, β or ζ chains of T-cell receptors, CD28, CD3e, CD45, CD4, CD5, CD8a, CD9, CD16, CD22, CD33, CD37, CD64, CD80, CD86, CD134, CD137, and CD154. In some embodiments, the transmembrane domain comprises an amino acid sequence as set forth in SEQ ID NO: 6 or a functional variant thereof.

In some embodiments, the co-stimulatory domain of the CAR comprises a polypeptide selected from the following proteins: CD28, 4-1BB, OX-40, and ICOS. In some embodiments, the co-stimulatory domain comprises an amino acid sequence as set forth in SEQ ID NO: 8 or a functional variant thereof.

In some embodiments, the intracellular signaling domain of the CAR comprises a signaling domain from CD3ζ. In some embodiments, the intracellular signaling domain comprises an amino acid sequence as set forth in SEQ ID NO: 10 or a functional variant thereof.

In some embodiments, the CAR further comprises a hinge region connecting the antibody and the transmembrane domain. In some embodiments, the hinge region comprises an amino acid sequence as set forth in SEQ ID NO: 4 or a functional variant thereof.

In some embodiments, the CAR is also linked to a CD8α signal peptide. In some embodiments, the signal peptide comprises an amino acid sequence as set forth in SEQ ID NO: 2 or a functional variant thereof.

In some embodiments, the CAR is also linked to a cleaving peptide. In some embodiments, the cleaving peptide comprises an amino acid sequence from a T2A peptide. In some embodiments, the cleaving peptide comprises an amino acid sequence as set forth in SEQ ID NO: 12 or a functional variant thereof. In some embodiments, the CAR is also linked to a truncated EGFR molecule (tEGFR) by linking to the cleaving peptide and a CSF2RA signal peptide. In some embodiments, the truncated EGFR molecular comprises an amino acid sequence as set forth in SEQ ID NO: 16 or a functional variant thereof. The CSF2RA signal peptide comprises an amino acid sequence as set forth in SEQ ID NO: 14 or a functional variant thereof.

In some embodiments, the CAR comprises an amino acid sequence selected from SEQ ID NO: 22-24, 98, 100 or 102 or a functional variant thereof.

In another aspect, the present application further includes an isolated nucleic acid molecule encoding the CAR of the present application.

In another aspect, the present application further includes an isolated nucleic acid molecule encoding CAR, which comprises a nucleic acid sequence selected from the group consisting of SEQ ID NO: 1, 3, 5, 7, 9, 11, 13, 15, 17, 19-21, 25-27, 31-33, 37-39, 79-87, 97, 99 or 101 or a functional variant thereof.

In another aspect, the present application further includes a vector comprising the nucleic acid molecule of the present application. In some embodiments, the vector is selected from a plasmid, a retroviral vector and a lentiviral vector.

In another aspect, the present application further includes an immune effector cell comprising the CAR of the present application, the nucleic acid molecule of the present application, or the vector of the present application. In some embodiments, the immune effector cell is selected from a T lymphocyte and a natural killer (NK) cells In some embodiments, CD5 on the immune effector cell is not expressed.

In another aspect, the present application further includes a method for preparing an immune effector cell, which comprises knocking out the CD5 gene of the immune effector cell, and introducing the vector of the present application into the immune effector cells. In some embodiments, the knocking out is carried out using the sgRNA of any one of SEQ ID NOs: 103-106.

In another aspect, the present application further includes a pharmaceutical composition, which comprises the immune effector cells of the present application and a pharmaceutically acceptable adjuvant.

In another aspect, the present application further includes use of the CAR, the nucleic acid molecule, the vector, or the immune effector cell in the preparation of a drug for treating a disease or condition associated with the expression of CD5. In some embodiments, the disease or condition associated with the expression of CD5 is a cancer or malignancy.

In another aspect, the present application further relates to use of the CAR, the nucleic acid molecule, the vector, or the immune effector cell or the pharmaceutical composition in the treatment of a disease or condition associated with the expression of CD5. In some embodiments, the disease or condition associated with the expression of CD5 is a cancer or malignancy. In another aspect, the present application further includes a method of treating a disease or condition associated with the expression of CD5, which comprises administering a therapeutically effective amount of the immune effector cell of the present application, or the pharmaceutical composition of the present application to a patient in need thereof. In some embodiments, anti-EGFR antibodies such as cetuximab can be further administered to patients in need thereof to inhibit the effect of the immune effector cell or the pharmaceutical composition, wherein the patients in need thereof include those patients who have serious adverse reactions to the immune effector cell or pharmaceutical composition of the present application.

Those skilled in the art can easily perceive other aspects and advantages of the present application from the following detailed description. In the following detailed description, only exemplary embodiments of the present application are shown and described. As those skilled in the art will appreciate, the content of the present application enables those skilled in the art to make changes to the disclosed specific embodiments without departing from the spirit and scope of the invention in which the present application is involved. Correspondingly, the drawings and descriptions in the specification of the present application are only exemplary rather than restrictive.

### DESCRIPTION OF DRAWINGS

Fig. 1 illustrates selecting of CD5 ko sgRNA with jurkat cell line and shows the results of flow cytometry of cell clones obtained by knocking out jurkat cells with different sgRNAs.
Fig. 2 shows the results of identification of CD5 expression in the monoclonal jurkat-CD5 ko cell line by flow cytometry.
Fig. 3 shows the CAR structure used in this experiment (Fig. 3A) and the CD5 and EGFRt expression detected in CAR-T cells after transfection (Fig. 3B).
Fig. 4 shows the results of CD107a degranulation of CAR-T cells by different target cells. Among the results of each CAR molecule, from left to right are the results of co-incubation of CAR-T cells with Jurkat, Jurkat CD5 ko, CCRF-CEM, MOLT4, K562-CD5, K562 and RAJI cells and the results of incubation of CAR-T cells alone.
Fig. 5 shows the results of killing of various target cells by CAR-T cells.
Fig. 6 shows the experimental results of repeated stimulation of CAR-T cell proliferation by CCRF-CEM cells treated with mitomycin.
Fig. 7 shows the dynamic binding curves and parameters KD, kon and kdis of affinity determination of H65, Clone 10, Clone 32 and Clone 35 with CD5 antigen.
Fig. 8 shows a general procedure for selecting specific antibodies targeting CD5 from a phage antibody library according to the present invention.
Fig. 9 shows the results of enzyme-linked immunosorbent assay (ELISA) of some of the panned phage monoclones with the target antigen and the control antigen.
Fig. 10 shows the results of flow cytometry for the binding of some phage monoclones to Raji and Jurkat cells.
Figs. 11 A-F shows the results of flow cytometry (peaks and MFI values) for the binding of the selected phage monoclones #1-64 to various CD5 positive and negative cell lines. Negative Control is a negative control phage antibody clone.

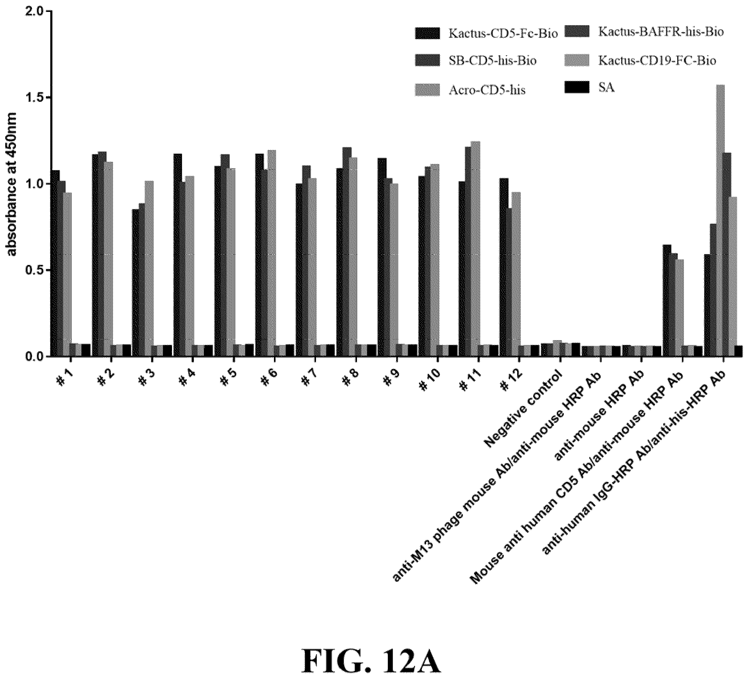

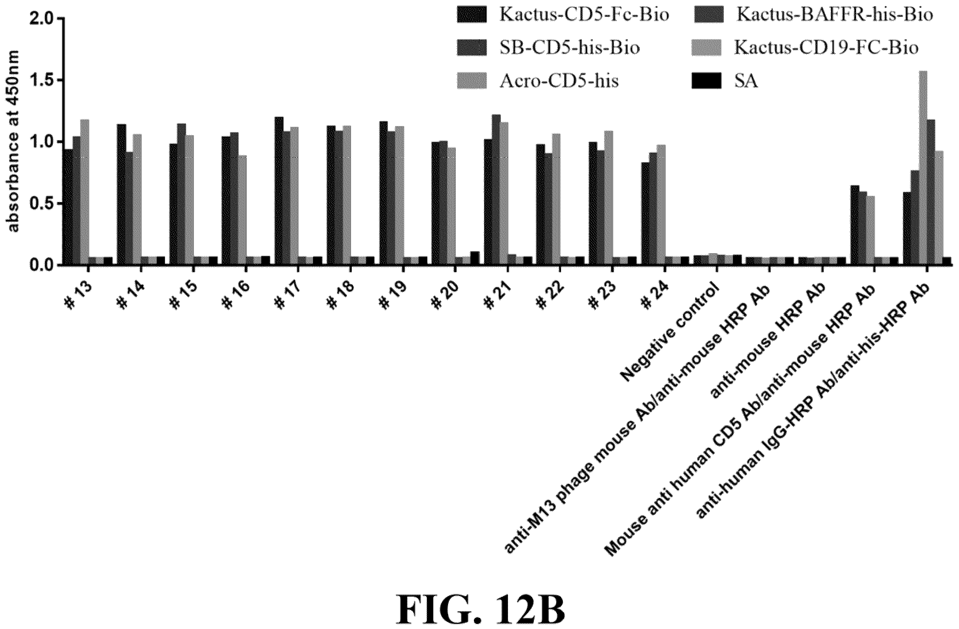

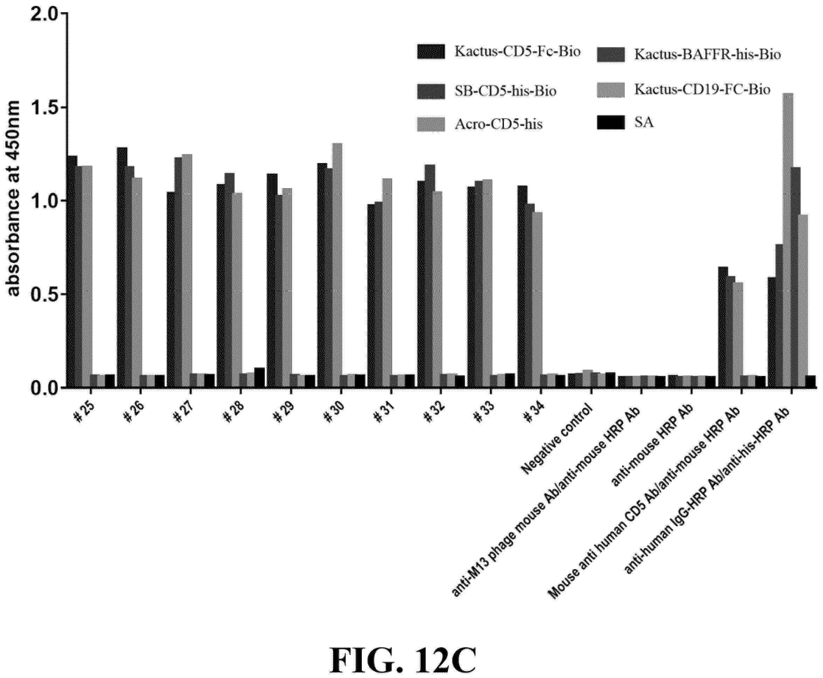

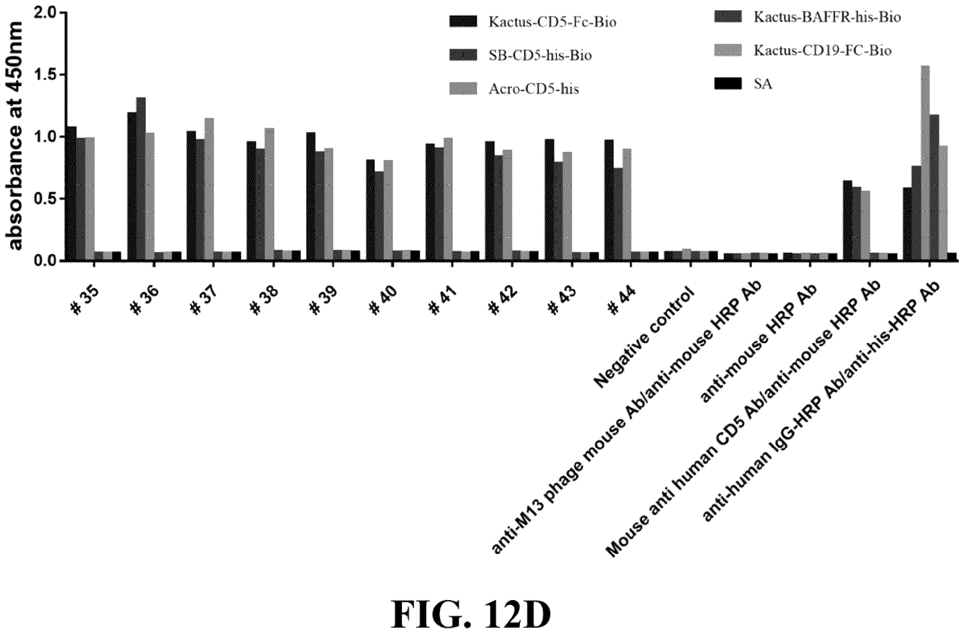

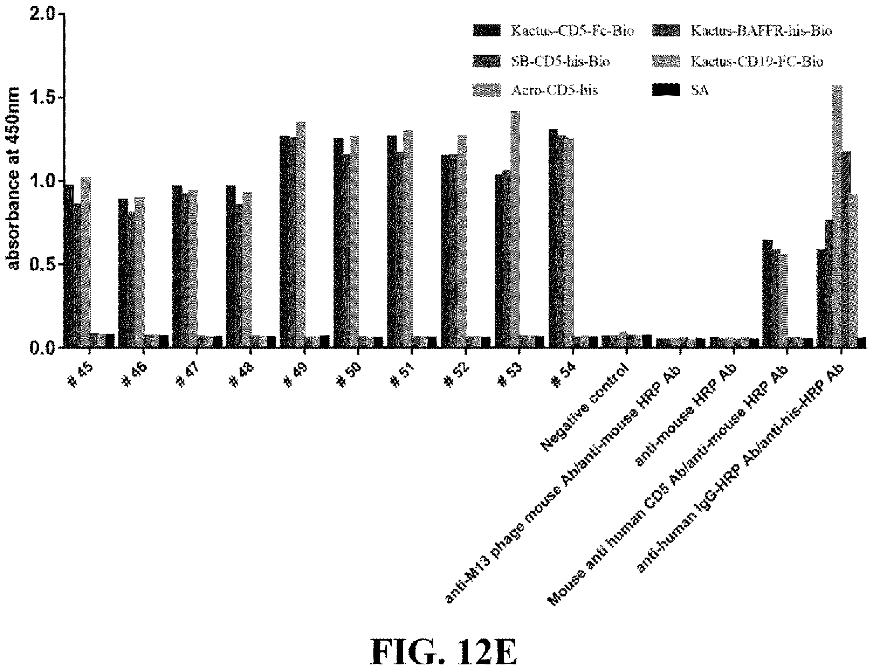

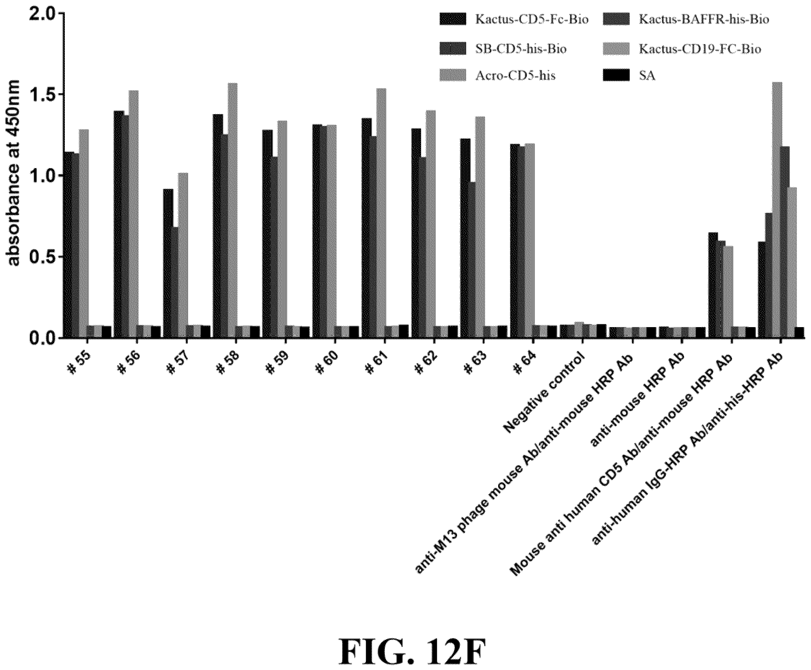

Figs. 12A-F show the results of ELISA for the selected phage monoclones #1-64 with various CD5 antigen proteins and non-related antigens from different companies. Negative control is a negative control phage antibody clone, anti-M13 phage mouse Ab/anti-mouse HRP Ab is a negative antibody control with only the primary and secondary antibodies, anti-mouse HRP Ab is a negative antibody control with only the secondary antibody, Mouse anti human CD5 Ab/antimouse HRP Ab is a positive antibody control for the target antigen (CD5-Fc-Bio), and anti-human IgG-HRP Ab/anti-his-HRP Ab is a positive antibody control for detecting the antigen label. Among them, in Figs. 12A-F, the histograms corresponding to each test antibody and the control group indicate from left to right the test results with proteins Kactus-CD5-Fc-Bio, SB-CD5-his-Bio, Acro-CD5-his, Kactus-BAFFR-his-Bio, Kactus-CD19-FC-Bio, and SA.

### DETAILED DESCRIPTION OF THE INVENTION

The implementation of the invention of the present application will be described in the following specific examples, and those skilled in the art can easily understand other advantages and effects of the invention of the present application from the content disclosed in the specification. The CAR of the present application can specifically bind to CD5, the CAR-T cells prepared by using the CAR can stably express the CAR, and the CAR-T cells prepared by using the CAR have a high CAR-positive rate. In addition, the CAR can promote the release of cytokines and can be used for treating diseases or conditions associated with the expression of CD5.

The implementation of the present application will employ, unless expressly indicated to the contrary, conventional methods of chemistry, biochemistry, organic chemistry, molecular biology, microbiology, recombinant DNA techniques, genetics, immunology and cell biology, within the skill of the art. Descriptions of these methods can be found, for example, in Sambrook et al., Molecular Cloning: A Laboratory Manual (3rd Ed., 2001); Sambrook et al., Molecular Cloning: A Laboratory Manual (2nd Ed., 1989); Maniatis et al., Molecular Cloning: A Laboratory Manual (1982); Ausubel et al., Current Protocols in Molecular Biology (John Wiley and Sons, updated in July 2008); Short Protocols in Molecular Biology: A Compendium of Methods from Current Protocols in Molecular Biology, Greene Pub. Associates and Wiley-Interscience; Glover, DNA Cloning: A Practical Approach, vol.I&II (IRL Press, Oxford, 1985); Anand, Techniques for the Analysis of Complex Genomes, (Academic Press, New York, 1992); Transcription and Translation (B. Hames&S. Higgins, Eds., 1984); Perbal, A Practical Guide to Molecular Cloning (1984); Harlow and Lane, Antibodies, (Cold Spring Harbor Laboratory Press, Cold Spring Harbor, NY, 1998) Current Protocols in Immunology Q.E. Coligan, A.M. Kruisbeek, D.H. Margulies, E.M. Shevach and W. Strober, eds., 1991); Annual Review of Immunology; and journal monographs such as Advances in Immunology.

Unless otherwise defined, all technical and scientific terms used in the present application have the same meaning as commonly understood by one of ordinary skill in the art. For the purposes of the present application, the following terms are defined below.

In the present application, the term "chimeric antigen receptor" (CAR) is a fusion protein of the variable region of a single-chain antibody and a T-cell signaling molecule. It allows T-cells to recognize specific antigens in a non-MHC-restricted manner and play a killing role. CAR is the core component of chimeric antigen receptor T-cells (CAR-Ts), and it can comprise a tumorassociated antigen (TAA) binding region, a transmembrane domain, a co-stimulatory domain and an intracellular signaling domain, and can further comprise a hinge region between the tumorassociated antigen binding region and the transmembrane domain. In the present application, the CAR can be a genetically engineered chimeric protein capable of redirecting the cytotoxicity of immune effector cells to B-cells, which combines antibody-based antigen (such as CD5) specificity with the T-cell receptor activation intracellular domain. T-cells genetically modified to express CAR can specifically recognize and eliminate malignant cells expressing the target antigen. For descriptions of CAR and CAR T-cells, see, for example, Sadelain M, Brentjens R, Rivi 'ere I. The basic principles of chimeric antigen receptor design. Cancer Discov. 2013;3(4):388-398; Turtle CJ, Hudecek M, Jensen MC, Riddell SR. Engineered T-cells for anticancer therapy. Curr Opin Immunol. 2012;24(5):633-639; Dotti G, Gottschalk S, Savoldo B, Brenner MK. Design and development of therapies using chimeric antigen receptor-expressing T-cells. Immunol Rev. 2014;257(1):107-126; and WO2013154760, WO2016014789.

In the present application, the term "CD5" is a type I transmembrane glycosylated protein that plays an important role in the negative regulation of T-cell receptor signaling and promotes the survival of normal and malignant lymphocytes. CD5 is one of the characteristic surface markers of T-cell malignancies, and 80% of T-cell acute lymphocytic leukemia (T-ALL) and peripheral T-cell lymphomas express CD5. In the present application, the CD5 may be human CD5, whose GenBank accession number is NM_014207.4. The CD5 proteins may also include fragments of CD5, such as the extracellular domain and fragments thereof.

In the present application, the term "CD5 binding domain" generally refers to the extracellular domain of CD5 CAR, which can specifically bind to an antigen. For example, the CD5 extracellular binding domain may comprise a chimeric antigen receptor capable of specifically binding to a CD5 polypeptide expressed on a human cell, an anti-CD5 antibody or an antigen-binding fragment thereof. The terms "binding domain", "extracellular domain", "extracellular binding domain", "antigen-specific binding domain" and "extracellular antigen-specific binding domain" are used interchangeably in the present application, and a CAR with the ability to specifically bind to a target antigen of interest (e.g., CD5) is provided. The CD5 binding domain may be of natural, synthetic, semi-synthetic or recombinant origin.

In the present application, the term "antibody" generally refers to a polypeptide molecule capable of specifically recognizing and/or neutralizing a specific antigen. For example, an antibody may comprise an immunoglobulin composed of at least two heavy (H) chains and two light (L) chains inter-connected by disulfide bonds, and includes any molecule comprising an antigen-binding portion thereof. The term "antibody" includes monoclonal antibodies, antibody fragments or antibody derivatives, including but not limited to human antibodies, humanized antibodies, chimeric antibodies, single domain antibodies (e.g., dAb), single chain antibodies (e.g., scFv). In the present application, a "fragment" of an antibody may refer to an antigen-binding fragment of an antibody, for example, Fab, Fab' and (Fab')₂ fragments,etc. The term "antibody" also includes all recombinant forms of antibodies, such as antibodies expressed in prokaryotic cells, unglycosylated antibodies, and any antigen-binding antibody fragments and derivatives thereof. Each heavy chain can be composed of a heavy chain variable region (VH) and a heavy chain constant region. Each light chain can be composed of a light chain variable region (VL) and a light chain constant region. The VH and VL regions can be further distinguished into hypervariable regions called complementarity determining regions (CDRs), which are interspersed in more conserved regions called framework regions (FRs). Each VH and VL may consist of three CDR and four FR regions, which may be arranged in the following order from amino-terminus to carboxy-terminus: FR1, CDR1, FR2, CDR2, FR3, CDR3 and FR4. The variable regions of the heavy and light chains contain binding domains that interact with the antigen. The constant regions of the antibodies mediate the binding of the immunoglobulin to host tissues or factors which include various cells of the immune system (e.g., effector cells) and the first component (Clq) of the classical complement system.

In the present application, the term "single-chain antibody", which may also be called scFv, refers to an antibody consisting of one chain composed of a heavy chain variable region and a light chain variable region connected by a linker peptide.

"Murine antibody" is an antibody produced by murine against a specific antigen, usually referring to an antibody produced by mouse B lymphocytes. In most cases, the murine antibody is a monoclonal antibody produced by hybridoma cells. The fully human antibody of the present application is selected from a human phage antibody library, which reduces the immunogenicity compared with the murine antibody, and is more conducive to the therapeutic use in the human body.

The "fully human antibody or single-chain antibody or fragment thereof" of the present application generally refers to any form of antigen-binding molecule capable of binding to a target antigen. For example, the antigen-binding molecule can be a protein or a polypeptide, including, for example, an antibody and its antigen-binding fragments, single-chain scFv antibodies, single-domain antibodies, various fusions and conjugates constructed based on scFv, such as scFv-Fc antibodies, immunoconjugates, antibody drug conjugates (ADCs), multi/bispecific antibodies, chimeric antigen receptors (CARs).

"Epitope" refers to a portion of a molecule that is bound by an antigen-binding protein (e.g., an antibody). Epitopes may comprise non-contiguous portions of the molecule (e.g., in polypeptides, amino acid residues that are not contiguous in the primary sequence of the polypeptide, but are sufficiently close to each other in the trivalent and tetravalent structure of the polypeptide to be bound by the antigen binding protein).

In the present application, the term "transmembrane domain" generally refers to a domain in CAR that passes through the cell membrane, which is connected to the intracellular signaling domain and plays a role in transmitting signals. In the present invention, the transmembrane domain may be CD28 transmembrane domain.

In the present application, the term "co-stimulatory domain" generally refers to an intracellular domain that can provide immune co-stimulatory molecules which are cell surface molecules required for an effective response of lymphocytes to antigens. The co-stimulatory domain may include the co-stimulatory domain of CD28, and may also include the co-stimulatory domain of the TNF receptor family, such as the co-stimulatory domain of OX40 and 4-1BB.

In the present application, the term "hinge region" generally refers to the connecting region between the antigen-binding region and the binding region of an immune cell Fc receptor (FcR). In the present invention, the hinge region may be CD28 hinge region.

In the present application, the term "intracellular signaling domain" generally refers to the component of CAR for signal transduction in cells, which includes a signaling domain and a domain that specifically binds to the receptor component, for example: it may be selected from CD3ζ intracellular domain, CD28 intracellular domain, CD28 intracellular domain, 4-1BB intracellular domain and OX40 intracellular domain.

In the present application, the term "CD8α signal peptide" generally refers to a short (5-30 amino acids in length) peptide chain that guides the transfer of newly synthesized proteins to the secretory pathway.

In the present application, the term "cleaving peptide" refers to a self-cleaving 2A peptide, which can realize the function of cleaving proteins through ribosome skipping instead of proteolytic hydrolysis, and may include T2A, F2A and P2A, etc.

In the present application, the term "marker detection signal" generally refers to a gene, protein or other molecules with a known function or sequence that can function as a specific marker and emit a detectable signal. The marker detection signal can be a fluorescent protein, such as: GFP, RFP, and YFP. The marker detection signal may be EGFRt. The terms "EGFRt" and "tEGFR" are used interchangeably in the present invention and refer to a gene encoding a truncated human epidermal growth factor receptor polypeptide, which lacks the distal membrane EGF binding domain and cytoplasmic signaling tail, but retains extracellular epitopes recognized by anti-EGFR antibodies. EGFRt can be used as a non-immunogenic selection tool as well as a tracking marker which has a function of genetically modifying cells. In the present application, it can be used as a marker molecule for CAR-T cells, which is used to clear the anti-EGFR antibody (for example, cetuximab) mediated ADCC pathway (cetuximab mediated ADCC pathway) of CAR-T cells in vivo if necessary (see US8802374B2), that is, it is used as a safety switch during clinical transformation.

In the present invention, the term "CSF2RA signal peptide", that is, colony stimulating factor 2 receptor subunit alpha signal peptide, is a peptide chain that can guide the expression of newly synthesized proteins on the surface of CAR-T cells.

In the present application, "anti-EGFR antibody" refers to an antibody that has the ability to trigger antibody dependent cell-mediated cytotoxicity, allowing immune cells to attack CAR-T cells with truncated epidermal growth factor receptor (EGFRt), and assist in the removal of CAR-T cells. The anti-EGFR antibody can be used when patients have severe adverse reactions after infusion of CAR-T or when removal of CAR-T cells is required. It can assist in the removal of CAR-T cells and alleviate symptoms related to CAR-T treatment. The anti-EGFR antibodies include, but are not limited to, cetuximab, panitumumab, necitumumab and nimotuzumab.

In the present application, "sequence identity" generally refers to the degree to which sequences are identical on a nucleotide-by-nucleotide or amino-acid-by-amino acid basis over a comparison window. "Percent sequence identity" can be calculated by comparing two optimally aligned sequences over a comparison window and determining the number of positions of identical nucleic acid bases (e.g., A, T, C, G, I) or identical amino acid residue (e.g., Ala, Pro, Ser, Thr, Gly, Val, Leu, Ile, Phe, Tyr, Trp, Lys, Arg, His, Asp, Glu, Asn, Gln, Cys, and Met) present in two sequences to obtain the number of matching positions, and dividing the number of matching positions by the total number of positions in the comparison window (i.e., window size), and multiplying the result by 100 to yield the percent sequence identity. Optimal alignment for purposes of determining percent sequence identity can be achieved in various ways known in the art, for example, using publicly available computer software such as BLAST, BLAST-2, ALIGN or Megalign (DNASTAR). Those skilled in the art can determine appropriate parameters for aligning sequences, including any algorithms needed to achieve maximal alignment over the full length of the sequences being compared or over a region of sequences of interest.

In some embodiments, the fully human antibody provided by the present application further comprises an amino acid sequence having at least 90% sequence identity (e.g., at least 95%, at least 98%, at least 99% or even 100% sequence identity) with an sequence as set forth in any one of SEQ ID NO: 28, 34, 40, 88, 91 or 94.

Those skilled in the art can understand that, on the basis of the specific sequences provided herein, the corresponding variants of the antibodies targeting CD5 provided herein can be obtained by substituting, deleting, adding a few amino acids, and verifying or selecting the resultant product for its binding ability with the corresponding antigen CD5 or its biological activity, and these variants should also be included within the scope of the present invention. For example, the fully human antibody or single-chain antibody or fragment thereof of the present application may have at least one and no more than 10, or no more than 5, 4, 3, 2 or 1 amino acid changes in the full-length or CDR sequence.

Those skilled in the art can also understand that, on the basis of the specific heavy chain variable region sequences provided herein, an antibody light chain library (such as a human phage light chain library) can be selected by using CD5 as the antigen, so as to obtain light chain variable regions matched with the heavy chain variable regions while maintaining CD5 binding ability. Anti-CD5 antibody molecules obtainable in this way are also included in the scope of the present invention.

In some embodiments, the antigen binding molecules of the present application may further comprise post-translational modifications. Examples of post-translational protein modifications include: phosphorylation, acetylation, methylation, ADP-ribosylation, ubiquitination, glycosylation, carbonylation, ubiquitination, biotinylation, or addition of polypeptide side chains or hydrophobic groups. Thus, a modified soluble polypeptide may comprise non-amino acid components such as lipoids, polysaccharides or monosaccharides, and phosphates. A preferred form of glycosylation is the modification by sialylation, the attachment of one or more sialic acid groups to a polypeptide. The sialic acid group improves the solubility and serum half-life of the protein, while reducing the possible immunoheritability of the protein. See Raju et al. Biochemistry. 2001 31;40(30):8868-76.

The term "functional variant" of a protein or polypeptide sequence in the present application refers to a sequence having amino acid changes compared to the parent sequence by 1 or more, such as 1-30, or 1-20 or 1-10, such as 1 or 2 or 3 or 4 or 5 amino acid substitutions, deletions and/or insertions. Functional variants substantially retain the biological properties of the protein or polypeptide sequence before the change. In one aspect, the present application encompasses variants of any protein or polypeptide sequence of the present application. In some embodiments, a functional variant of a protein or polypeptide sequence retains at least 60%, 70%, 80%, 90%, or 100% of the biological activity of the parent sequence before the change. The functional variants described in the present invention may be functional variants of signal peptide, antibody, hinge region, transmembrane domain, co-stimulatory domain, intracellular signaling domain, cleaving peptide, CSF2RA signal peptide, and EGFRt. When referring to functional variants of antibodies, functional variants such as antibody variable regions (such as VH or VL), antibody constant regions (such as CH or CL), heavy chain CDR regions (HCDR1, HCDR2 or HCDR3), and light chain CDR regions (LCDR1, LCDR2 or LCDR3) are also included. Amino acid substitutions, deletions and/or insertions may occur in either the heavy chain CDR region or the light chain CDR region, or the heavy chain FR region or the light chain FR region, or the heavy chain constant region or the light chain constant region, wherein the variant substantially retains the biological properties of the previous antibody molecule before the change. For antibodies, biological activities include, for example, antigen binding ability. In some embodiments, functional variants of antibodies comprise amino acid changes that do not result in the antibody variant losing binding to the antigen, but optionally confer properties such as increased affinity for the antigen and different effector functions. It is understood that the antibody heavy chain variable region or light chain variable region, or the CDR regions can be altered individually or in combination. In some embodiments, no more than 1, 2, 3, 4, 5, 6, 7, 8, 9 or 10 amino acid changes are present in one or more or all three heavy chain CDRs. In some embodiments, the amino acid changes may be amino acid substitutions, such as conservative substitutions. In some embodiments, the functional variant has at least 80%, 85%, 90% or 95% or 99% or more amino acid identity to the parent. Similarly, a "functional variant" of a nucleic acid molecule in the present application refers to a nucleic acid molecule that encodes the same amino acid sequence as the parent nucleic acid molecule.

In the present application, the term "isolated" generally means that an antibody is one that has been separated from components of its natural environment. In some embodiments, antibodies are purified to have a purity greater than 95% or 99% by, for example, electrophoresis (e.g., SDS-PAGE, isoelectric focusing (IEF), capillary electrophoresis) or chromatography (e.g., ion exchange or reversed-phase HPLC). A review of methods for assessing antibody purity can be found in Flatman, S. et al., J. Chrom. B 848 (2007) 79-87.

In the present application, the term "nucleic acid molecule" generally refers to isolated nucleotides, deoxyribonucleotides or ribonucleotides or analogs thereof of any length isolated from their natural environment or artificially synthesized. The nucleic acid molecule of the present application can be isolated. For example, it may be produced or synthesized by (i) amplification in vitro, such as amplification by polymerase chain reaction (PCR), (ii) cloning and recombination, (iii) purification, for example by fractionation through enzyme digestion and gel electrophoresis, or (iv) synthesis, for example by chemical synthesis. In some embodiments, the isolated nucleic acid is a nucleic acid molecule prepared by recombinant DNA technology. In the present application, nucleic acids encoding the antibodies or antigen-binding fragments thereof can be prepared by various methods known in the art, including, but not limited to, manipulations using restriction fragments or using overlap extension PCR of synthetic oligonucleotides. For specific operations, refer to Sambrook et al., Molecular Cloning, A Laboratory Manual, Cold Spring Harbor Laboratory Press, Cold Spring Harbor, N.Y., 1989; and Ausube et al., Current Protocols in Molecular Biology, Greene Publishing and Wiley-Interscience, New York N.Y., 1993.

In the present application, the term "vector" generally refers to a nucleic acid molecule capable of self-replicating in a suitable host for transferring an inserted nucleic acid molecule into and/or between host cells. The vector may include vectors mainly used for inserting DNA or RNA into cells, vectors mainly used for replicating DNA or RNA, and vectors mainly used for expression of transcription and/or translation of DNA or RNA. The vector also includes a vector having various functions as described above. The vector may be a polynucleotide capable of being transcribed and translated into a polypeptide when introduced into a suitable host cell. Generally, the vector can produce the desired expression product by culturing an appropriate host cell containing the vector. In the present application, one or more of the nucleic acid molecules may be comprised in the vector. In addition, other genes may be comprised in the vector, such as marker genes that allow selection of the vector in appropriate host cells and under appropriate conditions. In addition, the vector may also comprise expression control elements that permit proper expression of the coding region in an appropriate host. Such control elements are well known to those skilled in the art, and may include, for example, promoters, ribosome binding sites, enhancers, and other control elements that regulate gene transcription or mRNA translation. In some embodiments, the expression control sequences are regulatable elements. The specific structure of the expression control sequence may vary depending on the species or the function of the cell type, but generally includes 5' non-transcribed sequences and 5' and 3' non-translated sequences involved in the initiation of transcription and translation, respectively, such as TATA box, capping sequence, and CAAT sequence. For example, the 5' non-transcribed expression control sequence may comprise a promoter region which may comprise a promoter sequence for transcriptional control of the functionally linked nucleic acid. The vectors of the present application may be selected from plasmids, retroviral vectors and lentiviral vectors. The plasmids, retroviral vectors and lentiviral vectors of the present application may comprise CAR.

In the present application, the term "plasmid" generally refers to DNA molecules other than chromosomes or nucleoids in organisms such as bacteria and yeast, which exist in the cytoplasm and have the ability to replicate autonomously, enabling them to maintain a constant number of copies in daughter cells and express the genetic information they carry. Plasmids are used as vectors of genes in genetic engineering research.

In the present application, the term "retroviral vector" generally refers to a type of RNA virus whose genetic information is stored on ribonucleic acid, and most of these viruses have reverse transcriptase. Retroviruses contain at least three genes: *gag*, a gene for the proteins that make up the center and structure of the virus; *pol*, a gene for the reverse transcriptase enzyme; and *env*, a gene for the proteins that make up the viral capsid. Through retroviral transfection, the retroviral vector can randomly and stably integrate its own genome and the foreign genes that it carries into the host cell genome, for example, integrate CAR molecules into the host cell.

In the present application, the term "lentiviral vector" generally refers to a type of diploid RNA viral vector belonging to retroviruses. The lentiviral vector is based on the genome of lentivirus. Multiple sequence structures related to viral activity in the genome of lentivirus are removed to make it biologically safe, and then a vector prepared from the sequence and expression structure of the target gene required for the experiment is introduced into the genome skeleton. Compared with other retroviruses, lentiviral vectors have a wider range of hosts, have the ability to infect both dividing and non-dividing cells, and for some cells that are difficult to transfect, such as primary cells, stem cells and undifferentiated cells, can greatly improve the transduction efficiency of the target gene (see Chen Chen and Wan Haisu, "Lentiviral vectors and their research progress, Chinese Journal of Lung Cancer 17.12 (2014): 870-876. PMC). Through lentiviral vector transfection, the lentiviral vector can randomly and stably integrate its own genome and the foreign genes that it carries into the host cell genome, for example, integrate CAR molecules into the host cell.

In the present application, the term "transposon" generally refers to a discrete segment of DNA containing a transposase gene, flanked by terminal inverted repeats (TIRs) containing transposase binding sites. Transposases can bind to TIRs and transfer the transposon to a new site. The transposon of the present application is a two-component system consisting of a plasmid carrying CAR (transposon) and another plasmid carrying a transposase. The transposon can be introduced into target cells by means of electrotransduction and the like. For example, first, the two components are electroporated into peripheral blood mononuclear cells (PBMCs), and the expressed transposase acts on the terminal inverted repeats (TIRs) on both sides of the CAR for cleavage of the CAR (transposon) and subsequent integration into the TA dinucleotide sequence in the genome of the target cell (e.g., T-cell). After transposition and stable genomic integration, the CAR protein can be expressed on the surface of the target cell (see Cheng Zhang, Jun Liu, Jiang F Zhong, et al. Engineering CAR-T cells. Biomarker Research. 2017, 5:22).

In the present application, the term "gene editing" generally refers to the technology of sitedirected modification of the genome, which may include those based on zinc finger nucleases (ZFNs), transcription activator like effector nucleases (TALENs), clustered regularly interspaced short palindromic repeats/CRISPR-associated protein (Cas9) (CRISPR/Cas9), etc. It enables highly efficient targeted modification of the genome by adding, removing or changing genetic materials at specific positions in the genome. The gene editing of the present application may include introducing CAR molecules into the genome of recipient cells through gene editing technologies (such as CRISPR-Cas9).

In the present application, the term "immune effector cell" generally refers to immune cells involved in clearing foreign antigens and performing effector functions in an immune response. For example, plasma cells, cytotoxic T-cells, NK cells, APSC pluripotent cells, mast cells, etc. are included.

In the present application, the term "pharmaceutically acceptable adjuvant" generally refers to a pharmaceutically acceptable formulation carrier, solution or additive that enhances the properties of the formulation. Such additives are well known to those skilled in the art.

In the present application, the term "cancer" generally refers to or describes a physiological condition in mammals that is typically characterized by dysregulation of cell proliferation or survival. In the present application, hyperproliferative diseases referred to as cancers include, but are not limited to, solid tumors such as those occurring in the breast, respiratory tract, brain, reproductive organs, digestive tract, urethra, eye, liver, skin, head and neck, thyroid, and parathyroid glands, and their distant metastases. Such diseases also include lymphomas, sarcomas, and leukemias. Examples of breast cancer include, but are not limited to, invasive ductal carcinoma, invasive lobular carcinoma, ductal carcinoma in situ, and lobular carcinoma in situ. Examples of cancers of the respiratory tract include, but are not limited to, small cell lung cancer and non-small cell lung cancer, as well as bronchial adenoma and pleuropulmonary blastoma. Examples of brain cancers include, but are not limited to, brainstem and hypothalamic keratinomas, cerebellar and cerebral astrocytomas, medulloblastomas, ependymomas, and neuroectodermal and pineal tumors. Tumors of the male genitalia include, but are not limited to, prostate and testicular cancers. Tumors of the female genitalia include, but are not limited to, cancers of the endometrium, cervix, ovary, vagina, and vulva, and uterine tumors. Gastrointestinal tumors include, but are not limited to, cancers of the anus, colon, colorectum, esophagus, gallbladder, stomach, pancreas, rectum, small intestine, and salivary glands. Tumors of the urethra include, but are not limited to, cancers of the bladder, penis, kidney, renal pelvis, ureter, and urethra. Eye cancers include, but are not limited to, intraocular melanoma and retinoblastoma. Examples of liver cancer include, but are not limited to, hepatocellular carcinoma (hepatocellular carcinoma with or without the fibrolamellar variation), cholangiocarcinoma (intrahepatic biliary cancer), and mixed hepatocellular cholangiocarcinoma. Skin cancers include, but are not limited to, squamous cell carcinoma, Kaposi's sarcoma, malignant melanoma, Merkel cell skin cancer, and non-melanoma skin cancer. Head and neck cancers include, but are not limited to, cancers of the larynx/hypopharynx/nasopharynx/oropharynx, and cancers of the lips and mouth. Lymphatic cancers include, but are not limited to, AIDS-related lymphoma, non-Hodgkin's lymphoma, cutaneous T-cell lymphoma, Hodgkin's disease, and central nervous system lymphoma. Sarcomas include, but are not limited to, soft tissue sarcomas, osteosarcomas, malignant fibrous histiocytomas, lymphosarcomas, and rhabdomyosarcomas. Leukemias include, but are not limited to, acute myeloid leukemia, acute lymphocytic leukemia, chronic lymphocytic leukemia, chronic myeloid leukemia, and hairy cell leukemia.

When referring to pharmaceutical compositions, "pharmaceutically acceptable carrier" is used to refer to substances such as solid or liquid diluents, fillers, antioxidants, and stabilizers, which are safe for administration, and which are suitable for administration to humans and/or animals without undue adverse side effects, while being suitable for maintaining the viability of the drug or active agent therein.

A "therapeutically effective amount" refers to an amount of an active compound or cell sufficient to elicit the biological or medical response desired by a clinician in a subject. The "therapeutically effective amount" can be determined by those skilled in the art according to the administration route, the subject's body weight, age, condition and other factors. For example, a typical daily dose may range from 0.01 mg to 100 mg of active ingredient per kg of body weight for an antibody. The administration mode of the antibody of the present application includes but is not limited to injection, such as intravenous, intramuscular, intraarterial, subcutaneous, intraperitoneal injection and the like.

The term "and/or" should be understood as any one of the alternatives or both of the alternatives.

As used in the present application, the term "comprise" or "include" means including stated elements, integers or steps, but not excluding any other elements, integers or steps. In the present application, when the term "comprise" or "include" is used, unless otherwise specified, situations consisting of the mentioned elements, integers or steps are also covered. For example, when referring to an antibody variable region that "comprises" a particular sequence, it is also intended to encompass an antibody variable region that consists of that particular sequence.

In the present application, the term "about" generally refers to a variation with the range of 0.5%-10% above or below a specified value, such as a variation within the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

### Anti-CD5 antibody

The present invention uses fully human phages for antibody selection to directly obtain fully human monoclonal antibodies. Compared with traditional hybridoma technology, it dispenses with the difficult step of humanizing murine antibodies. Also, fully human antibodies have lower immunogenicity than humanized murine antibodies and have better potential in antibody drugs (including monoclonal antibodies, biclonal antibodies, ADC, etc.), cell therapy drugs (including CAR-T, CAR-NK, etc.), detection reagents and other applications.

The present invention uses the method of antigen protein panning, which can efficiently enrich antibodies that simultaneously bind to recombinant CD5 and natural CD5 on the cell membrane, greatly reducing the difficulty of later antibody selection and improving efficiency.

We used a large-capacity phage antibody library to select fully human CD5-specific antibodies, and evaluated the specificity of these antibodies at the phage level by ELISA and FACS experiments. Finally, we obtained several fully human antibody clones with good specificity.

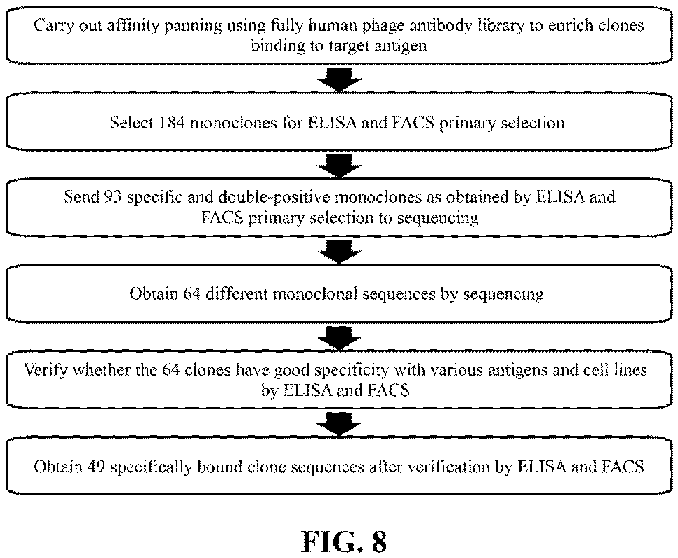

With different antibody libraries and through recombinant CD5 protein panning, we selected 184 monoclonal antibodies for preliminary selection by enzyme-linked immunosorbent assay (ELISA) and flow cytometry (FACS), among which 93 clones specifically bound to CD5-Fc-Bio protein and CD5-expressing positive cell Jurkat, but not to control protein CD19-Fc-Bio and CD5-expressing negative cell Raji. After sequencing, 64 different monoclonal sequences were obtained. Subsequently, we identified these 64 antibodies through flow cytometry (FACS) with various CD5 positive (Jurkat, CCRF-CEM) and negative (Raji, NALM6) cell lines, and through enzyme-linked immunosorbent assay (ELISA) with CD5 proteins (Kactus-CD5- Fc-Bio, Acro-CD5-his, SB-CD5-his-Bio), non-related proteins (Kactus-BAFFR-his-Bio, Kactus-CD19-FC-Bio, SA) from different companies, of which 49 clones showed good binding and specificity on multiple cell lines and various protein antigens. The acquisition of these clones laid the foundation for the subsequent development of fully human CD5 CAR-T products or antibody drugs. The overall project flow is shown in Fig. 8.

### Chimeric antigen receptor

In the present application, the CAR may comprise an extracellular domain specifically binding to CD5, a transmembrane domain, an intracellular co-stimulatory signaling domain, and an intracellular signaling domain. In the present application, the extracellular domain of the CAR may comprise the single-chain scFv antibody of the present invention. For example, the single chain antibody may be linked to the transmembrane domain via a hinge region, such as CD8 hinge. In the present application, the CAR can be used to transduce immune effector cells (such as T-cells) and be expressed on the cell surface. Therefore, the present application can also provide T-cells expressing the chimeric antigen receptor, and the use of the T-cells and/or the CAR in the preparation of drugs for treating CD5-related diseases.

In the present application, the chimeric antigen receptor (CAR) may comprise a CD5-binding domain, a transmembrane domain, a co-stimulatory domain and an intracellular signaling domain. In the present application, the CD5 binding domain may comprise an antibody specifically binding to CD5 or a fragment thereof. The antibody may comprise a heavy chain complementarity determining region 1 (HCDR1), a heavy chain complementarity determining region 2 (HCDR2) and a heavy chain complementarity determining region 3 (HCDR3), and the amino acid sequences of HCDR1-3 are set forth in SEQ ID NOs: 43-45, 49-51, 55-57, 61-63, 67-69 or 73-75. The antibody may also comprise a light chain complementarity determining region 1 (LCDR1), a light chain complementarity determining region 2 (LCDR2) and a light chain complementarity determining region 3 (LCDR3), and the amino acid sequences of LCDR1-3 are set forth in SEQ ID NOs: 46-48, 52-54, 58-60, 64-66, 70-72 or 76-78. In the present application, the antibody may comprise a heavy chain variable region, and the amino acid sequence of the heavy chain variable region is set forth in SEQ ID NO: 30, 36, 42, 90, 93 or 96. In the present application, the antibody may comprise a light chain variable region, and the amino acid sequence of the light chain variable region is set forth in SEQ ID NO: 29, 35, 41, 89, 92 or 95.

In the present application, the antibody may be a single chain antibody. In some embodiments, the antibody may comprise an amino acid sequence as set forth in SEQ ID NO: 28, 34, 40, 88, 91 or 94 or a functional variant thereof. For example, the single-chain antibody may include scFv10 having a sequence as set forth in SEQ ID NO: 28; the single-chain antibody may include scFv32 having a sequence as set forth in SEQ ID NO: 34; the single-chain antibody may include scFv35 having a sequence as set forth in SEQ ID NO: 40; the single-chain antibody may include scFv6 having a sequence as set forth in SEQ ID NO: 88; the single-chain antibody may include scFv7 having a sequence as set forth in SEQ ID NO: 91; and the single-chain antibody may include scFv9 having a sequence as set forth in SEQ ID NO: 94.

For example, the single-chain antibody of the present application may be scFv10 (originally numbered as clone 10), the sequence of which is set forth in SEQ ID NO: 28. The amino acid sequences of LCDR1-3 of the single-chain antibody scFv10 are set forth in SEQ ID NO: 46, SEQ ID NO: 47 and SEQ ID NO: 48, respectively; the amino acid sequence of VL is set forth in SEQ ID NO: 29; the amino acid sequences of HCDR1-3 are set forth in SEQ ID NO: 43, SEQ ID NO: 44 and SEQ ID NO: 45, respectively; and the amino acid sequence of VH is set forth in SEQ ID NO: 30. The single-chain antibody of the present application may be scFv32 (originally numbered as clone 32), the sequence of which is set forth in SEQ ID NO: 34. The amino acid sequences of LCDR1-3 of the single-chain antibody scFv32 are set forth in SEQ ID NO: 52, SEQ ID NO: 53 and SEQ ID NO: 54, respectively; the amino acid sequence of VL is set forth in SEQ ID NO: 35; the amino acid sequences of HCDR1-3 are set forth in SEQ ID NO: 49, SEQ ID NO: 50 and SEQ ID NO: 51, respectively; and the amino acid sequence of VH is set forth in SEQ ID NO: 36. The single-chain antibody of the present application may be scFv35 (originally numbered as clone 35), the sequence of which is set forth in SEQ ID NO: 40. The amino acid sequences of LCDR1-3 of the single-chain antibody scFv35 are set forth in SEQ ID NO: 58, SEQ ID NO: 59 and SEQ ID NO: 60, respectively; the amino acid sequence of VL is set forth in SEQ ID NO: 41; the amino acid sequences of HCDR1-3 are set forth in SEQ ID NO: 55, SEQ ID NO: 56 and SEQ ID NO: 57, respectively; and the amino acid sequence of VH is set forth in SEQ ID NO: 42. The single-chain antibody of the present application may be scFv6 (originally numbered as clone 6), the sequence of which is set forth in SEQ ID NO: 88. The amino acid sequences of LCDR1-3 of the single-chain antibody scFv6 are set forth in SEQ ID NO: 64, SEQ ID NO: 65 and SEQ ID NO: 66, respectively; the amino acid sequence of VL is set forth in SEQ ID NO: 89; the amino acid sequences of HCDR1-3 are set forth in SEQ ID NO: 61, SEQ ID NO: 62 and SEQ ID NO: 63, respectively; and the amino acid sequence of VH is set forth in SEQ ID NO: 90. The single-chain antibody of the present application may be scFv7 (originally numbered as clone 7), the sequence of which is set forth in SEQ ID NO: 91. The amino acid sequences of LCDR1-3 of the single-chain antibody scFv7 are set forth in SEQ ID NO: 70, SEQ ID NO: 71 and SEQ ID NO: 72, respectively; the amino acid sequence of VL is set forth in SEQ ID NO: 92; the amino acid sequences of HCDR1-3 are set forth in SEQ ID NO: 67, SEQ ID NO: 68 and SEQ ID NO: 69, respectively; and the amino acid sequence of VH is set forth in SEQ ID NO: 93. The single-chain antibody of the present application may be scFv9 (originally numbered as clone 9), the sequence of which is set forth in SEQ ID NO: 94. The amino acid sequences of LCDR1-3 of the single-chain antibody scFv9 are set forth in SEQ ID NO: 76, SEQ ID NO: 77 and SEQ ID NO: 78, respectively; the amino acid sequence of VL is set forth in SEQ ID NO: 95; the amino acid sequences of HCDR1-3 are set forth in SEQ ID NO: 73, SEQ ID NO: 74 and SEQ ID NO: 75, respectively; and the amino acid sequence of VH is set forth in SEQ ID NO: 96.

The CAR of the present application may include a transmembrane domain, and the transmembrane domain may comprises a polypeptide selected from the following proteins: α, β or ζ chains of T-cell receptors, CD28, CD3e, CD45, CD4, CD5, CD8a, CD9, CD16, CD22, CD33, CD37, CD64, CD80, CD86, CD134, CD137, and CD154. In some embodiments, the transmembrane domain may comprise an amino acid sequence as set forth in SEQ ID NO: 6 or a functional variant thereof. For example, the transmembrane domain of the present application may include CD8a, the sequence of which is set forth in SEQ ID NO: 6.

In the present application, the co-stimulatory domain may comprise a polypeptide selected from the following proteins: CD28, 4-1BB, OX40 and ICOS. In the present application, the co-stimulatory domain may comprise an amino acid sequence as set forth in SEQ ID NO: 8 or a functional variant thereof.

The CAR described in the present application may include an intracellular signaling domain, which can comprise a signaling domain from CD3ζ. In the present application, the intracellular signaling domain may comprises an amino acid sequence as set forth in SEQ ID NO: 10 or a functional variant thereof.

The CAR of the present application may include a hinge region, and the hinge region may connect the antibody and the transmembrane domain. In the present application, the hinge region may comprise an amino acid sequence as set forth in SEQ ID NO: 4 or a functional variant thereof.

The CAR of the present application may include a signal peptide, and for example, the signal peptide may be located at the N-terminus of the extracellular domain that specifically binds to CD5. The signal peptide may comprise an amino acid sequence as set forth in SEQ ID NO: 2 or a functional variant thereof. For example, the signal peptide may be CD8a signal peptide, the sequence of which is set forth in SEQ ID NO: 2.

In the present application, the CAR can also be linked to a cleaving peptide. In the present application, the cleaving peptide may comprise an amino acid sequence derived from a T2A peptide. In the present application, the cleaving peptide may comprise an amino acid sequence as set forth in SEQ ID NO: 12 or a functional variant thereof. For example, the cleaving peptide can be T2A, the sequence of which is set forth in SEQ ID NO: 12.

In the present application, the CAR can also be connected to an EGFRt fragment, and the EGFRt fragment can be used for signal detection, or used as a molecular switch for CAR-T cells.

In the present application, the CAR may comprise an amino acid sequence as set forth in SEQ ID NO: 22-24, 98, 100 or 102 or a functional variant thereof. For example, the CAR can be selected from CAR001, the sequence of which is set forth in SEQ ID NO: 22. For another example, the CAR can be selected from CAR002 having a sequence as set forth in SEQ ID NO: 23; the CAR can be selected from CAR003 having a sequence as set forth in SEQ ID NO: 24; the CAR can be selected from CAR004 having a sequence as set forth in SEQ ID NO: 98; the CAR can be selected from CAR005 having a sequence as set forth in SEQ ID NO: 100; and the CAR can be selected from CAR006 having a sequence as set forth in SEQ ID No: 102.

In some embodiments, the CAR of the present application may sequentially include a CD5 binding domain, a transmembrane domain, a co-stimulatory domain, and an intracellular signaling domain from the N-terminus. Among others, the CAR may include a CD5 binding domain, and the sequence of the CD5 binding domain is set forth in SEQ ID NO: 28. Among others, the CD5 binding domain may include HCDR1-3, whose sequences are set forth in SEQ ID NOs: 43-45, respectively; and, the CD5 binding domain may include LCDR1-3, whose sequences are set forth in SEQ ID NOs: 46-48, respectively. For example, the CAR may include CAR001 or the CAR of the present application having the same LCDR1-3 and HCDR1-3 as CAR001. The CD5 binding domain may include a heavy chain variable region having a sequence as set forth in SEQ ID NO: 30; and, the CD5 binding domain may also include a light chain variable region having a sequence as set forth in SEQ ID NO: 29. For example, the CAR may include CAR001 or the CAR of the present application having the same light chain variable region and heavy chain variable region as CAR001. A linker peptide may also be included between the light chain variable region and the heavy chain variable region, the sequence of which is set forth in SEQ ID NO: 18. For example, the CAR may include CAR001 or the CAR of the present application having the same linker peptide as CAR001. The transmembrane domain may include a transmembrane domain from CD8a, the sequence of which may be as set forth in SEQ ID NO: 6. For example, the CAR may include CAR001 or the CAR of the present application having the same transmembrane domain as CAR001. The co-stimulatory domain may comprise a co-stimulatory structure from 4-1BB, the sequence of which may be as set forth in SEQ ID NO: 8. For example, the CAR may include CAR001 or the CAR of the present application having the same co-stimulatory domain as CAR001. The intracellular signaling domain may comprise a signaling domain from CD3ζ, the sequence of which is set forth in SEQ ID NO: 10. For example, the CAR may include CAR001 or the CAR of the present application having the same intracellular signaling domain as CAR001. The CAR can also include a hinge region, which may be located at the C-terminus of the CD5 binding domain and at the N-terminus of the transmembrane domain, and the sequence of the hinge region may be as set forth in SEQ ID NO: 4, for example. For example, the CAR can include CAR001 or the CAR of the present application having the same hinge region as CAR001. The CAR can also be linked to a signal peptide, which can be located at the N-terminus of the CAR, and the sequence of the signal peptide can be as set forth in SEQ ID NO: 2.

The CAR can also be linked to a cleaving peptide, such as T2A. The cleaving peptide can be located at the C-terminus of the intracellular signal transduction domain, the sequence of which can be as set forth in SEQ ID NO: 12. The CAR can also be linked to CSF2RA signal peptide, which can be located before EGFRt, and the sequence of the CSF2RA signal peptide can be as set forth in SEQ ID NO: 14, for example. The CAR can also be linked to a marker detection signal, which can be located at the C-terminus of the CAR (or, the cleaving peptide). The marker detection signal can be selected from the group consisting of GFP, RFP, YFP or EGFRt, and the sequence of EGFRt can be as set forth in SEQ ID NO: 16, for example.

For example, the CAR of the present application can be CAR001, the amino acid sequences of its LCDR1-3 are set forth in SEQ ID NO: 46, SEQ ID NO: 47 and SEQ ID NO: 48, respectively; the amino acid sequence of its VL is set forth in SEQ ID NO: 29; the amino acid sequences of its HCDR1-3 are set forth in SEQ ID NO: 43, SEQ ID NO: 44 and SEQ ID NO: 45, respectively; the amino acid sequence of its VH is set forth in SEQ ID NO: 30; the sequence of the linker peptide between VH and VL is set forth in SEQ ID NO: 18; its hinge region is set forth in SEQ ID NO: 4; its transmembrane domain is set forth in SEQ ID NO: 6; its co-stimulatory domain is 4-1BB co-stimulatory domain, as set forth in SEQ ID NO: 8; and its CD3ζ intracellular signaling domain is set forth in SEQ ID NO: 10. The CAR001 further comprises a cleaving peptide as set forth in SEQ ID NO: 12, a CSF2RA signal peptide as set forth in SEQ ID NO: 14, and EGFRt as set forth in SEQ ID NO: 16; and a CD8a signal peptide as set forth in SEQ ID NO: 2.

For example, the CAR of the present application can be CAR002, the amino acid sequences of its LCDR1-3 are set forth in SEQ ID NO: 52, SEQ ID NO: 53 and SEQ ID NO: 54, respectively; the amino acid sequence of its VL is set forth in SEQ ID NO: 35; the amino acid sequences of its HCDR1-3 are set forth in SEQ ID NO: 49, SEQ ID NO: 50 and SEQ ID NO: 51, respectively; the amino acid sequence of its VH is set forth in SEQ ID NO: 36; the sequence of the linker peptide between VH and VL is set forth in SEQ ID NO: 18; its hinge region is set forth in SEQ ID NO: 4; its transmembrane domain is set forth in SEQ ID NO: 6; its co-stimulatory domain is 4-1BB co-stimulatory domain, as set forth in SEQ ID NO: 8; and its CD3ζ intracellular signaling domain is set forth in SEQ ID NO: 10. The CAR002 further comprises a cleaving peptide as set forth in SEQ ID NO: 12, a CSF2RA signal peptide as set forth in SEQ ID NO: 14, and EGFRt as set forth in SEQ ID NO: 16; and a CD8a signal peptide as set forth in SEQ ID NO: 2.

For example, the CAR of the present application can be CAR003, the amino acid sequences of its LCDR1-3 are set forth in SEQ ID NO: 58, SEQ ID NO: 59 and SEQ ID NO: 60, respectively; the amino acid sequence of its VL is set forth in SEQ ID NO: 41; the amino acid sequences of its HCDR1-3 are set forth in SEQ ID NO: 55, SEQ ID NO: 56 and SEQ ID NO: 57, respectively; the amino acid sequence of its VH is set forth in SEQ ID NO: 42; the sequence of the linker peptide between VH and VL is set forth in SEQ ID NO: 18; its hinge region is set forth in SEQ ID NO: 4; its transmembrane domain is set forth in SEQ ID NO: 6; its co-stimulatory domain is 4-1BB co-stimulatory domain, as set forth in SEQ ID NO: 8; and its CD3ζ intracellular signaling domain is set forth in SEQ ID NO: 10. The the CAR003 further comprises a cleaving peptide as set forth in SEQ ID NO: 12, a CSF2RA signal peptide as set forth in SEQ ID NO: 14, and EGFRt as set forth in SEQ ID NO: 16; and a CD8a signal peptide as set forth in SEQ ID NO: 2.

For example, the CAR of the present application can be CAR004, the amino acid sequences of its LCDR1-3 are set forth in SEQ ID NO: 64, SEQ ID NO: 65 and SEQ ID NO: 66, respectively; the amino acid sequence of its VL is set forth in SEQ ID NO: 89; the amino acid sequences of its HCDR1-3 are set forth in SEQ ID NO: 61, SEQ ID NO: 62 and SEQ ID NO: 63, respectively; the amino acid sequence of its VH is set forth in SEQ ID NO: 90; the sequence of the linker peptide between VH and VL is set forth in SEQ ID NO: 18; its hinge region is set forth in SEQ ID NO: 4; its transmembrane domain is set forth in SEQ ID NO: 6; its co-stimulatory domain is 4-1BB co-stimulatory domain, as set forth in SEQ ID NO: 8; and its CD3ζ intracellular signaling domain is set forth in SEQ ID NO: 10. The CAR004 further comprises a cleaving peptide as set forth in SEQ ID NO: 12, a CSF2RA signal peptide as set forth in SEQ ID NO: 14, and EGFRt as set forth in SEQ ID NO: 16; and a CD8a signal peptide as set forth in SEQ ID NO: 2.

For example, the CAR of the present application can be CAR005, the amino acid sequences of its LCDR1-3 are set forth in SEQ ID NO: 70, SEQ ID NO: 71 and SEQ ID NO: 72, respectively; the amino acid sequence of its VL is set forth in SEQ ID NO: 92; the amino acid sequences of its HCDR1-3 are set forth in SEQ ID NO: 67, SEQ ID NO: 68 and SEQ ID NO: 69, respectively; the amino acid sequence of its VH is set forth in SEQ ID NO: 93; the sequence of the linker peptide between VH and VL is set forth in SEQ ID NO: 18; its hinge region is set forth in SEQ ID NO: 4; its transmembrane domain is set forth in SEQ ID NO: 6; its co-stimulatory domain is 4-1BB co-stimulatory domain, as set forth in SEQ ID NO: 8; and its CD3ζ intracellular signaling domain is set forth in SEQ ID NO: 10. The CAR005 further comprises a cleaving peptide as set forth in SEQ ID NO: 12, a CSF2RA signal peptide as set forth in SEQ ID NO: 14, and EGFRt as set forth in SEQ ID NO: 16; and a CD8a signal peptide as set forth in SEQ ID NO: 2.

For example, the CAR of the present application can be CAR006, the amino acid sequences of its LCDR1-3 are set forth in SEQ ID NO: 76, SEQ ID NO: 77 and SEQ ID NO: 78, respectively; the amino acid sequence of its VL is set forth in SEQ ID NO: 95; the amino acid sequences of its HCDR1-3 are set forth in SEQ ID NO: 73, SEQ ID NO: 74 and SEQ ID NO: 75, respectively; the amino acid sequence of its VH is set forth in SEQ ID NO: 96; the sequence of the linker peptide between VH and VL is set forth in SEQ ID NO: 18; its hinge region is set forth in SEQ ID NO: 4; its transmembrane domain is set forth in SEQ ID NO: 6; its co-stimulatory domain is 4-1BB co-stimulatory domain, as set forth in SEQ ID NO: 8; and its CD3ζ intracellular signaling domain is set forth in SEQ ID NO: 10. The CAR006 further comprises a cleaving peptide as set forth in SEQ ID NO: 12, a CSF2RA signal peptide as set forth in SEQ ID NO: 14, and EGFRt as set forth in SEQ ID NO: 16; a CD8a signal peptide as set forth in SEQ ID NO: 2.

### Nucleic acid, vector, cell, preparation method and composition

In another aspect, the present application provides an isolated nucleic acid molecule encoding the CAR of the present application. The isolated nucleic acid molecule encoding CAR of the present application can comprise a nucleic acid sequence selected from the group consisting of SEQ ID NO: 1, 3, 5, 7, 9, 11, 13, 15, 17, 19-21, 25-27, 31-33, 37-39, 79-87, 97, 99 or 101 or a functional variant thereof. The nucleic acid molecule of the present application can be isolated. For example, it may be produced or synthesized by (i) amplification in vitro, such as amplification by polymerase chain reaction (PCR), (ii) cloning and recombination, (iii) purification, for example by fractionation through enzyme digestion and gel electrophoresis, or (iv) synthesis, for example by chemical synthesis. In some embodiments, the isolated nucleic acid is a nucleic acid molecule prepared by recombinant DNA technology.

In another aspect, the present application provides a vector comprising the nucleic acid molecule. In the present application, the vector can be selected from one or more of plasmids, retroviral vectors and lentiviral vectors. The lentiviral vector of the present application may comprise CAR. For example, lentiviral vector of the present application can comprise a nucleic acid sequence selected from the group consisting of SEQ ID NO: 1, 3, 5, 7, 9, 11, 13, 15, 17, 19-21, 25-27, 31-33, 37-39, 79-87, 97, 99 or 101 or a functional variant thereof. In addition, other genes may be comprised in the vector, such as marker genes that allow selection of the vector in appropriate host cells and under appropriate conditions. In addition, the vector may also comprise expression control elements that permit proper expression of the coding region in an appropriate host. Such control elements are well known to those skilled in the art, and may include, for example, promoters, ribosome binding sites, enhancers, and other control elements that regulate gene transcription or mRNA translation. In some embodiments, the expression control sequences are regulatable elements. The specific structure of the expression control sequence may vary depending on the species or the function of the cell type, but generally includes 5' non-transcribed sequences and 5' and 3' non-translated sequences involved in the initiation of transcription and translation, respectively, such as TATA box, capping sequence, and CAAT sequence. For example, the 5' non-transcribed expression control sequence may comprise a promoter region which may comprise a promoter sequence for transcriptional control of the functionally linked nucleic acid. One or more nucleic acid molecules of the present application can be operably linked to the expression control element. Such vectors may include, for example, plasmids, cosmids, viruses, phages, or other vectors commonly used in, for example, genetic engineering. For example, the vector is an expression vector, including vector scFv plasmid and/or CAR plasmid.

In another aspect, the present application provides an immune effector cell comprising the CAR, the nucleic acid molecule, or the vector of the present application. In the present application, the immune effector cells may be mammalian cells. In the present application, the immune effector cells may be selected from T lymphocytes and natural killer (NK) cells.

In another aspect, the present application provides a method for preparing immune effector cells, which comprises knocking out the CD5 gene of the immune effector cells, and introducing the vector of the present application into the immune effector cells. For example, the vectors of the present application can be introduced into the immune effector cells, such as T lymphocytes or natural killer (NK) cells. In some embodiments, each type of cells or each cell may comprise one or more of the vectors of the present application. In some embodiments, each type of cells or each cell may comprise multiple (e.g., 2 or more) or multiple types of (e.g., 2 or more types of) vectors of the present application. In the present application, for introducing the vector into immune effector cells, the vector of the present application can be introduced into the cells by methods known in the art. For example, immune effector cells can be transfected with retroviral vectors to integrate the viral genome with CAR molecules into the host genome to ensure long-term and stable expression of the target gene. For another example, transposons can be used and introduced into target cells through CAR (transposon)-carrying plasmids and transposasecarrying plasmids . As another example, CAR molecules can be added to the genome through gene editing (such as CRISPR/Cas9). In the present application, the vector carrying the CAR molecule of the present application can be introduced into the cells by methods known in the art, such as electroporation, and liposome transfection (lipofectamine 2000, Invitrogen).

In another aspect, the present application provides a pharmaceutical composition, which comprises the immune effector cells and a pharmaceutically acceptable adjuvant. The pharmaceutically acceptable adjuvant may include buffers, antioxidants, preservatives, low molecular weight polypeptides, proteins, hydrophilic polymers, amino acids, sugars, chelating agents, counterions, metal complexes and/or non-ionic surfactants, etc. In the present application, the pharmaceutical composition can be formulated for administration orally, intravenously (e.g., intravenous injection, I.V.), intramuscularly (e.g., intramuscular injection, I.M.), in situ at the site of the tumor, by inhalation, rectally, vaginally, transdermally or via a subcutaneous depot.

### Pharmaceutical use

In another aspect, the present application provides use of the antibody, the CAR, the nucleic acid molecule, the vector, or the immune effector cell in the preparation of a drug for treating a disease or condition associated with the expression of CD5. In the present application, the disease or condition associated with the expression of CD5 is a cancer or malignancy. In some embodiments, the cancer or malignancy may be selected from a T-cell malignancy or a B-cell malignancy. Among them, the T-cell malignancy can be selected from T-cell acute lymphocytic leukemia (T-ALL), T-cell lymphoma (TCL) (such as peripheral T-cell lymphoma, cutaneous T-cell lymphoma (CTCL), T-cell non- Hodgkin's Lymphoma (T-NHL)); the malignant B-cell neoplasm may be selected from chronic lymphocytic leukemia (B-CLL) (e.g. hairy cell leukemia cell (HCL)), mantle cell lymphoma (B-MCL), diffuse large B-cell lymphoma (DLBCL).

In another aspect, the present application provides use of the antibody, the CAR, the nucleic acid molecule, the vector, or the immune effector cell in the treatment of a disease or condition associated with the expression of CD5.

In another aspect, the present application provides a method for treating a disease or condition associated with the expression of CD5, which comprises administering the antibody, the CAR, the nucleic acid molecule, the vector, or the immune effector cell to a patient.

Without intending to be bound by any theory, the following examples are only to illustrate the working mode of the chimeric antigen receptor, vector, cell, and composition of the present application, and are not intended to limit the scope of the invention of the present application.

### Research overview

The researchers use fully human phages for antibody selection to directly obtain fully human monoclonal antibodies. Compared with traditional hybridoma technology, it dispenses with the difficult step of humanizing murine antibodies, and fully human antibodies have lower immunogenicity than humanized murine antibodies, providing better potential in the development of CAR-T. After obtaining antibody clones that specifically bind to the cell surface CD5 antigen and CD5 recombinant protein, the applicant conducted in-depth research, constructed these clones and the control clone H65 onto the second-generation CAR structure, then packaged them with lentivirus and transfected them into T-cells, and at the level of CAR-T cells, selected fully human CD5 antibody clones and candidate CAR-T molecules with stronger functions than the control clone H65 in terms of target cell activation and killing, and target cell stimulation and proliferation.

In addition, according to the literature reports, in most constructed tumor animal models, reemerging tumor cells retain CD5 expression, which suggests that tumor recurrence is not due to the loss of antigen, and that failure to eradicate all xenografts is due to the poor persistence of CD5 CAR-T cells in mice^{[13]}. The present application uses CRISPR technology to knock out the CD5 gene in the jurkat cell line, and successfully selects sgRNAs with high knockout efficiency. The experimental results show that CD5-knockout CAR-T cells can expand normally, and maintain the function of killing CD5-positive target cells, while minimizing the self-activation and suicide of CD5 CAR-T, ensuring its persistence and effectiveness in clinical verification.

### Examples

### Example 1. Enrichment of specific antibody clones targeting CD5 protein from phage antibody library by affinity panning

Appropriate negative panning and positive panning strategies were used to enrich the specific antibody clones we need from the phage antibody library.

### Construction of phage antibody library

The phage antibody libraries we constructed include natural libraries, semi-synthetic libraries and single-domain libraries. The semi-synthetic phage antibody library, used together with the natural library, solves the problem that the natural library may lack CD5 high-affinity antibody clones. The single-domain phage antibody library is an antibody library composed only of the variable region amino acids of heavy chain antibodies, which have similar or higher specificity and affinity than traditional antibodies although their molecular weight is only 12-15 kDa. In addition, single-domain antibodies have attracted much attention because of their stable physical and chemical properties, high affinity, easy recombinant expression and preparation, and easy combination with other target or epitope antibodies.

### CD5 protein panning

Using CD5-Fc-Bio as the positive panning protein and CD19-Fc-Bio as the negative panning protein, multiple rounds of panning were performed to obtain a phage pool enriched with the target antibody clones. The experimental steps are briefly described as follows:
1) firstly blocking SA magnetic beads with blocking buffer for 2 h, and then binding the target antigen (CD5-Fc-Bio) to the blocked SA magnetic beads;
2) adding a phage library (containing 5×10¹² phage particles) and incubating it with the control antigen, so as to deduct phage antibody clones that do not specifically bind to Fc tags;
3) after incubation, transferring the supernatant to SA magnetic beads bound to the target antigen, and continuing to incubate to allow the phage to bind to the target antigen;
4) washing the magnetic beads with wash buffer to wash away unbound phage;
5) eluting the positive phage from the target antigen with an eluent, and adding a neutralizing solution to neutralize;
6) re-infecting the host strain XL1-blue with the eluted phage, and amplifying the recovered phage; leaving a small amount of sample for gradient dilution, infecting the host bacteria with the sample, applying the infected host bacteria to Amp resistance plates, and calculating the number of recovered phages;
7) repeating steps 1) to 6), wherein usually three rounds of panning are needed until a significant increase in the recovery rate of phages (number of eluted phages/number of input phages) is observed.

The enriched phage pool can be used for subsequent monoclonal selection and ELISA/FACS selection.

### Main materials and reagents:

Fully human phage antibody library, including natural library, semi-synthetic library and single domain library;
Helper phage KO7, Thermo/Invitrogen, 18311019;
Recombinant biotinylated Human CD5-Fc Protein, Kactus, CD5-HM401;
Recombinant biotinylated Human CD19 Protein-Fc, Kactus, CD2-HE121
BeaverBeads^{™} Streptavidin, Beaver Bio, 22307-10;
High binding ELSIA plate, Costar, #3590
Blocking buffer: PBS+3% BSA
Wash buffer: PBS+ 0.1% Tween20
Eluent: 0.2M Glycine, pH2.2
Neutralizing solution: 1M Tris, pH9.1

### Experimental results:

Using different antibody libraries, through 3 rounds of protein panning, a significant increase in the recovery rate was observed in each round of panning (Table 1), proving that antibody clones were effectively enriched.

**Table 1 Protein panning experiment results**

| Antibody library | Round | Recovery rate | Enrichment factor |
|---|---|---|---|
| XL-SD-1 | 1st | 3.34E-05 | / |
| | 2nd | 7.74E-05 | 2.32 |
| | 3rd | 4.54E-03 | 58.66 |
| XI,-NVH | 1st | 4.54E-05 | / |
| | 2nd | 7.46E-05 | 1.64 |
| | 3rd | 1.20E-03 | 16.09 |
| XL-V1 | 1st | 4. 14E-05 | / |
| | 2nd | 3.46E-05 | 0.84 |
| | 3rd | 1.20E-03 | 34.68 |
| XL-V2 | 1st | 3.60E-05 | / |
| | 2nd | 4.80E-05 | 1.33 |
| | 3rd | 2.06E-03 | 42.92 |

It can be seen that after three rounds of panning, different antibody libraries were enriched (the recovery rate of the third round was significantly higher than that of the previous round).

### Example 2. Selection of specific clones from enriched phage pools by enzyme-linked immunosorbent assay (ELISA) and flow cytometry (FACS)

### Purpose and principle:

The phage pool enriched by the affinity panning step contains phage antibodies of various properties: specific clones, non-specific clones, and negative clones. In order to obtain specific clones, we need to isolate monoclones, package the monoclones into monoclonal phages, and conduct preliminary selection on a large number of monoclones by enzyme-linked immunoassay (ELISA) and flow cytometry (FACS) to select monoclones specifically binding to both CD5 protein and CD5 positive cell line Jurkat. The specific monoclones are further determined by DNA sequencing to determine unique antibody sequences contained therein.

In the primary selection by ELISA, through the binding of streptavidin and biotin, the biotinylated target protein (CD5-Fc-Bio) is closer to the natural antigen conformation in the reaction solution. Those that only bind to CD5-Fc-Bio but not to the control antigen CD19-Fc-Bio are identified as specific clones. FACS primary selection is carried out using the positive cell line Jurkat with high expression of CD5 and the cell line Raji negative for CD5, and those that only bind to Jurkat cells but not to Raji cells are identified as specific clones. Through ELISA and FACS, we can obtain candidate antibodies that can bind to recombinantly expressed CD5 proteins and recognize the natural CD5 molecules on the cell surface for subsequent further selection.

Brief steps of ELISA primary screening experiment:
1) culturing and packaging monoclonal phages in a deep-well 96-well plate;
2) diluting strepavidin to 2 µg/mL with PBS, adding it at 100 µL/well to a high-binding ELISA plate for binding at room temperature for 2 h;
3) discarding the coating solution, and adding 250 µL of blocking buffer to each well for blocking overnight at 4°C;
4) washing the plate twice with 250 µL of wash buffer;
5) diluting the biotin-labeled target protein and control protein to 2 µg/mL with PBS, adding the protein at 100 µg/well to the ELISA plate pre-coated with strepavidin for binding at room temperature for 1 h;
6) washing the plate twice with 250 µL of wash buffer;
7) adding 100 µL of the phage supernatant cultured in step 1) to the wells coated with the target antigen for binding at room temperature for 2 h;
8) washing the plate 4 times with 250 µL of wash buffer;
9) adding mouse anti-M13 primary antibody diluted at 1:2000, 100 µL/well, and incubating at room temperature for 45 min;
10) washing the plate 4 times with 250 µL of wash buffer;
11) adding 1:2000 diluted HRP Donkey anti-mouse IgG,100 µL/well, and incubating at room temperature for 45 min;
12) washing the plate 6 times with 250 µL of wash buffer;
13) adding 100 µL of TMB chromogenic substrate for color development for 5-10 min;
14) adding 100 µL of 2M H₂SO₄ to terminate the reaction, and reading the result on a microplate reader.

Brief steps of FACS primary screening experiment:
1) culturing and packaging monoclonal phages in a deep-well 96-well plate;
2) washing Raji and Jurkat cells twice with PBS, resuspending the cells in PBS to a concentration of 1×10⁷/mL, and dispensing at 50 µL into 96- well deep-well plates;
3) adding 50 µL of packaged monoclonal phage to each well, mixing well, and binding at 4°C for 2 h;
4) washing twice with 200 µL of PBS;
5) adding mouse anti-M13 primary antibody diluted at 1:2000, 100 µL/well, well mixing by pipetting, and then incubating at room temperature for 45 min;
6) washing twice with 200 µL of PBS;
7) adding 1:300 diluted FITC horse anti mouse-IgG (H+L), 100 µL/well, well mixing by pipetting, and then incubating at room temperature for 45 min;
8) washing twice with 200 µL of PBS; finally resuspending the cells with 200 µL of PBS;
9) detecting the fluorescence intensity of the FITC channel of the sample on a flow cytometer, and analyzing the results.

### Main materials and reagents:

Helper phage KO7, Thermo/Invitrogen, 18311019
Streptavidin, Pierce, 21125
Recombinant biotinylated Human CD5 Protein , Kactus, CD5-HM401;
Recombinant biotinylated Human CD19 Protein, Kactus, CD2-HE121
High binding ELSIA plate, Costar, #3590
Corning 96 Well Clear Round Bottom TC-Treated Microplate, Costar, #3799
Blocking buffer: PBS+3% BSA
Wash buffer: PBS+ 0.1% Tween20
Soluble one-component TMB substrate solution, Tiangen, PA-107-02
Anti-M13 phage Coat Protein g8p antibody, abcam, ab9225
HRP Goat anti-mouse IgG(minimal x-reactivity) Antibody, Biolegend, 405306
FITC horse anti mouse-IgG(H+L), Vector, FI2000

### Experimental results:

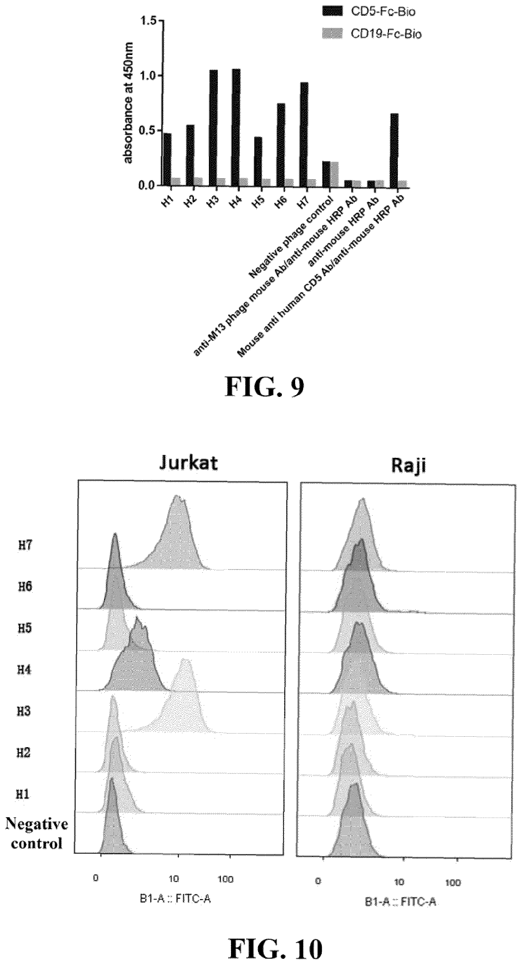

Monoclones were randomly selected from the enriched phage antibody pool, packaged into phages, and the binding of the monoclonal phages to CD5-Fc-Bio protein and control protein CD19-Fc-Bio was detected by phage ELISA to find CD5-specific phage antibody clones. The ELISA results of some clones are shown in Fig. 9. It can be seen from the figure that clones H1, H2, H3, H4, H5, H6 and H7 had strong binding to the target antigen CD5 (CD5-Fc-Bio), and did not bind to the control antigen CD19-Fc-Bio, showing good specificity. Negative phage control is a negative control phage antibody clone that binds to neither the target antigen nor the control antigen;anti-M13 phage mouse Ab/anti-mouse HRP Ab is a negative antibody control with only the primary and secondary antibodies; anti-mouse HRP Ab is a negative antibody control with only the secondary antibody, and it binds to neither the target antigen nor the control antigen; Mouse anti-human CD5 Ab/anti-mouse HRP Ab is a positive antibody control of the target antigen (CD5-Fc-Bio) which binds to the target antigen and does not bind to the control antigen.

The results of FACS primary selection of antibody clones corresponding to ELISA are shown in Fig. 10. Among them, H3, H4 and H7 clones bind to Jurkat but not to Raji cells, and they are specific clones; other clones are negative clones (which bind to neither of the two kinds of cells).

Through ELISA detection and FACS primary selection, we obtained a total of 93 ELISA and FACS double-positive clones with good specificity, and then we sequenced the 93 double-positive clones with good specificity, and obtained 64 different monoclone sequences after sequencing. Then, these 64 monoclones with different sequences were further tested for the binding specificity of the candidate clones through FACS identification with multiple cell lines and ELISA identification with various antigens.

### Example 3. Identification of monoclonal specificity by FACS using multiple cell lines

### Experimental purpose and principle:

Antibodies used for therapy must have very good target specificity, binding only to the target antigen and not to any unrelated antigen; on the other hand, the amino acid sequence of the same antigen on different cell lines will vary (isomers or mutants) or the bound ligands will be different, and it is also necessary to investigate whether our antibodies can bind to various cells positive for the target protein. In order to further analyze the specificity and universality of these monoclones and find the best candidate clones, we further evaluated the specificity of the primarily selected clones by flow cytometry. In this experiment, we used a variety of CD5-positive cell lines and a variety of CD5-negative cell lines to react with these monoclonal phage antibodies, and analyzed whether these clones could bind to the CD5 antigen on different cell lines and whether they had any non-specific binding to other cell lines that do not express CD5. Through this experiment, we obtained several clones with excellent specificity.

Experimental method: the same as the FACS primary selection;

### Main samples and reagents:

Jurkat cell line, a CD5 positive cell line;
Raji cell line, a CD5 negative cell line;
CCRF-CEM cell line, a CD5 positive cell line;
NALM6 cell line, a CD5 negative cell line;

The rest of the reagents were the same as the FACS primary selection.

### Experimental results:

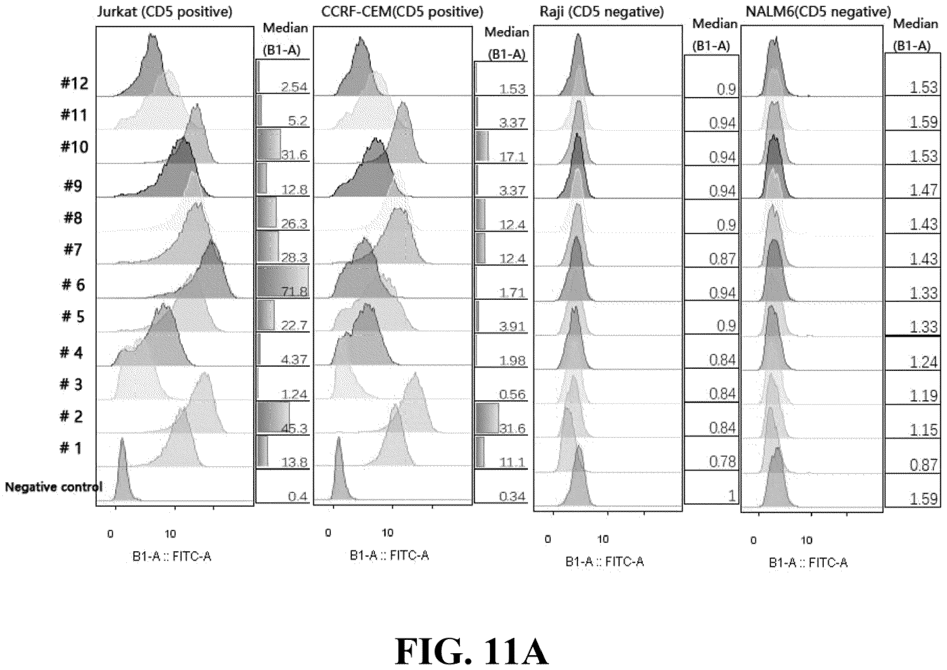

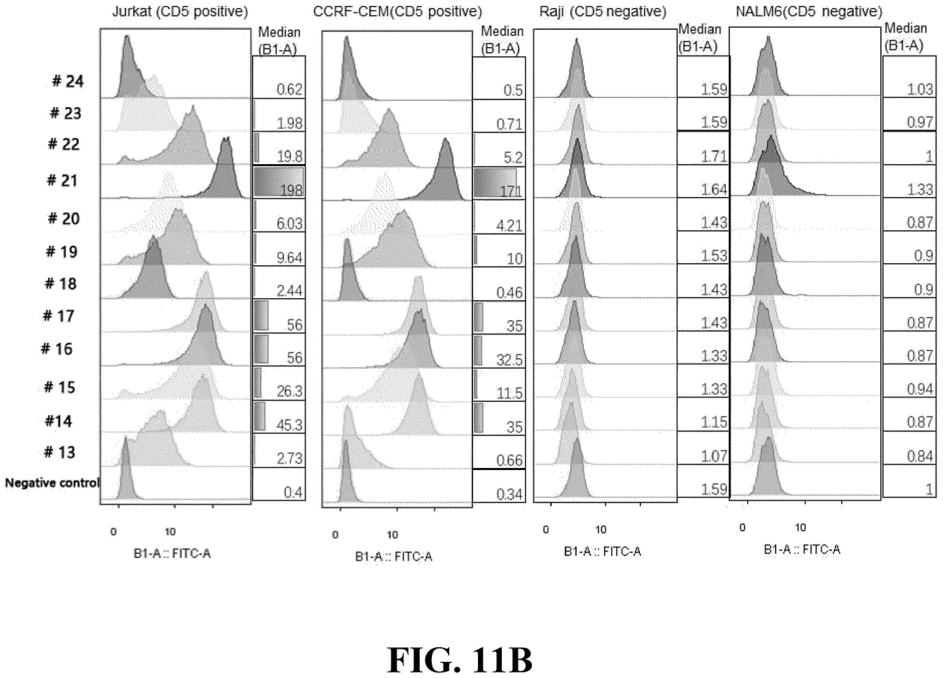

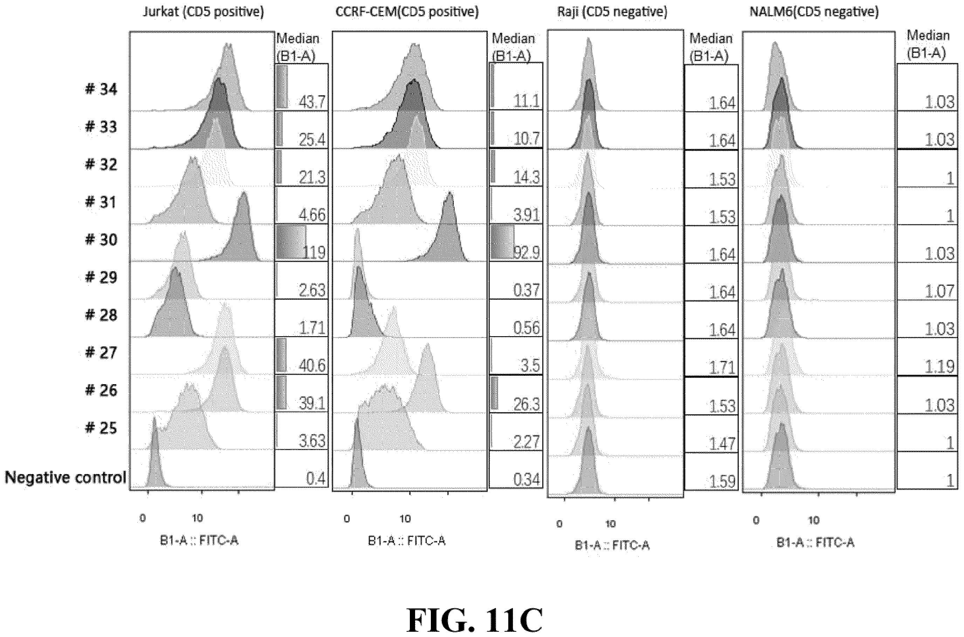

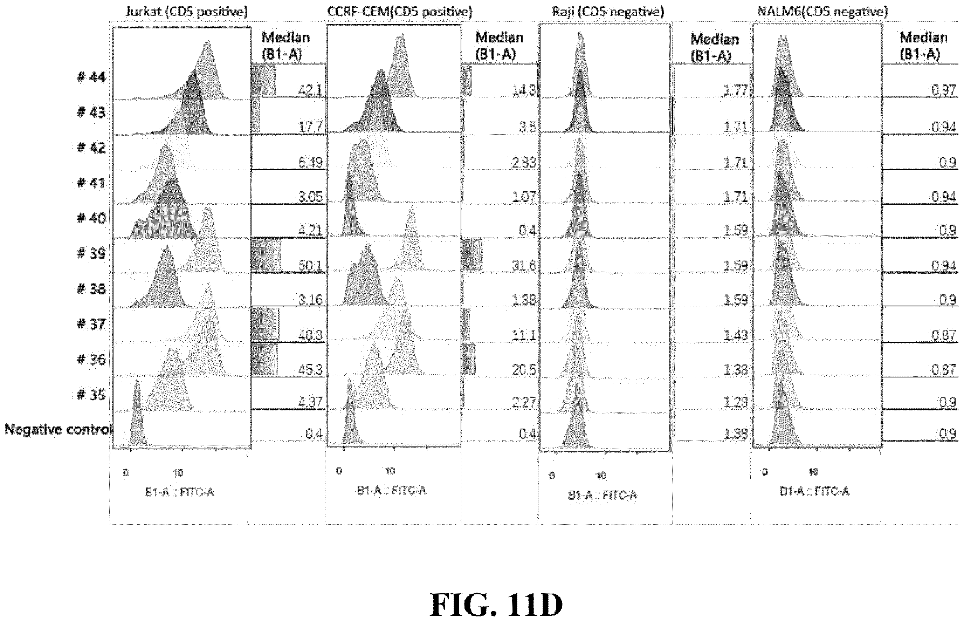

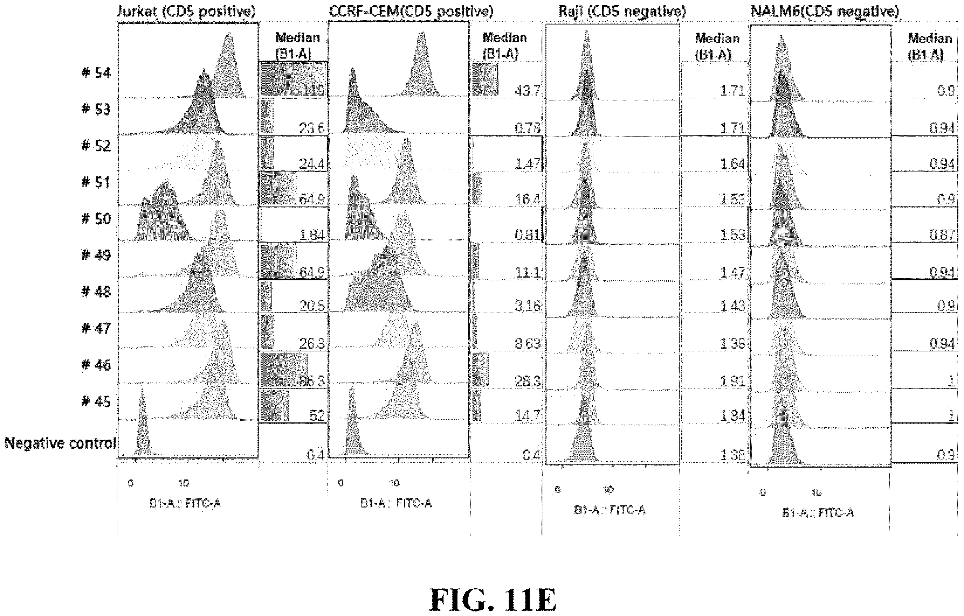

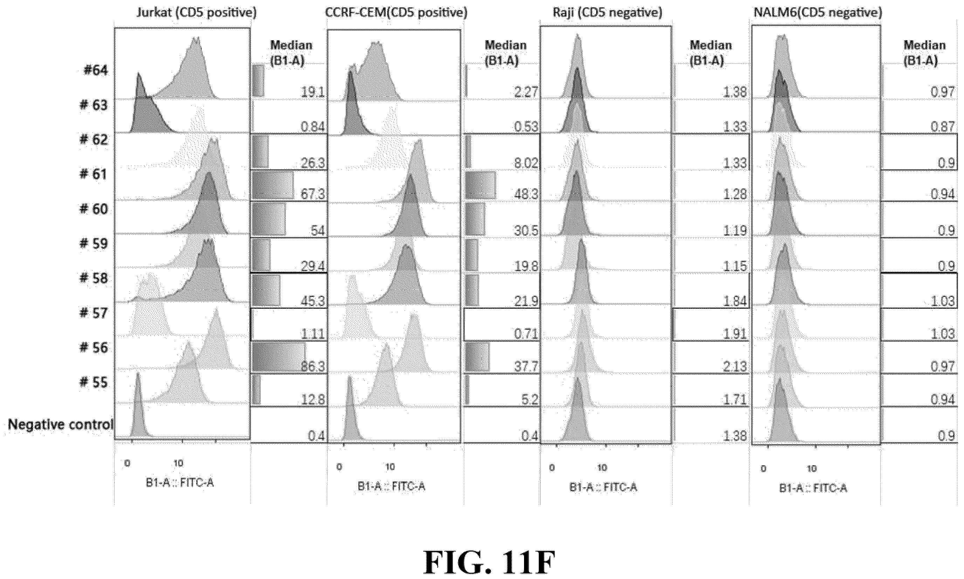

Antibodies used for therapy must have very good target specificity. In order to further analyze the specificity of these monoclonal antibodies, we identified the unique specific clones obtained in Example 2 on more antigens and cell lines by using ELISA and flow cytometry. The results are shown in Figs. 11A-F, Negative Control is the negative control phage antibody clone. Clones #1-2, #4-12, #14-17, #19-22, #25-27, #30-39, #42-49, #51, #54-55, #58-62, #64 bound to the two CD5-positive cell lines Jurkat and CCRF-CEM, with strong or weak median fluorescence intensity (MFI), and they did not bind to the two CD5-negative cell lines Raji and NALM6, with low MFI and good specificity; clones #3, #13, #18, #23, #24, #28, #29, #40, #41, #50, #57 and #63 bound weakly to or not to the positive cell line Jurkat, and did not bind to the positive cell line CCRF-CEM, and they are negative clones; clones #52 and #53 only bound to the positive cell line Jurkat, but not to CCRF-CEM, indicating that they cannot recognize CD5antigens of different conformations or isomers expressed by different cell lines, and do not meet the experimental requirements; clone #56 could bind to the two positive cell lines, but weakly bound to the negative cell line Raji, indicating that its binding may be non-specific and does not meet the experimental requirements.

### Example 4. Identification of monoclonal specificity by ELISA using antigens from different companies

### Experimental purpose and principle:

Antibodies used for therapy must have very good target specificity, binding only to the target antigen and not to any unrelated antigen; on the other hand, the amino acid sequence of the same antigen produced by different companies will vary (isomers or mutants), and it is also necessary to investigate whether our antibodies can bind to various target proteins. In order to further analyze the specificity and universality of these monoclones and find the best candidate clones, we further evaluated the specificity of the primarily selected clones by enzyme linked immunosorbent assay (ELISA). In this experiment, we used CD5 antigens purchased from different companies and various CD5-unrelated antigens to react with these monoclonal phage antibodies, and analyzed whether these clones could bind to different CD5 antigens and whether they had any non-specific binding to other CD5-unrelated antigens. Through this experiment, we obtained several clones with excellent specificity.

Experimental method: the same as the ELISA primary screening;

### Main samples and reagents:

| **Abbreviation** | **Name** | **Manufacturer** | **Item No.** |
|---|---|---|---|
| Acro-CD5-his | Human CD5 Protein, his tag | ACRObiosystem | CD5-H52H5 |
| Kactus-CD5-Fc-Bio | Recombinant biotinylated | Kactus | CD5-HM401 |
| SB-CD5-his-Bio | Human CD5 Protein SB-human CD5 Protein, his tag, Bio | Sino Biological | 11027-H27H-B |
| Kactus-BAFFR-his-Bio | Biotinylated Recombinant human BAFFR Protein | Kactus | BAF-HM40RB |
| Kactus-CD19-FC-Bio | Recombinant biotinylated Human CD19 Protein | Kactus | CD2-HE121 |
| SA | Streptavidin, APC-mouse anti-human CD5 | Pierce | 21125 |
| Mouse anti-human CD5 Ab | | BD | 555355 |

The rest of the reagents were the same as in the ELISA primary screening.

### Experimental results:

Antibodies used for therapy must have very good target specificity. In order to further analyze the specificity of these monoclonal antibodies, we identified multiple clones obtained in Example 2 on various antigens using enzyme-linked immunosorbent assay (ELISA). As shown in Figs. 12A-F, Negative control is a negative control phage antibody clone that binds to neither the target antigen nor the control antigen; anti-M13 phage mouse Ab/anti-mouse HRP Ab is a negative antibody control with only the primary and secondary antibodies; anti-mouse HRP Ab is a negative antibody control with only the secondary antibody, and it binds to neither the target antigen nor the control antigen; Mouse anti-human CD5 Ab/anti-mouse HRP Ab is a positive antibody control of the target antigen (CD5-Fc-Bio) which binds to the target antigen and does not bind to the control antigen. Anti-human IgG-HRP Ab/anti-his-HRP Ab is a positive antibody control for detecting antigen tags, which binds to antigens containing Fc tags or his tags, indicating that the coated antigens have been bound to the ELISA plate. Clones #1-64 bound to all three CD5 antigens, but none of the three non-related antigens, indicating that they could bind to CD5 antigens from different companies with good specificity.

The following table lists the amino acid and nucleic acid sequence numbers corresponding to the heavy chain variable region (HCVR), light chain variable region (LCVR), ScFv and CDR sequences of some selected anti-CD5 antibodies:

| Antibody No. | Amino acid sequence SEQ ID NO: | | | | | | | | | DNA sequence SEQ ID NO: | | |
|---|---|---|---|---|---|---|---|---|---|---|---|---|
| | HCDR 1 | HCDR 2 | HCDR 3 | VH | LCDR1 | LCDR 2 | LCDR3 | VL | ScFv | VH | VL | ScFv |
| #10 | 43 | 44 | 45 | 30 | 46 | 47 | 48 | 29 | 28 | 27 | 26 | 25 |
| #32 | 49 | 50 | 51 | 36 | 52 | 53 | 54 | 35 | 34 | 33 | 32 | 31 |
| #35 | 55 | 56 | 57 | 42 | 58 | 59 | 60 | 41 | 40 | 39 | 38 | 37 |
| #6 | 61 | 62 | 63 | 90 | 64 | 65 | 66 | 89 | 88 | 81 | 80 | 79 |
| #7 | 67 | 68 | 69 | 93 | 70 | 71 | 72 | 92 | 91 | 84 | 83 | 82 |
| #9 | 73 | 74 | 75 | 96 | 76 | 77 | 78 | 95 | 94 | 87 | 86 | 85 |

### Example 5. Knocking out CDS antigen on the surface of T-cells by CRISPR/Cas9 technology

### (1) Experiment purpose and principle:

All mature T-cells express the CD5 antigen on the surface, while the CD5 CAR-T without CD5 knockout developed by Mamonkin M and other researchers has been reported to have a certain degree of suicide¹, and survive in the patient for a limited period of time, which greatly limits the disease remission time of the patient and the application of the CAR-T product. In order to solve this problem, the present application uses the CRISPR/Cas9 technology to knock out the CD5 antigen on the surface of T-cells, which minimizes the self-activation and suicide of CD5 CAR-T, and ensure its persistence and effectiveness.

In order to select sgRNAs with high knockout efficiency, we firstly use different sgRNAs to knock out the CD5 gene in jurkat cells that naturally highly express CD5, so as to determine the efficient sgRNAs for knocking out the CD5 antigen of T-cells and obtain jurkat-CD5ko pool with high knockout efficiency, and then obtained CD5-negative monoclonal jurkat-CD5ko cells through monoclonal selection. In this way, the difference between jurkat and jurkat-CD5ko cells lies in this knocked-out gene. This pair of cells will have important applications in the identification of monoclonal antibody binding specificity and CAR-T functional verification.

### (2) The brief experimental steps are as follows:

1) selecting multiple sgRNA sequences (sgRNA1 to sgRNA4) and performing primer synthesis;
2) synthesizing sgRNA by chemical synthesis;
3) electroporating jurkat cells with sgRNA/Cas9 RNP;
4) detecting the gene knockout efficiency of different sgRNAs by FACS, selecting the jurkat-CD5ko pool with higher knockout efficiency, and isolating monoclones by limiting dilution assay;
5) identifying monoclones by FACS;
6) constructing and preserving jurkat-CD5ko monoclonal cell libarary.

### (3) Main materials and reagents:

Chemically synthesized EasyEdit sgRNA, Nanjing GenScript Biotech Co., Ltd.
sgRNA1: aagcgtcaaaagtctgccag (SEQ ID NO: 103, this sequence is provided in the form of its target sequence, the same below)
sgRNA2: ccgttccaactcgaagtgcc (SEQ ID NO: 104)
sgRNA3: gctgtagaactccaccacgc (SEQ ID NO: 105)
sgRNA4: aatcatctgctacggacaac (SEQ ID NO: 106)
TrueCut^{™} Cas9 Protein v2, thermo, A36498
APC Mouse Anti-Human CD5 antibody, Clone UCHT2, BD Pharmingen, 555355

### (4) Experimental results:

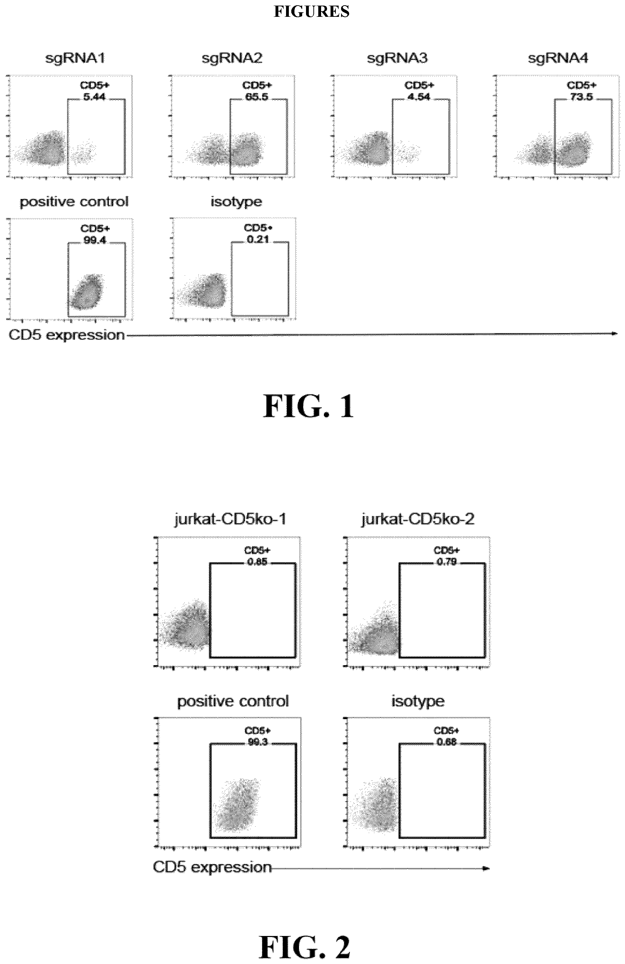

As shown in Fig. 1, the CD5 knockout efficiency of cells corresponding to sgRNA3 was the highest, and 95.46% of CD5 were knocked out. Therefore, we chose the jurkat-CD5ko pool electroporated with sgRNA3 for monoclonal selection. Monoclones were isolated from the cell pool by limiting dilution assay, and after the monoclones were amplified, the CD5 expression of these monoclones was detected by FACS. The results are shown in Fig. 2, in which the CD5 expression of jurkat-CD5ko-1 and 2 clones was basically undetectable, and these clones can be considered as monoclones with successful knockout. These two clones were cultured and frozen. We used clone 1 in subsequent studies.

### Example 6. Preparation and detection of CAR-T cells

The present application uses lentiviral transfection to make the knockout T-cells express CAR. For the preparation process of CAR-T, please refer to the patent (Zhou J, Liu J, Hu G, et al. Chimeric antigen receptor (car) binding to bcma, and uses thereof: U.S. Patent Application 16/650,580[P]. 2020-8-6.). The lentiviral vector is based on the genome of lentivirus. Multiple sequence structures related to viral activity in the genome of lentivirus are removed to make it biologically safe, and then a vector prepared from the sequence and expression structure of the target gene required for the experiment is introduced into the genome skeleton. Compared with other retroviruses, lentiviral vectors have a wider range of hosts, have the ability to infect both dividing and non-dividing cells, and for some cells that are difficult to transfect, such as primary cells, stem cells and undifferentiated cells, can greatly improve the transduction efficiency of the target gene (see Chen Chen and Wan Haisu, "Lentiviral vectors and their research progress, Chinese Journal of Lung Cancer 17.12 (2014): 870-876. PMC). Through lentiviral vector transfection, CAR molecules can be integrated into host cells.

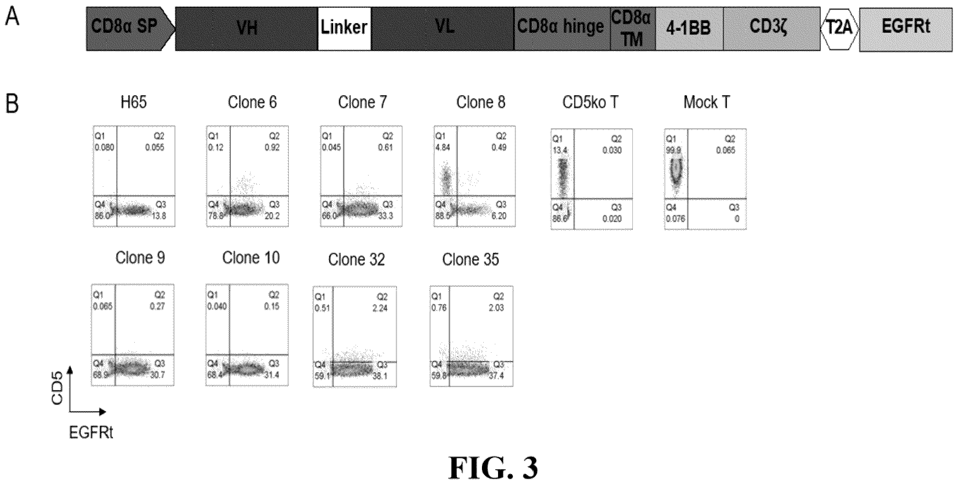

The T-cells were knocked out of CD5 and transfected with the CAR lentivirus with the structure shown in Fig. 3A (the specific CAR molecular structure is shown in Table 2). After 5 to 7 days, the expression of CD5 and EGFRt and the binding of CD5 antigen were detected (Fig. 3B). The CAR structure comprises CD8α signal peptide, scFv, CD8α hinge region, CD8α transmembrane region, 4-1BB co-stimulatory molecule and CD3ζ and is linked through T2A to a truncated EFGR molecule (EGFRt) , which can be used as a safety switch during clinical transformation. Since EGFRt is co-expressed with CAR molecules, it can be used as an indirect detection index for the distribution of CAR molecules on the surface of T-cells without affecting the structure and function of CAR.

**Table 2. Exemplary CAR molecules and antigen recognition portions thereof**

| CAR 001: CD8α signal peptide-Scfv 10--CD8ahinge/TM--4-1BB **co-stimulatory molecule**--CD3ζ--T2A- -CSF2RA signal-- tEGFR | | | | | | | |
|---|---|---|---|---|---|---|---|
| CDRs of Scfv 10 (clone 10): | | | | | | | |
| SEQ ID NO: 43 | HCDR1 | GFTFNNYT | | | SEQ ID NO: 46 | LCDR1 | SSNIGAGYD |
| SEQ ID NO: 44 | HCDR2 | ISSSSSYI | | | SEQ ID NO: 47 | LCDR2 | GNI |
| SEQ ID NO: 45 | HCDR3 | ARYFSGSAGDY | | | SEQ ID NO: 48 | LCDR3 | GTWDNSLSAHYV |

| CAR 002: CD8α signal peptide-Scfv 32--CD8ahinge/TM--4-1BB **co-stimulatory molecule**--CD3ζ--T2A- -CSF2RA signal-- tEGFR | | | | | | | |
|---|---|---|---|---|---|---|---|
| CDRs of Scfv 32 (clone 32): | | | | | | | |
| SEQ ID NO: 49 | HCDR1 | GFTFSSYS | | | SEQ ID NO: 52 | LCDR1 | NSNIGNNY |
| SEQ ID NO: 50 | HCDR2 | ISSSSSYI | | | SEQ ID NO: 53 | LCDR2 | DNN |
| SEQ ID NO: 51 | HCDR3 | ARGNPWYGVDY | | | SEQ ID NO: 54 | LCDR3 | GIWDSSLDAYV |

| CAR003: CD8α signal peptide-Scfv 35--CD8αhinge/TM--4-1BB **co-stimulatory molecule**--CD3ζ--T2A-CSF2RA signal-- tEGFR | | | | | | | |
|---|---|---|---|---|---|---|---|
| CDRs of Scfv 35 (clone 35): | | | | | | | |
| SEQ ID NO: 55 | HCDR1 | GFTFSSYA | | | SEQ ID NO: 58 | LCDR1 | SSNIGNNY |
| SEQ ID NO: 56 | HCDR2 | ITDSGDIT | | | SEQ ID NO: 59 | LCDR2 | DNN |
| SEQ ID NO: 57 | HCDR3 | ARMSSHWYFSA DY | | | SEQ ID NO: 60 | LCDR3 | GTWDSSSAW |

| CAR004: CD8α signal peptide-Scfv6--CD8αhinge/TM--4-1BB co-stimulatory molecule--CD3ζ--T2A-CSF2RA signal-- tEGFR | | | | | | | |
|---|---|---|---|---|---|---|---|
| Scfv6 (Clone6) CDRs: | | | | | | | |
| SEQ ID NO: 61 | HCDR1 | GGTFSSNA | | | SEQ ID NO: 64 | LCDR1 | SGSIARNY |
| SEQ ID NO: 62 | HCDR2 | IIPMFGTT | | | SEQ ID NO: 65 | LCDR2 | EDN |
| SEQ ID NO: 63 | HCDR3 | ARDGGGRHPYY YYGMDV | | | SEQ ID NO: 66 | LCDR3 | QSYDDNTSWV |

| CAR005: CD8α signal peptide-Scfv002--CD8ahinge/TM--4-1BB co-stimulatory molecule--CD3ζ--T2A-CSF2RA signal-- tEGFR | | | | | | | |
|---|---|---|---|---|---|---|---|
| CDRs of Scfv7 (Clone7): | | | | | | | |
| SEQ ID NO: 67 | HCDR1 | | GYSFTSYW | | SEQ ID NO: 70 | LCDR1 | SSNIGNND |
| SEQ ID NO: 68 | HCDR2 | | IYPDDSDT | | SEQ ID NO: 71 | LCDR2 | DND |
| SEQ ID NO: 69 | HCDR3 | | ARMSLTSYLA TDG | | SEQ ID NO: 72 | LCDR3 | AVWDSSLSAAV |

| CAR006: CD8α signal peptide-Scfv003--CD8αhinge/TM--4-1BB co-stimulatory molecule--CD3ζ--T2A-CSF2RA signal-- tEGFR | | | | | | | |
|---|---|---|---|---|---|---|---|
| CDRs of Scfv9 (Clone9): | | | | | | | |
| SEQ ID NO: 73 | HCDR1 | | | TYTFTNYY | SEQ ID NO: 76 | LCDR1 | SDDIGNYKR |
| SEQ ID NO: 74 | HCDR2 | | | INPSGDTT | SEQ ID NO: 77 | LCDR2 | EVT |
| SEQ ID NO: 75 | HCDR3 | | | ARLSWYWG GSFDD | SEQ ID NO: 78 | LCDR3 | SSYASGDTYV |

The brief experimental steps for CD5 antigen expression detection and EGFRt expression detection of CAR-T/T cells are as follows:
1) taking 1 × 10⁶ CAR-T/T cells per well, adding PBS and washing once, centrifuging at 300g for 5 min, and discarding the supernatant;
2) resuspending the cell pellets in 100 µL of PBS, adding 5 µL of APC-CD5 antibody and 5 µL of PE-EGFR antibody respectively, and incubating at 4°C in the dark for 15 min;
3) washing twice with PBS and centrifuging at 300g for 5 min; and
4) resuspending with 200 µL of PBS, and detecting by flow cytometry.

### Main materials and reagents:

APC Mouse Anti-Human CD5 antibody, Clone UCHT2, BD Pharmingen, Cat. No.555355;
PE anti-human EGFR Antibody, Clone AY13, BioLegend, Cat. No. 352904;
Fetal bovine serum (FBS), Gibco, Cat. No. 10099141;

### Experimental results:

As shown in Fig. 3B, on the seventh day, the CD5 knockout efficiency on T-cells was detected to reach 86.6%, and all cloned CAR molecules could be expressed on the surface of T-cells. The CAR-T studied in the present application targets the CD5 target, and if the CAR -T cells function well, after knockout and transfection with lentivirus, the CAR-T cells can kill CAR-T/T cells that still express the CD5 antigen. In this experiment, except for Clone 8 CAR-T, the CAR-T cells of other clones could clear up the cells without CD5 knockout.

### Example 7. In vitro function verification of CAR-T cells

### Experimental purpose and principle:

In the present application, a variety of specific antibody clones targeting CD5 protein that are enriched, selected and identified from the phage antibody library by affinity panning are constructed on the second-generation CAR, and considering the fact that the scFv that binds to the CD5 antigen may not have good activation function, its function on CAR-T cells needs to be further confirmed to select the most active CAR molecules. To this end, we prepare lentiviral vectors of these CAR molecular clones, and transduce T-cells to prepare CAR-T cells. Then, the in vitro biological efficacy of CAR-T cells is evaluated by CD107a degranulation assay and in vitro cytotoxicity assay. Through these functional verifications at CAR-T level, CAR molecules with ideal efficacy and safety are finally selected for downstream CAR-T product development.

### CD107a degranulation assay

CD107a is a marker for intracellular microvesicles, and CD107a on the cell membrane increases after granzyme-loaded microvesicles fuse with the cell membrane, and when its recovery is blocked by monesin (purchased from BioLegend), the strength of microvesicle release can be quantitatively reflected. When CAR-T is stimulated by the target antigen on the target cell, it will cause the release of granzyme, and the activation of T-cells can be judged by the increase of CD107a by flow cytometry.

Brief experimental steps of CD107a degranulation:
1) centrifuging CAR-T cells to be tested and target cells separately at room temperature at 300 g for 5 min, discarding the supernatant, and resuspending the cells with 1640 culture medium + 10% FBS to 1×10° cells/mL;
2) in a 24-well plate, adding 500 µL of CAR-T cells to be tested and 500 µL of target cells, and mixing well;
3) adding 5 µL of PE/Cy7 mouse anti-human CD107a antibody and 1 µL of monensin to the cells in each well, and then placing the cells in a cell incubator (37°C, 5% CO₂) for 3 h;
4) after incubation, taking out 500 of µL of cell suspension from the 24-well plate, centrifuging at 4°C at 300 g for 5 min, then discarding the supernatant, and washing the cells twice with 1 mL of PBS;
5) resuspending the cells with 100 µL of PBS, and adding 5 µL of APC mouse anti-human CD8 and 5 µL of Alexa Fluor 488 anti-human EGFR antibody, respectively, well mixing, and incubating them on ice for 20 min in the dark; and
6) after incubation, washing the cells with 1 mL of PBS for 3 times; and after resuspending with 400 µL of PBS, detecting the cells by flow cytometry.

### Main samples and reagents:

Target cells jurkat, jurkat CD5 ko, CCRF-CEM, MOLT4, K562-CD5, K562, RAJI;
Fetal bovine serum (FBS), Gibco, Cat. No. 10099141;
Monensin, BioLegend, Cat. No. 420701;
PE/Cy7 mouse anti-human CD107a, BD, Cat. No. 561348;
APC mouse anti-human CD8, BD, Cat. No. 555369;
Alexa Fluor 488 anti-human EGFR, BioLegend, Cat. No. 352908.

### Experimental results:

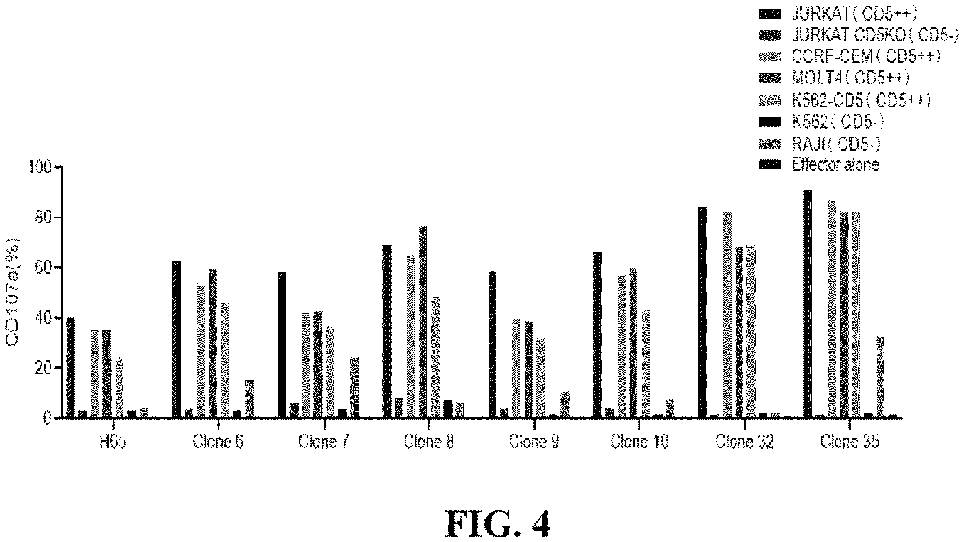

The CAR-T cells were obtained by lentiviral transduction, and CD107a degranulation assay was performed after the CAR-T cells were cultured in vitro for 9-12 days. The CAR-T cells to be tested were incubated with target cells, monensin and CD107a antibody for 4 h, and the cell density of both CAR-T cells and target cells was 5×10⁵ cells/mL. Then, after the samples were labeled with anti-CD8 antibody and anti-EGFR antibody, flow cytometry was performed. Analysis was conducted with Flowjo software. In the scatter plot, living cell gate (P1) was selected and cell debris was removed; for the cells in the P1 gate, a single scattered cell gate (P2) was selected after analysis; then, CD8 positive cells were further selected in the P2 gate (P3); finally, in the P3 gate, the proportion of CD107a-positive cells in positive cells (i.e., CAR-positive cells) found through anti-EGFR antibody staining was analyzed. The analysis results are shown in Fig. 4, suggesting that the CAR-T cells of Clone 8, Clone 9, Clone 10 and Clone 32 had stronger CD107a degranulation function than the control CAR-T cells (H65 CAR-T cells), while the CAR-T cells of Clone 6, Clone 7 and Clone 35 have a certain degree of non-specific activation when co-incubated with the CD5-negative cell line RAJI cells, and because the CAR-T cells of Clone 8 could not completely eliminate CD5+ T-cells in the above experiments, Clone 9, Clone 10 and Clone 32 CAR-T cells can be used as the cells for the specific activation of the target cells.

### In vitro cytotoxicity assay

The purpose and principle of the assay: in the in vitro cytotoxicity assay, CCRF-CEM and MOLT4 were used as CD5-positive target cells, and K562 and RAJI cells are used as CD5-negative target cells to evaluate the antigen-specific killing ability of CD5 CAR-T cells. Among others, the above cells are respectively transduced by lentivirus to obtain target cells stably expressing firefly luciferase, so the activity of luciferase in the sample can reflect the number of target cells. The CAR-T cells and target cells are co-incubated and cultured. When target cells are killed by CAR-T cells, luciferase is released and quickly inactivated (firefly luciferase has a half-life of about 0.5 h). If the target cells are not killed or inhibited by CAR-T cells, more luciferases will be produced as the target cells proliferate and continue to express luciferase. Therefore, the killing of target cells by CAR-T can be detected by the activity of luciferase.

### Brief steps of in vitro cytotoxicity assay

1) centrifuging the above cells at room temperature at 300 g for 5 min separately, discarding the supernatant, and then re-suspending the cells in T-cell culture medium to 2×10⁵ cells/mL; adding 100 µL of target cells to each well of the 96-well plate;
2) according to the CAR positive rate and the effector-target ratio of the CAR-T sample to be tested, adding 100 µL of CAR-T cells into each well of the 96-well plate, and mixing the CAR-T cells with the target cells; then, putting the cells into a carbon dioxide incubator to incubate and culture for 24 h; and
3) detecting the luciferase activity of the sample in each well using a luciferase detection kit.

### Main samples and reagents:

Target cells CCRF-CEM, MOLT4, K562, RAJI;
Steady-Glo Luciferase Assay System, Promega, Cat. No. E2520.

### Experimental results:

The CAR-T cell sample and a fixed number of target cells (1×10⁴) were mixed according to different effector-target ratios (E:T), and incubated together for 24 h, and then the luciferase activity (RLU) in the sample was detected. Among them, CD5 ko T-cells were the control sample comprising only the target cells. Since the luciferase activity can reflect the number of target cells in the sample, the killing/inhibiting ability of CAR-T cells on target cells can be obtained through the change of luciferase activity in the sample. The lower the luciferase activity readout (RLU), the more target cells are killed.

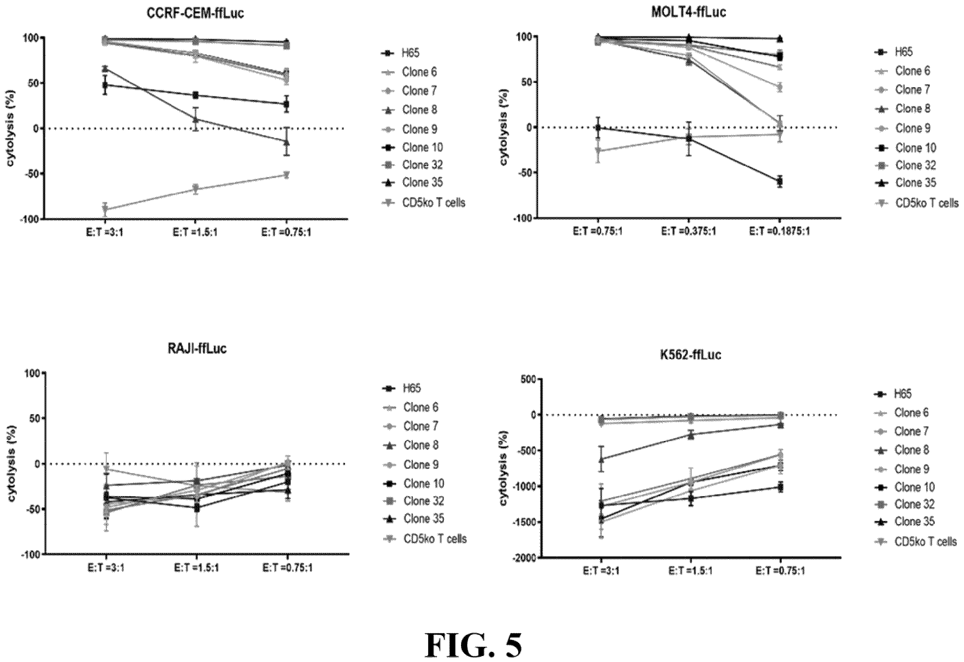

As shown in Fig. 5, except for Clone 8 CAR-T cells, other clone CAR-T cell samples were more effective in killing positive target cells than the control H65 CAR-T cells. When T-cells were co-incubated with positive target cells, there was no obvious killing. All CAR-T and T-cell samples had no obvious killing when co-incubated with negative target cells. Therefore, the CAR-T samples of clone 6, clone 7, clone 9, clone 10, clone 32, and clone 35 could specifically kill CD5-positive target cells, and did not non-specifically kill CD5-negative target cells.

### Repeated stimulation proliferation assay

### Experimental purpose and principle:

Mitomycin-treated target cells (CCRF-CEM) were mixed with different groups of CD5 CAR-T cells for 4 stimulations, and then the CAR-T cells and target cells were co-incubated to determine the proliferation ability of different scFv CAR-T cells after being repeatedly stimulated by the target cells.

Brief steps of cytotoxicity assay after repeated stimulation:
1) taking 4×10⁶ CCRF-CEM cells, centrifuging at 300 g at room temperature for 5 min;
2) adjusting the cell density to 0.2×10⁶ cells/mL with the complete medium, adding 4 µL of Mitomycin stock solution (5 µg/µL) and mixing well, culturing for 24 h before use;
3) taking the CCRF-CEM-Mitomycin cells after 24 h treatment, centrifuging at 300 g to change the medium, washing 6 times with PBS, resuspending the CCRF-CEM-Mitomycin cells in CTS medium, counting the cells and adjusting the cell density to 2×10⁶ cells/ mL before use; and
4) according to the positive rate of CAR-T CAR, taking 1×10⁵ CAR+ cells and transferring them into 24-well plates; adding 50 µL of CCRF-CEM-Mitomycin cells to the CAR-T in each well, and adjusting the effect-target ratio (effector cells are counted as CAR+ ) E:T = 1:1; replenishing with CTS complete medium until the final culture volume reaches 500 µL, mixing well, culturing at 37°C, 5% CO₂ for 72 h and counting, treating the target cells (CCRF-CEM) with Mitomycin again and detecting CAR-T CAR positive rate, repeating the above steps 4 times, and then plotting the amplification curve.

### Experimental results:

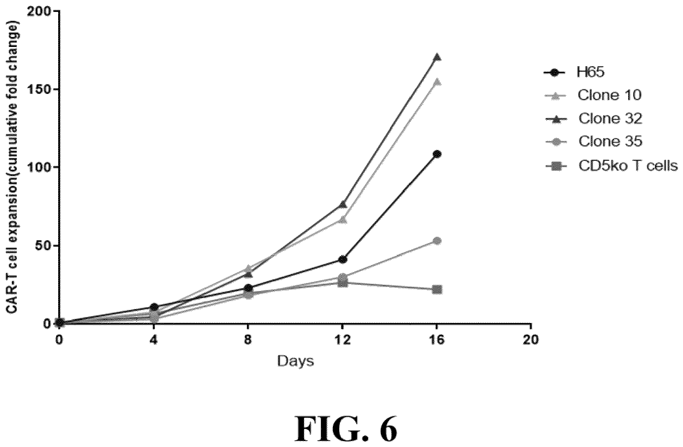

As shown in Fig. 6, after repeated stimulation, the 5 groups of CAR-T/T cell samples were ranked as follows in terms of proliferation ability: Clone 32 CAR-T > Clone 10 CAR-T > H65 CAR-T > Clone 35 CAR-T > CD5ko T-cells, and after 4 times of stimulation with the target cell, the CAR-T cells of Clone 10, Clone32 and H65 can still effectively expand. The proliferation ability of CAR-T cells after being stimulated by target cells is closely related to the long-term prognosis of patients. Therefore, Clone 32 CAR-T and Clone 10 CAR-T can be considered to have the potential to proliferate and eliminate tumor cells in vivo for a long time.

### Example 8. Determination of anti-CD5 scFvs affinity

### Experimental purpose and principle:

The affinity between CD5 scFvs and the antigen may also have an important impact on the killing effect and duration of CAR-T in patients. In order to determine this important property, the present application uses the Octet molecular interaction technology of ForteBio to determine it. The biofilm interferometry technology used in the Octet system is a label-free technology that provides high-throughput biomolecular interaction information in real time. The instrument emits white light to the surface of the sensor and collects the reflected light. The reflected light spectrum of different frequencies is affected by the thickness of the optical film layer of the biosensor. Some frequencies of reflected light form constructive interference (blue), while others form destructive interference (red). These interferences are detected by the spectrometer and form an interference spectrum, which is demonstrated by the phase shift intensity (nm) of the interference spectrum. Therefore, once the number of molecules bound to the sensor surface increases or decreases, the spectrometer will detect the shift of the interference spectrum in real time, and this shift directly reflects the thickness of the biofilm on the sensor surface, from which high-quality data of biomolecular interaction can be obtained, so as to determine the kinetic parameters (Kon, Kdis and KD) of biomolecular interactions, providing important information for the research and development process.

### Brief experimental steps:

1) diluting anti-CD5 IgG to 20 µg/mL with loading buffer (1×PBS, pH 7.4, 0.01% BSA and 0.02% Tween 20), and loading the sample on the biosensor at about 0.8 nM;
2) after a 60s equilibration period, monitoring the binding kinetics of the CD5 antigen at various antigen concentrations (400 to 12.5 nM); carrying out 160 s association and 300 s dissociation at each concentration, respectively;
3) washing 3 times with 10 mM Glycine-HCl, pH1.5 to regenerate the chip; and
4) analyzing for binding constant by using a 1:1 binding site model (Biacore X-100 evaluation software).

### Experimental results:

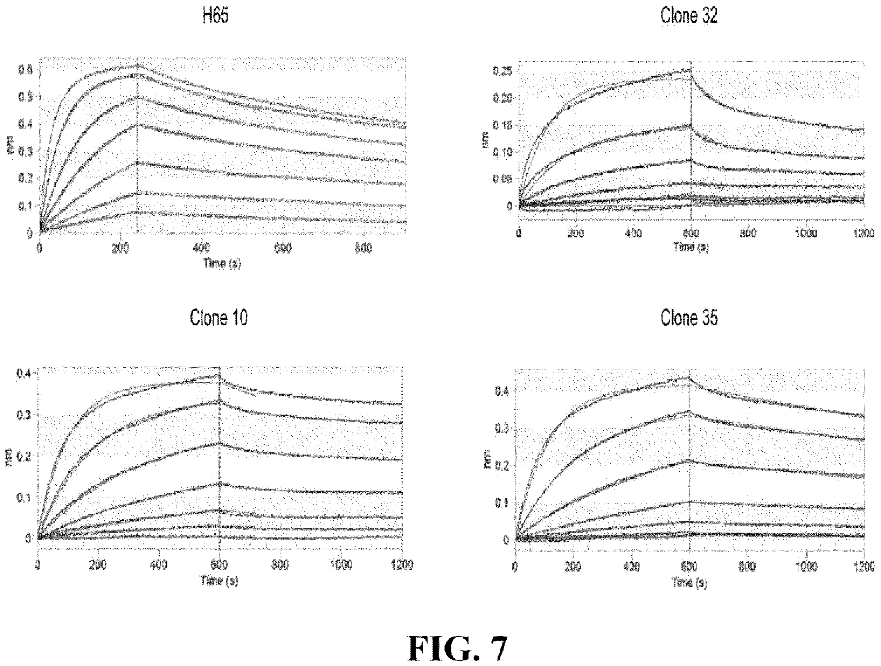

Affinity refers to the strength of the binding of a single molecule to its ligand and is usually measured and reported by the equilibrium dissociation constant (KD), which is used to assess and rank the strength of the interaction between two molecules. The binding of an antibody to its antigen is a reversible process, and the rate of the binding reaction is proportional to the concentration of the reactants. The smaller the KD value, the greater the affinity of the antibody for its target. As shown in Table 3 and Fig. 7, H65, Clone 10, Clone 32, and Clone 35 could all bind to the CD5 antigen, and Clone 10 showed a level of affinity comparable to that of the control antibody H65, which was slightly higher than that of Clone 32 and Clone 35.

**Table 3 Anti-CD5 IgG affinity determination**

| Analyte | KD (M) | kon (1/Ms) | kdis (1/s) |
|---|---|---|---|
| H65 | 2.25E-09 | 3.61E+05 | 8.13E-04 |
| Clone 10 | 4.49E-09 | 3.18E+05 | 1.43E-03 |
| Clone 32 | 2.83E-08 | 7.65E+04 | 2.17E-03 |
| Clone 35 | 1.36E-08 | 2.81E+04 | 3.82E-04 |

The foregoing detailed description has been offered by way of explanation and exemplification, and it not intended to limit the scope of the appended claims. Variations of the presently recited embodiments of the present application will be apparent to those of ordinary skill in the art and remain within the scope of the appended claims and their equivalents.

### References:

1. Kochenderfer. J.N., et al.. Chemotherapy-refractory diffuse large B-cell lymphoma and indolent B-cell malignancies can be effectively treated with autologous T cells expressing an anti-CD19 chimeric antigen receptor. J. Clin. Oncol.. 2015. 33(6): p. 540-9.
2. Gill. S., et al., CD19 CAR-T cells combined with ibrutinib to induce complete remission in CLL. Journal of Clinical Oncology, 2017. 35(15_suppl): p. 7509-7509.
3. Neelapu. S.S., et al., Axicabtagene Ciloleucel CAR T-Cell Therapy in Refractory Large B-Cell Lymphoma. New England Journal of Medicine. 2017. 377(26): p. 2531-2544.
4. Maude, S.L., et al.. CD19-targeted chimeric antigen receptor T-cell therapy for acute lymphoblastic leukemia. Blood. 2015. 125(26): p. 4017.
5. Jacobson. C.A., CD19 Chimeric Antigen Receptor Therapy for Refractory Aggressive B-Cell Lymphoma. Journal of Clinical Oncology, 2018. 37(4): p. 328-335.
6. Kochenderfer. J., et al., Anti-CD19 chimeric antigen receptor T cells preceded by low-dose chemotherapy to induce remissions of advanced lymphoma. Journal of Clinical Oncology. 2016. 34(18_suppl): p. LBA3010-LBA3010.
7. Grupp. S.A., et al., Chimeric Antigen Receptor-Modified T Cells for Acute Lymphoid Leukemia. New England Journal of Medicine, 2013. 368(16): p. 1509-1518.
8. Hirayama. A.V., et al.. The response to lymphodepletion impacts PFS in aggressive non-Hodgkin lymphoma patients treated with CD19 CAR-T cells. Blood, 2019:p. blood-2018-11-887067.
9. Davila. M.L., et al., Efficacy and toxicity management of 19-28z CAR T cell therapy in B cell acute lymphoblastic leukemia. Sci Transl Med, 2014. 6(224):p. 224ra25.
10. Jones. N.H., et al., Isolation of complementary DNA clones encoding the human lymphocyte glycoprotein T1/Leu-1. Nature, 1986. 323(6086):p. 346-349.
11. Huang, H.J., et al., Molecular cloning of Ly-1, a membrane glycoprotein of mouse T lymphocytes and a subset of B cells: molecular homology to its human counterpart Leu-1/T1 (CD5). Proceedings of the National Academy of Sciences of the United States of America. 1987. 84(1): p. 204-208.
12. Fleischer, L.C., H.T. Spencer, and S.S. Raikar. Targeting T cell malignancies using CAR-based immunotherapy: challenges and potential solutions. Journal of Hematology & Oncology. 2019. 12(1):p. 141.
13. Mamonkin. M., et al., A T-cell-directed chimeric antigen receptor for the selective treatment of T-cell malignancies. Blood, 2015. 126(8): p. 983-992.

The specific sequences in the Examples of the present application are as follows:
CD8a signal nucleic acid sequence (SEQ ID NO: 1) 
ATGGCCCTGCCTGTGACAGCTCTGCTCCTCCCTCTGGCCCTGCTGCTCCATGCCGC
CAGACCC
CD8a signal protein sequence (SEQ ID NO: 2)
   MALPVTALLLPLALLLHAARP
CD8a hinge nucleic acid sequence (SEQ ID NO: 3) 
TTCGTGCCCGTGTTCCTGCCCGCCAAACCTACTACTACCCCTGCACCTAGGCCTCC
CACCCCAGCCCCAACAATCGCCAGCCAGCCTCTGTCTCTGCGGCCCGAAGCCTGTAG
ACCTGCTGCCGGCGGAGCCGTGCACACCAGAGGCCTGGACTTCGCCTGCGAC
CD8a hinge protein sequence (SEQ ID NO: 4)
   FVPVFLPAKPTTTPAPRPPTPAPTIASQPLSLRPEACRPAAGGAVHTRGLDFACD
CD8a TM nucleic acid sequence (SEQ ID NO: 5) 
ATCTACATCTGGGCCCCTCTGGCCGGCACCTGTGGCGTGCTGCTGCTGAGCCTGG
TGATCACCCTGTACTGCAACCACCGGAAC
CD8a TM protein sequence (SEQ ID NO: 6)
   IYIWAPLAGTCGVLLLSLVITLYCNHRN
4-1BB intracellular domain nucleic acid sequence (SEQ ID NO: 7) 
AAACGGGGCAGAAAGAAACTCCTGTATATATTCAAACAACCATTTATGAGACCA
GTACAAACTACTCAAGAGGAAGATGGCTGTAGCTGCCGATTTCCAGAAGAAGAAGA
AGGAGGATGTGAACTG
4-1BB intracellular domain protein sequence (SEQ ID NO: 8)
   KRGRKKLLYIFKQPFMRPVQTTQEEDGCSCRFPEEEEGGCEL
CDSζ intracellular signaling domain nucleic acid sequence (SEQ ID NO: 9) 
AGAGTGAAGTTCAGCAGATCCGCCGACGCCCCTGCCTACCAGCAGGGACAGAAC
CAGCTGTACAACGAGCTGAACCTGGGCAGACGGGAAGAGTACGACGTGCTGGACAA
GCGGAGAGGCCGGGACCCCGAGATGGGCGGAAAGCCCAGACGGAAGAACCCCCAG
GAAGGCCTGTATAACGAACTGCAGAAAGACAAGATGGCCGAGGCCTACAGCGAGAT
CGGCATGAAGGGCGAGCGGAGGCGCGGCAAGGGCCACGATGGCCTGTACCAGGGC
CTGAGCACCGCCACCAAGGACACCTACGACGCCCTGCACATGCAGGCCCTGCCCCCC
AGA
CD3ζ intracellular signaling domain protein sequence (SEQ ID NO: 10) 
RVKFSRSADAPAYQQGQNQLYNELNLGRREEYDVLDKRRGRDPEMGGKPRRKNPQ
EGLYNELQKDKMAEAYSEIGMKGERRRGKGHDGLYQGLSTATKDTYDALHMQALPPR
Cleaving peptide T2A nucleic acid sequence (SEQ ID NO: 11)
   GAGGGAAGGGGCAGCTTATTAACATGTGGCGATGTGGAAGAGAACCCCGGTCCC
Cleaving peptide T2A protein sequence (SEQ ID NO: 12)
   EGRGSLLTCGDVEENPGP
CSF2RA signal nucleic acid sequence (SEQ ID NO: 13) 
ATGCTGCTGCTCGTGACCTCTTTACTGTTATGTGAGCTGCCCCACCCCGCTTTTTT
ACTGATCCCT
CSF2RA signal protein sequence (SEQ ID NO: 14)
   MLLLVTSLLLCELPHPAFLLIP
tEGFR nucleic acid sequence (SEQ ID NO: 15) 
CGTAAGGTGTGTAACGGAATCGGCATTGGCGAGTTCAAGGACTCTTTAAGCATCA
ACGCCACAAACATCAAGCACTTCAAGAATTGTACCTCCATCAGCGGCGATTTACACA
TTCTCCCCGTGGCTTTTCGTGGCGATTCCTTCACCCACACCCCCCCTCTGGACCCCCA
AGAGCTGGACATTTTAAAAACCGTGAAGGAGATCACCGGCTTTCTGCTGATCCAAGC
TTGGCCCGAGAATCGTACCGACCTCCACGCCTTCGAGAATTTAGAGATTATTCGTGG
AAGGACCAAGCAGCACGGCCAGTTCTCTTTAGCCGTCGTGTCTTTAAACATTACCAG
CCTCGGTTTAAGGTCTTTAAAGGAGATTAGCGACGGCGACGTGATCATCTCCGGCAA
CAAGAACCTCTGCTACGCCAACACCATCAACTGGAAGAAGCTGTTCGGAACCAGCG
GCCAAAAGACCAAGATCATCAGCAATCGTGGAGAGAACTCTTGTAAGGCCACTGGT
CAAGTTTGCCACGCCCTCTGTAGCCCCGAAGGATGTTGGGGCCCCGAGCCTAGGGAC
TGTGTTAGCTGCAGAAACGTGAGCAGAGGCAGAGAGTGTGTGGACAAATGCAATTT
ACTGGAAGGAGAGCCTAGGGAGTTCGTGGAGAACAGCGAATGTATCCAGTGCCACC
CCGAGTGTTTACCTCAAGCCATGAACATCACTTGTACCGGAAGGGGCCCCGATAACT
GCATCCAATGCGCCCACTACATCGACGGACCCCACTGCGTGAAAACTTGTCCCGCCG
 
GAGTGATGGGAGAGAATAACACTTTAGTGTGGAAGTACGCCGACGCTGGCCACGTC
TGCCATCTGTGCCACCCCAACTGTACCTACGGCTGCACTGGTCCCGGTTTAGAGGGA
TGTCCTACCAACGGCCCCAAGATCCCCTCCATCGCCACCGGAATGGTGGGCGCTCTG
TTATTACTGCTGGTGGTGGCTCTGGGCATCGGTTTATTCATG
tEGFR protein sequence (SEQ ID NO: 16) 
RKVCNGIGIGEFKDSLSINATNIKHFKNCTSISGDLHILPVAFRGDSFTHTPPLDPQELD
ILKTVKEITGFLLIQAWPENRTDLHAFENLEIIRGRTKQHGQFSLAVVSLNITSLGLRSLKE
ISDGDVIISGNKNLCYANTINWKKLFGTSGQKTKIISNRGENSCKATGQVCHALCSPEGC
WGPEPRDCVSCRNVSRGRECVDKCNLLEGEPREFVENSECIQCHPECLPQAMNITCTGR
GPDNCIQCAHYIDGPHCVKTCPAGVMGENNTLVWKYADAGHVCHLCHPNCTYGCTGP
GLEGCPTNGPKIPSIATGMVGALLLLLVVALGIGLFM
VH-VL linker peptide nucleic acid sequence (SEQ ID NO: 17) 
TCTAGAGGTGGTGGTGGTAGCGGCGGCGGCGGCTCTGGTGGTGGTGGATCCCTC
GAG
VH-VL linker peptide protein sequence (SEQ ID NO: 18)
   SRGGGGSGGGGSGGGGSLE
CAR001 nucleic acid sequence, 2658bp, (SEQ ID NO: 19) 
ATGGCCCTGCCTGTGACAGCTCTGCTCCTCCCTCTGGCCCTGCTGCTCCATGCCGC
CAGACCCCAGTCTGTCGTGACGCAGCCGCCCTCAGTGTCTGGGGCCCCAGGGCAGA
GGGTCACCATCTCCTGCACTGGGAGCAGCTCCAACATCGGGGCAGGTTATGATGTAC
ACTGGTACCAGCAGCTTCCAGGAACAGCCCCCAAACTCCTCATCTATGGTAACATCA
ATCGGCCCTCGGGGGTCCCTGACCGATTCTCTGGCTCCAAGTCTGGCACCTCAGCCT
CCCTGGCCATCACTGGGCTCCAGGCTGAGGATGAGGCTGATTATTACTGCGGAACAT
GGGACAACAGCCTGAGCGCCCATTATGTCTTCGGAACTGGGACCAAGGTCACCGTCC
TAGGTTCTAGAGGTGGTGGTGGTAGCGGCGGCGGCGGCTCTGGTGGTGGTGGATCCC
TCGAGGAGGTGCAGCTGTTGGAGTCTGGGGGAGGCCTGGTCAAGCCTGGGGGGTCC
CTGAGACTCTCCTGTGCAGCCTCTGGATTCACCTTCAATAACTATACCATGAACTGG
 
GTCCGCCAGGCTCCAGGGAAGGGGCTGGAGTGGGTCTCATCCATTAGTAGTAGTAGT
AGTTACATCTACTACGCAGACTCAGTGAAGGGCCGATTCACCATCTCCAGAGACAAC
GCCAAGAACTCACTGTTTCTGCAAATGAACAGCCTGAGAGGCGAGGACACGGCCGT
GTATTACTGTGCGCGCTACTTCTCTGGTTCTGCTGGTGATTACTGGGGTCAAGGTACT
CTGGTGACCGTCTCCTCATTCGTGCCCGTGTTCCTGCCCGCCAAACCTACTACTACCC
CTGCACCTAGGCCTCCCACCCCAGCCCCAACAATCGCCAGCCAGCCTCTGTCTCTGC
GGCCCGAAGCCTGTAGACCTGCTGCCGGCGGAGCCGTGCACACCAGAGGCCTGGAC
TTCGCCTGCGACATCTACATCTGGGCCCCTCTGGCCGGCACCTGTGGCGTGCTGCTG
CTGAGCCTGGTGATCACCCTGTACTGCAACCACCGGAACAAACGGGGCAGAAAGAA
ACTCCTGTATATATTCAAACAACCATTTATGAGACCAGTACAAACTACTCAAGAGGA
AGATGGCTGTAGCTGCCGATTTCCAGAAGAAGAAGAAGGAGGATGTGAACTGAGAG
TGAAGTTCAGCAGATCCGCCGACGCCCCTGCCTACCAGCAGGGACAGAACCAGCTG
TACAACGAGCTGAACCTGGGCAGACGGGAAGAGTACGACGTGCTGGACAAGCGGAG
AGGCCGGGACCCCGAGATGGGCGGAAAGCCCAGACGGAAGAACCCCCAGGAAGGC
CTGTATAACGAACTGCAGAAAGACAAGATGGCCGAGGCCTACAGCGAGATCGGCAT
GAAGGGCGAGCGGAGGCGCGGCAAGGGCCACGATGGCCTGTACCAGGGCCTGAGC
ACCGCCACCAAGGACACCTACGACGCCCTGCACATGCAGGCCCTGCCCCCCAGAGG
ATCCGGAGAGGGAAGGGGCAGCTTATTAACATGTGGCGATGTGGAAGAGAACCCCG
GTCCCATGCTGCTGCTCGTGACCTCTTTACTGTTATGTGAGCTGCCCCACCCCGCTTT
TTTACTGATCCCTCGTAAGGTGTGTAACGGAATCGGCATTGGCGAGTTCAAGGACTC
TTTAAGCATCAACGCCACAAACATCAAGCACTTCAAGAATTGTACCTCCATCAGCGG
CGATTTACACATTCTCCCCGTGGCTTTTCGTGGCGATTCCTTCACCCACACCCCCCCT
CTGGACCCCCAAGAGCTGGACATTTTAAAAACCGTGAAGGAGATCACCGGCTTTCTG
CTGATCCAAGCTTGGCCCGAGAATCGTACCGACCTCCACGCCTTCGAGAATTTAGAG
ATTATTCGTGGAAGGACCAAGCAGCACGGCCAGTTCTCTTTAGCCGTCGTGTCTTTA
AACATTACCAGCCTCGGTTTAAGGTCTTTAAAGGAGATTAGCGACGGCGACGTGATC
ATCTCCGGCAACAAGAACCTCTGCTACGCCAACACCATCAACTGGAAGAAGCTGTTC
 
GGAACCAGCGGCCAAAAGACCAAGATCATCAGCAATCGTGGAGAGAACTCTTGTAA
GGCCACTGGTCAAGTTTGCCACGCCCTCTGTAGCCCCGAAGGATGTTGGGGCCCCGA
GCCTAGGGACTGTGTTAGCTGCAGAAACGTGAGCAGAGGCAGAGAGTGTGTGGACA
AATGCAATTTACTGGAAGGAGAGCCTAGGGAGTTCGTGGAGAACAGCGAATGTATC
CAGTGCCACCCCGAGTGTTTACCTCAAGCCATGAACATCACTTGTACCGGAAGGGGC
CCCGATAACTGCATCCAATGCGCCCACTACATCGACGGACCCCACTGCGTGAAAACT
TGTCCCGCCGGAGTGATGGGAGAGAATAACACTTTAGTGTGGAAGTACGCCGACGC
TGGCCACGTCTGCCATCTGTGCCACCCCAACTGTACCTACGGCTGCACTGGTCCCGG
TTTAGAGGGATGTCCTACCAACGGCCCCAAGATCCCCTCCATCGCCACCGGAATGGT
GGGCGCTCTGTTATTACTGCTGGTGGTGGCTCTGGGCATCGGTTTATTCATG
CAR002 nucleic acid sequence, 2652bp, (SEQ ID NO: 20) 
ATGGCCCTGCCTGTGACAGCTCTGCTCCTCCCTCTGGCCCTGCTGCTCCATGCCGC
CAGACCCCAGGTGCAGCTGGTGGAGTCTGGGGGAGGCCTGGTCAAGCCTGGGGGGT
CCCTGAGACTCTCCTGTGCAGCCTCTGGATTCACCTTCAGTAGCTATAGCATGAACTG
GGTCCGCCAGGCTCCAGGGAAGGGGCTGGAGTGGGTCTCATCCATTAGTAGTAGTA
GTAGTTACATATACTACGCAGACTCAGTGAAGGGCCGATTCACCATCTCCAGAGACA
ACGCCAAGAACTCACTGTATCTGCAAATGAACAGCCTGAGAGCCGAGGACACGGCT
GTGTATTACTGTGCGAGAGGAAATCCGTGGTACGGAGTTGACTACTGGGGCCAGGG
AACCCTGGTCACCGTCTCCTCAACTAGTGGTGGTGGTGGTAGCGGCGGCGGCGGCTC
TGGTGGTGGTGGATCCGAGCTCCAGTCTGTGTTGACGCAGCCGCCCTCAGTGTCTGC
GGCCCCAGGACAGAAGGTCACCATCTCCTGCTCTGGAAGCAACTCCAATATTGGCAA
TAATTATGTATCCTGGTACCAGCAGCTCCCAGGAACAGCCCCCAAACGCCTCATTTA
TGACAATAATAAGCGACCCTCAGGGATTCCTGACCGATTCTCTGGCTCCAAGTCTGG
CACGTCAGCCACCCTGGGCATCACCGGACTCCAGACTGGGGACGAGGCCGATTATT
ACTGCGGAATATGGGATAGCAGCCTGGATGCTTATGTCTTCGGAACTGGGACCAAGG
TCACCGTCCTAGGTTTCGTGCCCGTGTTCCTGCCCGCCAAACCTACTACTACCCCTGC
ACCTAGGCCTCCCACCCCAGCCCCAACAATCGCCAGCCAGCCTCTGTCTCTGCGGCC
 
CGAAGCCTGTAGACCTGCTGCCGGCGGAGCCGTGCACACCAGAGGCCTGGACTTCG
CCTGCGACATCTACATCTGGGCCCCTCTGGCCGGCACCTGTGGCGTGCTGCTGCTGA
GCCTGGTGATCACCCTGTACTGCAACCACCGGAACAAACGGGGCAGAAAGAAACTC
CTGTATATATTCAAACAACCATTTATGAGACCAGTACAAACTACTCAAGAGGAAGAT
GGCTGTAGCTGCCGATTTCCAGAAGAAGAAGGAGGATGTGAACTGAGAGTGAA
GTTCAGCAGATCCGCCGACGCCCCTGCCTACCAGCAGGGACAGAACCAGCTGTACA
ACGAGCTGAACCTGGGCAGACGGGAAGAGTACGACGTGCTGGACAAGCGGAGAGG
CCGGGACCCCGAGATGGGCGGAAAGCCCAGACGGAAGAACCCCCAGGAAGGCCTG
TATAACGAACTGCAGAAAGACAAGATGGCCGAGGCCTACAGCGAGATCGGCATGAA
GGGCGAGCGGAGGCGCGGCAAGGGCCACGATGGCCTGTACCAGGGCCTGAGCACCG
CCACCAAGGACACCTACGACGCCCTGCACATGCAGGCCCTGCCCCCCAGAGGATCC
GGAGAGGGAAGGGGCAGCTTATTAACATGTGGCGATGTGGAAGAGAACCCCGGTCC
CATGCTGCTGCTCGTGACCTCTTTACTGTTATGTGAGCTGCCCCACCCCGCTTTTTTA
CTGATCCCTCGTAAGGTGTGTAACGGAATCGGCATTGGCGAGTTCAAGGACTCTTTA
AGCATCAACGCCACAAACATCAAGCACTTCAAGAATTGTACCTCCATCAGCGGCGAT
TTACACATTCTCCCCGTGGCTTTTCGTGGCGATTCCTTCACCCACACCCCCCCTCTGG
ACCCCCAAGAGCTGGACATTTTAAAAACCGTGAAGGAGATCACCGGCTTTCTGCTGA
TCCAAGCTTGGCCCGAGAATCGTACCGACCTCCACGCCTTCGAGAATTTAGAGATTA
TTCGTGGAAGGACCAAGCAGCACGGCCAGTTCTCTTTAGCCGTCGTGTCTTTAAACA
TTACCAGCCTCGGTTTAAGGTCTTTAAAGGAGATTAGCGACGGCGACGTGATCATCT
CCGGCAACAAGAACCTCTGCTACGCCAACACCATCAACTGGAAGAAGCTGTTCGGA
ACCAGCGGCCAAAAGACCAAGATCATCAGCAATCGTGGAGAGAACTCTTGTAAGGC
CACTGGTCAAGTTTGCCACGCCCTCTGTAGCCCCGAAGGATGTTGGGGCCCCGAGCC
TAGGGACTGTGTTAGCTGCAGAAACGTGAGCAGAGGCAGAGAGTGTGTGGACAAAT
GCAATTTACTGGAAGGAGAGCCTAGGGAGTTCGTGGAGAACAGCGAATGTATCCAG
TGCCACCCCGAGTGTTTACCTCAAGCCATGAACATCACTTGTACCGGAAGGGGCCCC
GATAACTGCATCCAATGCGCCCACTACATCGACGGACCCCACTGCGTGAAAACTTGT
 
CCCGCCGGAGTGATGGGAGAGAATAACACTTTAGTGTGGAAGTACGCCGACGCTGG
CCACGTCTGCCATCTGTGCCACCCCAACTGTACCTACGGCTGCACTGGTCCCGGTTTA
GAGGGATGTCCTACCAACGGCCCCAAGATCCCCTCCATCGCCACCGGAATGGTGGG
CGCTCTGTTATTACTGCTGGTGGTGGCTCTGGGCATCGGTTTATTCATG
CAR003 nucleic acid sequence, 2664bp, (SEQ ID NO: 21) 
ATGGCCCTGCCTGTGACAGCTCTGCTCCTCCCTCTGGCCCTGCTGCTCCATGCCGC
CAGACCCCAGTCTGTGCTGACGCAGCCGCCCTCAGTGTCTGCGGCCCCAGGACAGAA
GGTCACCATCTCCTGCTCTGGAAGCAGCTCCAACATTGGGAATAATTATGTATCCTG
GTACCAGCAGCTCCCAGGAACAGCCCCCAAACTCCTCATTTATGACAATAATAAGCG
ACCCTCAGGGATTCCTGACCGATTCTCTGGCTCCAAGTCTGGCACGTCAGCCACCCT
GGGCATCACCGGACTCCAGACTGGGGACGAGGCCGATTATTACTGCGGAACATGGG
ATAGCAGCCTGAGTGCCGTGGTATTCGGCGGAGGGACCAAGCTGACCGTCCTAGGTT
CTAGAGGTGGTGGTGGTAGCGGCGGCGGCGGCTCTGGTGGTGGATCCCTCGAG
ATGGCCCAGGTGCAGCTGGTGGAGTCTGGGGGAGGCTTGGTACAGCCTGGGGGGTC
CCTGAGACTTTCCTGTGCAGCCTCTGGATTCACCTTTAGCAGCTATGCCATGAGCTGG
GTCCGCCAGACTCCAGGGAAGGGGCTGGAGTGGGTCTCAACTATTACTGATAGTGGT
GATATCACATACTACACAGCCTCCGTGCAGGGCCGGTTCACCATCTCCAGAGACAAT
TCCAAGAACACGTTGTATCTGCAAATGAACAGCCTGAGAGCCGAGGACACGGCCGT
ATATTACTGTGCGCGCATGTCTTCTCATTGGTACTTCTCTGCAGATTACTGGGGTCAA
GGTACTCTGGTGACCGTCTCCTCATTCGTGCCCGTGTTCCTGCCCGCCAAACCTACTA
CTACCCCTGCACCTAGGCCTCCCACCCCAGCCCCAACAATCGCCAGCCAGCCTCTGT
CTCTGCGGCCCGAAGCCTGTAGACCTGCTGCCGGCGGAGCCGTGCACACCAGAGGC
CTGGACTTCGCCTGCGACATCTACATCTGGGCCCCTCTGGCCGGCACCTGTGGCGTG
CTGCTGCTGAGCCTGGTGATCACCCTGTACTGCAACCACCGGAACAAACGGGGCAG
AAAGAAACTCCTGTATATATTCAAACAACCATTTATGAGACCAGTACAAACTACTCA
AGAGGAAGATGGCTGTAGCTGCCGATTTCCAGAAGAAGAAGAAGGAGGATGTGAAC
TGAGAGTGAAGTTCAGCAGATCCGCCGACGCCCCTGCCTACCAGCAGGGACAGAAC
 
CAGCTGTACAACGAGCTGAACCTGGGCAGACGGGAAGAGTACGACGTGCTGGACAA
GCGGAGAGGCCGGGACCCCGAGATGGGCGGAAAGCCCAGACGGAAGAACCCCCAG
GAAGGCCTGTATAACGAACTGCAGAAAGACAAGATGGCCGAGGCCTACAGCGAGAT
CGGCATGAAGGGCGAGCGGAGGCGCGGCAAGGGCCACGATGGCCTGTACCAGGGC
CTGAGCACCGCCACCAAGGACACCTACGACGCCCTGCACATGCAGGCCCTGCCCCCC
AGAGGATCCGGAGAGGGAAGGGGCAGCTTATTAACATGTGGCGATGTGGAAGAGAA
CCCCGGTCCCATGCTGCTGCTCGTGACCTCTTTACTGTTATGTGAGCTGCCCCACCCC
GCTTTTTTACTGATCCCTCGTAAGGTGTGTAACGGAATCGGCATTGGCGAGTTCAAG
GACTCTTTAAGCATCAACGCCACAAACATCAAGCACTTCAAGAATTGTACCTCCATC
AGCGGCGATTTACACATTCTCCCCGTGGCTTTTCGTGGCGATTCCTTCACCCACACCC
CCCCTCTGGACCCCCAAGAGCTGGACATTTTAAAAACCGTGAAGGAGATCACCGGCT
TTCTGCTGATCCAAGCTTGGCCCGAGAATCGTACCGACCTCCACGCCTTCGAGAATT
TAGAGATTATTCGTGGAAGGACCAAGCAGCACGGCCAGTTCTCTTTAGCCGTCGTGT
CTTTAAACATTACCAGCCTCGGTTTAAGGTCTTTAAAGGAGATTAGCGACGGCGACG
TGATCATCTCCGGCAACAAGAACCTCTGCTACGCCAACACCATCAACTGGAAGAAG
CTGTTCGGAACCAGCGGCCAAAAGACCAAGATCATCAGCAATCGTGGAGAGAACTC
TTGTAAGGCCACTGGTCAAGTTTGCCACGCCCTCTGTAGCCCCGAAGGATGTTGGGG
CCCCGAGCCTAGGGACTGTGTTAGCTGCAGAAACGTGAGCAGAGGCAGAGAGTGTG
TGGACAAATGCAATTTACTGGAAGGAGAGCCTAGGGAGTTCGTGGAGAACAGCGAA
TGTATCCAGTGCCACCCCGAGTGTTTACCTCAAGCCATGAACATCACTTGTACCGGA
AGGGGCCCCGATAACTGCATCCAATGCGCCCACTACATCGACGGACCCCACTGCGTG
AAAACTTGTCCCGCCGGAGTGATGGGAGAGAATAACACTTTAGTGTGGAAGTACGC
CGACGCTGGCCACGTCTGCCATCTGTGCCACCCCAACTGTACCTACGGCTGCACTGG
TCCCGGTTTAGAGGGATGTCCTACCAACGGCCCCAAGATCCCCTCCATCGCCACCGG
AATGGTGGGCGCTCTGTTATTACTGCTGGTGGTGGCTCTGGGCATCGGTTTATTCATG
CAR001 protein sequence, 886 aa (SEQ ID NO: 22) 
MALPVTALLLPLALLLHAARPQSVVTQPPSVSGAPGQRVTISCTGSSSNIGAGYDVH
WYQQLPGTAPKLLIYGNINRPSGVPDRFSGSKSGTSASLAITGLQAEDEADYYCGTWDN
SLSAHYVFGTGTKVTVLGSRGGGGSGGGGSGGGGSLEEVQLLESGGGLVKPGGSLRLSC
AASGFTFNNYTMNWVRQAPGKGLEWVSSISSSSSYIYYADSVKGRFTISRDNAKNSLFL
QMNSLRGEDTAVYYCARYFSGSAGDYWGQGTLVTVSSFVPVFLPAKPTTTPAPRPPTPA
PTIASQPLSLRPEACRPAAGGAVHTRGLDFACDIYIWAPLAGTCGVLLLSLVITLYCNHR
NKRGRKKLLYIFKQPFMRPVQTTQEEDGCSCRFPEEEEGGCELRVKFSRSADAPAYQQG
QNQLYNELNLGRREEYDVLDKRRGRDPEMGGKPRRKNPQEGLYNELQKDKMAEAYSE
IGMKGERRRGKGHDGLYQGLSTATKDTYDALHMQALPPRGSGEGRGSLLTCGDVEENP
GPMLLLVTSLLLCELPHPAFLLIPRKVCNGIGIGEFKDSLSINATNIKHFKNCTSISGDLHIL
PVAFRGDSFTHTPPLDPQELDILKTVKEITGFLLIQAWPENRTDLHAFENLEIIRGRTKQH
GQFSLAVVSLNITSLGLRSLKEISDGDVIISGNKNLCYANTINWKKLFGTSGQKTKIISNRG
ENSCKATGQVCHALCSPEGCWGPEPRDCVSCRNVSRGRECVDKCNLLEGEPREFVENSE
CIQCHPECLPQAMNITCTGRGPDNCIQCAHYIDGPHCVKTCPAGVMGENNTLVWKYAD
AGHVCHLCHPNCTYGCTGPGLEGCPTNGPKIPSIATGMVGALLLLLVVALGIGLFM
CAR002 protein sequence, 884aa (SEQ ID NO: 23) 
MALPVTALLLPLALLLHAARPQVQLVESGGGLVKPGGSLRLSCAASGFTFSSYSMN
WVRQAPGKGLEWVSSISSSSSYIYYADSVKGRFTISRDNAKNSLYLQMNSLRAEDTAVY
YCARGNPWYGVDYWGQGTLVTVSSTSGGGGSGGGGSGGGGSELQSVLTQPPSVSAAP
GQKVTISCSGSNSNIGNNYVSWYQQLPGTAPKRLIYDNNKRPSGIPDRFSGSKSGTSATL
GITGLQTGDEADYYCGIWDSSLDAYVFGTGTKVTVLGFVPVFLPAKPTTTPAPRPPTPAP
TIASQPLSLRPEACRPAAGGAVHTRGLDFACDIYIWAPLAGTCGVLLLSLVITLYCNHRN
KRGRKKLLYIFKQPFMRPVQTTQEEDGCSCRFPEEEEGGCELRVKFSRSADAPAYQQGQ
NQLYNELNLGRREEYDVLDKRRGRDPEMGGKPRRKNPQEGLYNELQKDKMAEAYSEI
GMKGERRRGKGHDGLYQGLSTATKDTYDALHMQALPPRGSGEGRGSLLTCGDVEENP
GPMLLLVTSLLLCELPHPAFLLIPRKVCNGIGIGEFKDSLSINATNIKHFKNCTSISGDLHIL
PVAFRGDSFTHTPPLDPQELDILKTVKEITGFLLIQAWPENRTDLHAFENLEIIRGRTKQH
 
GQFSLAVVSLNITSLGLRSLKEISDGDVIISGNKNLCYANTINWKKLFGTSGQKTKIISNRG
ENSCKATGQVCHALCSPEGCWGPEPRDCVSCRNVSRGRECVDKCNLLEGEPREFVENSE
CIQCHPECLPQAMNITCTGRGPDNCIQCAHYIDGPHCVKTCPAGVMGENNTLVWKYAD
AGHVCHLCHPNCTYGCTGPGLEGCPTNGPKIPSIATGMVGALLLLLVVALGIGLFM
CAR003 protein sequence, 888aa (SEQ ID NO: 24) 
MALPVTALLLPLALLLHAARPQSVLTQPPSVSAAPGQKVTISCSGSSSNIGNNYVSWY
QQLPGTAPKLLIYDNNKRPSGIPDRFSGSKSGTSATLGITGLQTGDEADYYCGTWDSSLS
AVVFGGGTKLTVLGSRGGGGSGGGGSGGGGSLEMAQVQLVESGGGLVQPGGSLRLSC
AASGFTFSSYAMSWVRQTPGKGLEWVSTITDSGDITYYTASVQGRFTISRDNSKNTLYL
QMNSLRAEDTAVYYCARMSSHWYFSADYWGQGTLVTVSSFVPVFLPAKPTTTPAPRPP
TPAPTIASQPLSLRPEACRPAAGGAVHTRGLDFACDIYIWAPLAGTCGVLLLSLVITLYCN
HRNKRGRKKLLYIFKQPFMRPVQTTQEEDGCSCRFPEEEEGGCELRVKFSRSADAPAYQ
QGQNQLYNELNLGRREEYDVLDKRRGRDPEMGGKPRRKNPQEGLYNELQKDKMAEA
YSEIGMKGERRRGKGHDGLYQGLSTATKDTYDALHMQALPPRGSGEGRGSLLTCGDVE
ENPGPMLLLVTSLLLCELPHPAFLLIPRKVCNGIGIGEFKDSLSINATNIKHFKNCTSISGDL
HILPVAFRGDSFTHTPPLDPQELDILKTVKEITGFLLIQAWPENRTDLHAFENLEIIRGRTK
QHGQFSLAVVSLNITSLGLRSLKEISDGDVIISGNKNLCYANTINWKKLFGTSGQKTKIIS
NRGENSCKATGQVCHALCSPEGCWGPEPRDCVSCRNVSRGRECVDKCNLLEGEPREFV
ENSECIQCHPECLPQAMNITCTGRGPDNCIQCAHYIDGPHCVKTCPAGVMGENNTLVWK
YADAGHVCHLCHPNCTYGCTGPGLEGCPTNGPKIPSIATGMVGALLLLLVVALGIGLFM
Scfv10 nucleic acid sequence (SEQ ID NO: 25) 
CAGTCTGTCGTGACGCAGCCGCCCTCAGTGTCTGGGGCCCCAGGGCAGAGGGTC
ACCATCTCCTGCACTGGGAGCAGCTCCAACATCGGGGCAGGTTATGATGTACACTGG
TACCAGCAGCTTCCAGGAACAGCCCCCAAACTCCTCATCTATGGTAACATCAATCGG
CCCTCGGGGGTCCCTGACCGATTCTCTGGCTCCAAGTCTGGCACCTCAGCCTCCCTG
GCCATCACTGGGCTCCAGGCTGAGGATGAGGCTGATTATTACTGCGGAACATGGGAC
AACAGCCTGAGCGCCCATTATGTCTTCGGAACTGGGACCAAGGTCACCGTCCTAGGT
 
TCTAGAGGTGGTGGTGGTAGCGGCGGCGGCTCTGGTGGTGGATCCCTCGAG

GAGGTGCAGCTGTTGGAGTCTGGGGGAGGCCTGGTCAAGCCTGGGGGGTCCCTGAG

ACTCTCCTGTGCAGCCTCTGGATTCACCTTCAATAACTATACCATGAACTGGGTCCGC

CAGGCTCCAGGGAAGGGGCTGGAGTGGGTCTCATCCATTAGTAGTAGTAGTAGTTAC

ATCTACTACGCAGACTCAGTGAAGGGCCGATTCACCATCTCCAGAGACAACGCCAA

GAACTCACTGTTTCTGCAAATGAACAGCCTGAGAGGCGAGGACACGGCCGTGTATTA

CTGTGCGCGCTACTTCTCTGGTTCTGCTGGTGATTACTGGGGTCAAGGTACTCTGGTG

ACCGTCTCCTCA
10VL (i.e., the VL of Scfv10 in CAR001) nucleic acid sequence: 339 bp (SEQ ID NO: 26) 
CAGTCTGTCGTGACGCAGCCGCCCTCAGTGTCTGGGGCCCCAGGGCAGAGGGTC
ACCATCTCCTGCACTGGGAGCAGCTCCAACATCGGGGCAGGTTATGATGTACACTGG
TACCAGCAGCTTCCAGGAACAGCCCCCAAACTCCTCATCTATGGTAACATCAATCGG
CCCTCGGGGGTCCCTGACCGATTCTCTGGCTCCAAGTCTGGCACCTCAGCCTCCCTG
GCCATCACTGGGCTCCAGGCTGAGGATGAGGCTGATTATTACTGCGGAACATGGGAC
AACAGCCTGAGCGCCCATTATGTCTTCGGAACTGGGACCAAGGTCACCGTCCTAGGT
10VH (i.e., the VH of Scfv10 in CAR001) protein sequence: 354 bp (SEQ ID NO: 27) 
GAGGTGCAGCTGTTGGAGTCTGGGGGAGGCCTGGTCAAGCCTGGGGGGTCCCTG
AGACTCTCCTGTGCAGCCTCTGGATTCACCTTCAATAACTATACCATGAACTGGGTCC
GCCAGGCTCCAGGGAAGGGGCTGGAGTGGGTCTCATCCATTAGTAGTAGTAGTAGTT
ACATCTACTACGCAGACTCAGTGAAGGGCCGATTCACCATCTCCAGAGACAACGCC
AAGAACTCACTGTTTCTGCAAATGAACAGCCTGAGAGGCGAGGACACGGCCGTGTA
TTACTGTGCGCGCTACTTCTCTGGTTCTGCTGGTGATTACTGGGGTCAAGGTACTCTG
GTGACCGTCTCCTCA
Scfv10 protein sequence (SEQ ID NO: 28) 
QSVVTQPPSVSGAPGQRVTISCTGSSSNIGAGYDVHWYQQLPGTAPKLLIYGNINRPS
GVPDRFSGSKSGTSASLAITGLQAEDEADYYCGTWDNSLSAHYVFGTGTKVTVLGSRG
GGGSGGGGSGGGGSLEEVQLLESGGGLVKPGGSLRLSCAASGFTFNNYTMNWVRQAPG
 
KGLEWVSSISSSSSYIYYADSVKGRFTISRDNAKNSLFLQMNSLRGEDTAVYYCARYFSG

SAGDYWGQGTLVTVSS
10VL (i.e., the VL of Scfv10 in CAR001) protein sequence: (SEQ ID NO: 29) 
QSVVTQPPSVSGAPGQRVTISCTGSSSNIGAGYDVHWYQQLPGTAPKLLIYGNINRPS
GVPDRFSGSKSGTSASLAITGLQAEDEADYYCGTWDNSLSAHYVFGTGTKVTVLG
10VH (i.e., the VH of Scfv10 in CAR001) protein sequence: (SEQ ID NO: 30) 
EVQLLESGGGLVKPGGSLRLSCAASGFTFNNYTMNWVRQAPGKGLEWVSSISSSSSY
IYYADSVKGRFTISRDNAKNSLFLQMNSLRGEDTAVYYCARYFSGSAGDYWGQGTLVT
VSS
Scfv32 nucleic acid sequence (SEQ ID NO: 31) 
CAGGTGCAGCTGGTGGAGTCTGGGGGAGGCCTGGTCAAGCCTGGGGGGTCCCTG
AGACTCTCCTGTGCAGCCTCTGGATTCACCTTCAGTAGCTATAGCATGAACTGGGTC
CGCCAGGCTCCAGGGAAGGGGCTGGAGTGGGTCTCATCCATTAGTAGTAGTAGT
TACATATACTACGCAGACTCAGTGAAGGGCCGATTCACCATCTCCAGAGACAACGCC
AAGAACTCACTGTATCTGCAAATGAACAGCCTGAGAGCCGAGGACACGGCTGTGTA
TTACTGTGCGAGAGGAAATCCGTGGTACGGAGTTGACTACTGGGGCCAGGGAACCC
TGGTCACCGTCTCCTCAACTAGTGGTGGTGGTGGTAGCGGCGGCGGCGGCTCTGGTG
GTGGTGGATCCGAGCTCCAGTCTGTGTTGACGCAGCCGCCCTCAGTGTCTGCGGCCC
CAGGACAGAAGGTCACCATCTCCTGCTCTGGAAGCAACTCCAATATTGGCAATAATT
ATGTATCCTGGTACCAGCAGCTCCCAGGAACAGCCCCCAAACGCCTCATTTATGACA
ATAATAAGCGACCCTCAGGGATTCCTGACCGATTCTCTGGCTCCAAGTCTGGCACGT
CAGCCACCCTGGGCATCACCGGACTCCAGACTGGGGACGAGGCCGATTATTACTGC
GGAATATGGGATAGCAGCCTGGATGCTTATGTCTTCGGAACTGGGACCAAGGTCACC
GTCCTAGGT
32VL (i.e., the VL of Scfv32 in CAR002) nucleic acid sequence: 333 bp (SEQ ID NO: 32) 
CAGTCTGTGTTGACGCAGCCGCCCTCAGTGTCTGCGGCCCCAGGACAGAAGGTCA
CCATCTCCTGCTCTGGAAGCAACTCCAATATTGGCAATAATTATGTATCCTGGTACCA
 
GCAGCTCCCAGGAACAGCCCCCAAACGCCTCATTTATGACAATAATAAGCGACCCTC
AGGGATTCCTGACCGATTCTCTGGCTCCAAGTCTGGCACGTCAGCCACCCTGGGCAT
CACCGGACTCCAGACTGGGGACGAGGCCGATTATTACTGCGGAATATGGGATAGCA
GCCTGGATGCTTATGTCTTCGGAACTGGGACCAAGGTCACCGTCCTAGGT
32 VH (i.e., the VH of Scfv32 in CAR002) nucleic acid sequence: 354 bp (SEQ ID NO: 33) 
CAGGTGCAGCTGGTGGAGTCTGGGGGAGGCCTGGTCAAGCCTGGGGGGTCCCTG
AGACTCTCCTGTGCAGCCTCTGGATTCACCTTCAGTAGCTATAGCATGAACTGGGTC
CGCCAGGCTCCAGGGAAGGGGCTGGAGTGGGTCTCATCCATTAGTAGTAGTAGTAGT
TACATATACTACGCAGACTCAGTGAAGGGCCGATTCACCATCTCCAGAGACAACGCC
AAGAACTCACTGTATCTGCAAATGAACAGCCTGAGAGCCGAGGACACGGCTGTGTA
TTACTGTGCGAGAGGAAATCCGTGGTACGGAGTTGACTACTGGGGCCAGGGAACCC
TGGTCACCGTCTCCTCA
Scfv32 protein sequence (SEQ ID NO: 34) 
QVQLVESGGGLVKPGGSLRLSCAASGFTFSSYSMNWVRQAPGKGLEWVSSISSSSSY
IYYADSVKGRFTISRDNAKNSLYLQMNSLRAEDTAVYYCARGNPWYGVDYWGQGTLV
TVSSTSGGGGSGGGGSGGGGSELQSVLTQPPSVSAAPGQKVTISCSGSNSNIGNNYVSW
YQQLPGTAPKRLIYDNNKRPSGIPDRFSGSKSGTSATLGITGLQTGDEADYYCGIWDSSL
DAYVFGTGTKVTVLG
32 VL (i.e., the VL of Scfv32 in CAR002) protein sequence: 111 aa (SEQ ID NO: 35) 
QSVLTQPPSVSAAPGQKVTISCSGSNSNIGNNYVSWYQQLPGTAPKRLIYDNNKRPSG
IPDRFSGSKSGTSATLGITGLQTGDEADYYCGIWDSSLDAYVFGTGTKVTVLG
32VH (i.e., the VH of Scfv32 in CAR002) protein sequence: 118 aa (SEQ ID NO: 36) 
QVQLVESGGGLVKPGGSLRLSCAASGFTFSSYSMNWVRQAPGKGLEWVSSISSSSSY
IYYADSVKGRFTISRDNAKNSLYLQMNSLRAEDTAVYYCARGNPWYGVDYWGQGTLV
TVSS
Scfv35 nucleic acid sequence (SEQ ID NO: 37) 
CAGTCTGTGCTGACGCAGCCGCCCTCAGTGTCTGCGGCCCCAGGACAGAAGGTC
ACCATCTCCTGCTCTGGAAGCAGCTCCAACATTGGGAATAATTATGTATCCTGGTAC
CAGCAGCTCCCAGGAACAGCCCCCAAACTCCTCATTTATGACAATAATAAGCGACCC
TCAGGGATTCCTGACCGATTCTCTGGCTCCAAGTCTGGCACGTCAGCCACCCTGGGC
ATCACCGGACTCCAGACTGGGGACGAGGCCGATTATTACTGCGGAACATGGGATAG
CAGCCTGAGTGCCGTGGTATTCGGCGGAGGGACCAAGCTGACCGTCCTAGGTTCTAG
AGGTGGTGGTGGTAGCGGCGGCGGCGGCTCTGGTGGTGGTGGATCCCTCGAGATGG
CCCAGGTGCAGCTGGTGGAGTCTGGGGGAGGCTTGGTACAGCCTGGGGGGTCCCTG
AGACTTTCCTGTGCAGCCTCTGGATTCACCTTTAGCAGCTATGCCATGAGCTGGGTCC
GCCAGACTCCAGGGAAGGGGCTGGAGTGGGTCTCAACTATTACTGATAGTGGTGAT
ATCACATACTACACAGCCTCCGTGCAGGGCCGGTTCACCATCTCCAGAGACAATTCC
AAGAACACGTTGTATCTGCAAATGAACAGCCTGAGAGCCGAGGACACGGCCGTATA
TTACTGTGCGCGCATGTCTTCTCATTGGTACTTCTCTGCAGATTACTGGGGTCAAGGT
ACTCTGGTGACCGTCTCCTCA
35VL (i.e., the VL of Scfv35 in CAR003) nucleic acid sequence: 333 bp (SEQ ID NO: 38) 
CAGTCTGTGCTGACGCAGCCGCCCTCAGTGTCTGCGGCCCCAGGACAGAAGGTC
ACCATCTCCTGCTCTGGAAGCAGCTCCAACATTGGGAATAATTATGTATCCTGGTAC
CAGCAGCTCCCAGGAACAGCCCCCAAACTCCTCATTTATGACAATAATAAGCGACCC
TCAGGGATTCCTGACCGATTCTCTGGCTCCAAGTCTGGCACGTCAGCCACCCTGGGC
ATCACCGGACTCCAGACTGGGGACGAGGCCGATTATTACTGCGGAACATGGGATAG
CAGCCTGAGTGCCGTGGTATTCGGCGGAGGGACCAAGCTGACCGTCCTAGGT
35VH (i.e., the VH of Scfv35 in CAR003) nucleic acid sequence: 366 bp (SEQ ID NO: 39) 
ATGGCCCAGGTGCAGCTGGTGGAGTCTGGGGGAGGCTTGGTACAGCCTGGGGGG
TCCCTGAGACTTTCCTGTGCAGCCTCTGGATTCACCTTTAGCAGCTATGCCATGAGCT
GGGTCCGCCAGACTCCAGGGAAGGGGCTGGAGTGGGTCTCAACTATTACTGATAGT
GGTGATATCACATACTACACAGCCTCCGTGCAGGGCCGGTTCACCATCTCCAGAGAC
AATTCCAAGAACACGTTGTATCTGCAAATGAACAGCCTGAGAGCCGAGGACACGGC
 
CGTATATTACTGTGCGCGCATGTCTTCTCATTGGTACTTCTCTGCAGATTACTGGGGT
CAAGGTACTCTGGTGACCGTCTCCTCA
Scfv35 protein sequence (SEQ ID NO: 40) 
QSVLTQPPSVSAAPGQKVTISCSGSSSNIGNNYVSWYQQLPGTAPKLLIYDNNKRPSG
IPDRFSGSKSGTSATLGITGLQTGDEADYYCGTWDSSLSAVVFGGGTKLTVLGSRGGGG
SGGGGSGGGGSLEMAQVQLVESGGGLVQPGGSLRLSCAASGFTFSSYAMSWVRQTPGK
GLEWVSTITDSGDITYYTASVQGRFTISRDNSKNTLYLQMNSLRAEDTAVYYCARMSSH
WYFSADYWGQGTLVTVSS
35 VL (i.e., the VL of Scfv35 in CAR003) protein sequence: 111 aa (SEQ ID NO: 41) 
QSVLTQPPSVSAAPGQKVTISCSGSSSNIGNNYVSWYQQLPGTAPKLLIYDNNKRPSG
IPDRFSGSKSGTSATLGITGLQTGDEADYYCGTWDSSLSAVVFGGGTKLTVLG
35 VH (i.e., the VH of Scfv35 in CAR003) protein sequence: 122 aa (SEQ ID NO: 42) 
MAQVQLVESGGGLVQPGGSLRLSCAASGFTFSSYAMSWVRQTPGKGLEWVSTITDS
GDITYYTASVQGRFTISRDNSKNTLYLQMNSLRAEDTAVYYCARMSSHWYFSADYWG
QGTLVTVSS
CDRs of Scfv10 (clone 10) antibody in CAR001:
   HCDR1 having an amino acid sequence of GFTFNNYT (SEQ ID NO: 43)
   HCDR2 having an amino acid sequence of ISSSSSYI (SEQ ID NO: 44)
   HCDR3 having an amino acid sequence of ARYFSGSAGDY (SEQ ID NO: 45)
   LCDR1 having an amino acid sequence of SSNIGAGYD (SEQ ID NO: 46)
   LCDR2 having an amino acid sequence of GNI (SEQ ID NO: 47)
   LCDR3 having an amino acid sequence of GTWDNSLSAHYV (SEQ ID NO: 48)
CDRs of Scfv32 (clone 32) antibody in CAR002:
   HCDR1 having an amino acid sequence of GFTFSSYS (SEQ ID NO: 49)
   HCDR2 having an amino acid sequence of ISSSSSYI (SEQ ID NO: 50)
   HCDR3 having an amino acid sequence of ARYFSGSAGDY (SEQ ID NO: 51)
   LCDR1 having an amino acid sequence of NSNIGNNY (SEQ ID NO: 52)
   LCDR2 having an amino acid sequence of DNN (SEQ ID NO: 53)
   LCDR3 having an amino acid sequence of GIWDSSLDAYV (SEQ ID NO: 54)
CDRs of Scfv35 (clone 35) antibody in CAR003:
   HCDR1 having an amino acid sequence of GFTFSSYA (SEQ ID NO: 55)
   HCDR2 having an amino acid sequence of ITDSGDIT (SEQ ID NO: 56)
   HCDR3 having an amino acid sequence of ARMSSHWYFSADY (SEQ ID NO: 57)
   LCDR1 having an amino acid sequence of SSNIGNNY (SEQ ID NO: 58)
   LCDR2 having an amino acid sequence of DNN (SEQ ID NO: 59)
   LCDR3 having an amino acid sequence of GTWDSSLSAVV (SEQ ID NO: 60)
CDRs of Scfv6 (clone 6) antibody in CAR004:
   HCDR1 having an amino acid sequence of GGTFSSNA (SEQ ID NO: 61)
   HCDR2 having an amino acid sequence of IIPMFGTT (SEQ ID NO: 62)
   HCDR3 having an amino acid sequence of ARDGGGRHPYYYYGMDV (SEQ ID NO: 63)
   LCDR1 having an amino acid sequence of SGSIARNY (SEQ ID NO: 64)
   LCDR2 having an amino acid sequence of EDN (SEQ ID NO: 65)
   LCDR3 having an amino acid sequence of QSYDDNTSWV (SEQ ID NO: 66)
CDRs of Scfv7 (clone 7) antibody in CAR005:
   HCDR1 having an amino acid sequence of GYSFTSYW (SEQ ID NO: 67)
   HCDR2 having an amino acid sequence of IYPDDSDT (SEQ ID NO: 68)
   HCDR3 having an amino acid sequence of ARMSLTSYLATDG (SEQ ID NO: 69)
   LCDR1 having an amino acid sequence of SSNIGNND (SEQ ID NO: 70)
   LCDR2 having an amino acid sequence of DND (SEQ ID NO: 71)
   LCDR3 having an amino acid sequence of AVWDSSLSAAV (SEQ ID NO: 72)
CDRs of Scfv9 (clone 9) antibody in CAR006:
   HCDR1 having an amino acid sequence of TYTFTNYY (SEQ ID NO: 73)
   HCDR2 having an amino acid sequence of INPSGDTT (SEQ ID NO: 74)
   HCDR3 having an amino acid sequence of ARLSWYWGGSFDD (SEQ ID NO: 75)
   LCDR1 having an amino acid sequence of SDDIGNYKR (SEQ ID NO: 76)
   LCDR2 having an amino acid sequence of EVT (SEQ ID NO: 77)
   LCDR3 having an amino acid sequence of SSYASGDTYV (SEQ ID NO: 78)
   ScFv6 DNA sequence: (SEQ ID NO: 79) 
AATTTTATGCTGACTCAGCCCCACTCTGTGTCGGAGTCTCCGGGGAAGACGGTAA
CCATCTCCTGCACCGGCAATAGTGGCAGCATTGCCAGAAACTATGTGCAGTGGTACC
AACAGCGCCCGGGCAGTGCCCCCACCATGCTGATCTATGAGGATAATCGAAGACCC
TCTGGGGTCCCTGATCGGTTCTCTGGCTCCATCGACAGCTCCTCCAACTCTGCCTCCC
TCACCATCTCTGGACTGAAGACTGAGGACGAGGCTGACTATTACTGTCAGTCTTATG
ATGACAACACTTCTTGGGTGTTCGGCGGAGGGACCAAGCTGACCGTCCTAGGTTCTA
GAGGTGGTGGTGGTAGCGGCGGCGGCGGCTCTGGTGGTGGTGGATCCCTCGAGATG
GCCGAGGTCCAGCTGGTACAGTCTGGGGCTGAGGTGAAGAAGCCTGGGTCCTCGGT
GAAGGTCTCCTGCAAGGCTTCTGGAGGGACCTTCAGCAGTAATGCTATCACCTGGGT
GCGACAGGCCCCTGGACAAGGCCTTGAGTGGATGGGAGGGATCATCCCTATGTTTGG
TACAACAGACTACGCACAGAAGTTCCAGGGCAGAGTCACGATGACCGCGGACGAAT
CCACGAGCACAGCCTACATGGAACTGCGCAGCCTGAGATCTGAAGACACGGCCGTG
TATTACTGTGCGAGAGATGGGGGGGGACGACACCCCTACTACTACTACGGTATGGAC
GTCTGGGGCCAAGGGACCACGGTCACCGTCTCCTCA
   10VL (i.e., the VL of Scfv6 in CAR004) DNA sequence: (SEQ ID NO: 80) 
AATTTTATGCTGACTCAGCCCCACTCTGTGTCGGAGTCTCCGGGGAAGACGGTAA
CCATCTCCTGCACCGGCAATAGTGGCAGCATTGCCAGAAACTATGTGCAGTGGTACC
AACAGCGCCCGGGCAGTGCCCCCACCATGCTGATCTATGAGGATAATCGAAGACCC
TCTGGGGTCCCTGATCGGTTCTCTGGCTCCATCGACAGCTCCTCCAACTCTGCCTCCC
TCACCATCTCTGGACTGAAGACTGAGGACGAGGCTGACTATTACTGTCAGTCTTATG
ATGACAACACTTCTTGGGTGTTCGGCGGAGGGACCAAGCTGACCGTCCTAGGT
   6VH (i.e., the VH of Scfv6 in CAR004) DNA sequence (SEQ ID NO: 81) 
ATGGCCGAGGTCCAGCTGGTACAGTCTGGGGCTGAGGTGAAGAAGCCTGGGTCC
TCGGTGAAGGTCTCCTGCAAGGCTTCTGGAGGGACCTTCAGCAGTAATGCTATCACC
TGGGTGCGACAGGCCCCTGGACAAGGCCTTGAGTGGATGGGAGGGATCATCCCTAT
GTTTGGTACAACAGACTACGCACAGAAGTTCCAGGGCAGAGTCACGATGACCGCGG
ACGAATCCACGAGCACAGCCTACATGGAACTGCGCAGCCTGAGATCTGAAGACACG
GCCGTGTATTACTGTGCGAGAGATGGGGGGGGACGACACCCCTACTACTACTACGGT
ATGGACGTCTGGGGCCAAGGGACCACGGTCACCGTCTCCTCA
   ScFv7 DNA sequence (SEQ ID NO: 82) 
CAGTCTGTCGTGACGCAGCCGCCCTCAGTGTCTGCGGCCCCAGGACAGAAGGCC
ACCATCTCCTGCTCTGGAAGCAGCTCCAACATTGGGAATAATGATGTATCCTGGTAC
CAGCAGCTTCCAGGAACAGCCCCCAAACTCCTCATTCAGGACAATGATAAGCGACC
CTCAGGTATTCCTGACCGATTCTCTGGCTCCAAGTCTGGCACGTCAGCCACCCTGGG
CATCACCGGACTCCAGACTGGGGACGAGGCCGATTATTACTGCGCAGTATGGGATA
GCAGCCTGAGTGCTGCCGTCTTCGGACCTGGGACCAAGGTCACCGTCCTAGGTTCTA
GAGGTGGTGGTGGTAGCGGCGGCGGCGGCTCTGGTGGTGGTGGATCCCTCGAGATG
GCCGAAGTGCAGCTGGTGCAGTCTGGAGCAGAGGTGAAAAAGCCCGGGGAGTCTCT
GAAGATCTCCTGTAAGGGTTCTGGATACAGCTTTACCAGCTACTGGATCGGCTGGGT
GCGCCAGATGCCCGGGAAAGGCCTGGAGTGGATGGGAATCATCTATCCTGATGACT
CTGATACCACGTACAGCCCGTCCTTCCAAGGCCAGGTCACCATGTCAGTCGACAAGT
CCATCAGCACCGCCTACCTGCAGTGGAACAGCCTGCAGGCCTCGGACACCGCCATGT
ATTACTGTGCGCGCATGTCTCTGACCTCTTACCTGGCAACCGATGGTTGGGGTCAAG
GTACTCTGGTGACCGTCTCCTCA
   7VL (i.e., the VL of Scfv7 in CAR005) DNA sequence (SEQ ID NO: 83) 
CAGTCTGTCGTGACGCAGCCGCCCTCAGTGTCTGCGGCCCCAGGACAGAAGGCC
ACCATCTCCTGCTCTGGAAGCAGCTCCAACATTGGGAATAATGATGTATCCTGGTAC
CAGCAGCTTCCAGGAACAGCCCCCAAACTCCTCATTCAGGACAATGATAAGCGACC
CTCAGGTATTCCTGACCGATTCTCTGGCTCCAAGTCTGGCACGTCAGCCACCCTGGG
 
CATCACCGGACTCCAGACTGGGGACGAGGCCGATTATTACTGCGCAGTATGGGATA
GCAGCCTGAGTGCTGCCGTCTTCGGACCTGGGACCAAGGTCACCGTCCTAGGT
   7VH (i.e., the VH of Scfv7 in CAR005) DNA sequence (SEQ ID NO: 84) 
ATGGCCGAAGTGCAGCTGGTGCAGTCTGGAGCAGAGGTGAAAAAGCCCGGGGA
GTCTCTGAAGATCTCCTGTAAGGGTTCTGGATACAGCTTTACCAGCTACTGGATCGG
CTGGGTGCGCCAGATGCCCGGGAAAGGCCTGGAGTGGATGGGAATCATCTATCCTG
ATGACTCTGATACCACGTACAGCCCGTCCTTCCAAGGCCAGGTCACCATGTCAGTCG
ACAAGTCCATCAGCACCGCCTACCTGCAGTGGAACAGCCTGCAGGCCTCGGACACC
GCCATGTATTACTGTGCGCGCATGTCTCTGACCTCTTACCTGGCAACCGATGGTTGGG
GTCAAGGTACTCTGGTGACCGTCTCCTCA
   ScFv9 DNA sequence (SEQ ID NO: 85) 
CAGCCTGTGCTGACTCAGCCTCCCTCCGTGTCCGGGTCTCCTGGACAGTCAGTCA
CCATCTCCTGCACTGGAACCAGCGATGACATTGGAAATTATAAGCGTGTCTCCTGGT
ACCAGCAGCCCCCTGGCACAGCCCCCAAACTCATGATTTATGAGGTCACTAATCGGC
CCCCAGGGGTCCCTAATCGCTTCTCTGGGTCCAAGTCTGGCAACACGGCCTCCCTGA
CCATCTCTGGGCTCCAGGCTGAGGACGAGGCTGATTATTACTGCAGCTCATATGCAA
GCGGCGACACTTATGTCTTCGGAACTGGGACCAAGGTCACCGTCCTAGGTTCTAGAG
GTGGTGGTGGTAGCGGCGGCGGCGGCTCTGGTGGTGGTGGATCCCTCGAGCAGGTG
CAGCTGGTGCAGTCTGGGGCTGAGGTGGAGAAGCCTGGGGCCTCAGTGAAGATTTC
CTGCAAGGCATCTACATACACCTTCACCAACTACTATATACACTGGGTACGACAGGC
CCCTGGACAAGGGCTTGAGTGGATGGGAATGATCAACCCTAGTGGTGATACCACAA
GCTACGCACAGAAGTTCCAGGGCAGAGTCACCATGACCAGGGACACGTCCACGAGC
ACTGTCTACATGGAGTTGAGCAGCCTGAGATCTGAGGACACGGCCGTGTATTACTGT
GCGCGCCTGTCTTGGTACTGGGGTGGTTCTTTCGATGACTGGGGTCAAGGTACTCTG
GTGACCGTCTCCTCA
   9VL (i.e., the VL of Scfv9 in CAR006) DNA sequence (SEQ ID NO: 86) 
CAGCCTGTGCTGACTCAGCCTCCCTCCGTGTCCGGGTCTCCTGGACAGTCAGTCA
CCATCTCCTGCACTGGAACCAGCGATGACATTGGAAATTATAAGCGTGTCTCCTGGT
ACCAGCAGCCCCCTGGCACAGCCCCCAAACTCATGATTTATGAGGTCACTAATCGGC
CCCCAGGGGTCCCTAATCGCTTCTCTGGGTCCAAGTCTGGCAACACGGCCTCCCTGA
CCATCTCTGGGCTCCAGGCTGAGGACGAGGCTGATTATTACTGCAGCTCATATGCAA
GCGGCGACACTTATGTCTTCGGAACTGGGACCAAGGTCACCGTCCTAGGT
   9 VH (i.e., the VH of Scfv9 in CAR006) DNA sequence (SEQ ID NO: 87) 
CAGGTGCAGCTGGTGCAGTCTGGGGCTGAGGTGGAGAAGCCTGGGGCCTCAGTG
AAGATTTCCTGCAAGGCATCTACATACACCTTCACCAACTACTATATACACTGGGTA
CGACAGGCCCCTGGACAAGGGCTTGAGTGGATGGGAATGATCAACCCTAGTGGTGA
TACCACAAGCTACGCACAGAAGTTCCAGGGCAGAGTCACCATGACCAGGGACACGT
CCACGAGCACTGTCTACATGGAGTTGAGCAGCCTGAGATCTGAGGACACGGCCGTGT
ATTACTGTGCGCGCCTGTCTTGGTACTGGGGTGGTTCTTTCGATGACTGGGGTCAAG
GTACTCTGGTGACCGTCTCCTCA
   ScFv6 amino acid sequence (SEQ ID NO: 88) 
NFMLTQPHSVSESPGKTVTISCTGNSGSIARNYVQWYQQRPGSAPTMLIYEDNRRPS
GVPDRFSGSIDSSSNSASLTISGLKTEDEADYYCQSYDDNTSWVFGGGTKLTVLGSRGGG
GSGGGGSGGGGSLEMAEVQLVQSGAEVKKPGSSVKVSCKASGGTFSSNAITWVRQAPG
QGLEWMGGIIPMFGTTDYAQKFQGRVTMTADESTSTAYMELRSLRSEDTAVYYCARDG
GGRHPYYYYGMDVWGQGTTVTVSS
   6 VL (i.e., the VL of Scfv6 in CAR004) amino acid sequence (SEQ ID NO: 89) 
NFMLTQPHSVSESPGKTVTISCTGNSGSIARNYVQWYQQRPGSAPTMLIYEDNRRPS
GVPDRFSGSIDSSSNSASLTISGLKTEDEADYYCQSYDDNTSWVFGGGTKLTVLG
   6 VH (i.e., the VH of Scfv6 in CAR004) amino acid sequence (SEQ ID NO: 90) 
MAEVQLVQSGAEVKKPGSSVKVSCKASGGTFSSNAITWVRQAPGQGLEWMGGIIPM
FGTTDYAQKFQGRVTMTADESTSTAYMELRSLRSEDTAVYYCARDGGRHPYYYYGM
DVWGQGTTVTVSS
   ScFv7 amino acid sequence (SEQ ID NO: 91) 
QSVVTQPPSVSAAPGQKATISCSGSSSNIGNNDVSWYQQLPGTAPKLLIQDNDKRPSG
IPDRFSGSKSGTSATLGITGLQTGDEADYYCAVWDSSLSAAVFGPGTKVTVLGSRGGGG
SGGGGSGGGGSLEMAEVQLVQSGAEVKKPGESLKISCKGSGYSFTSYWIGWVRQMPGK
GLEWMGIIYPDDSDTTYSPSFQGQVTMSVDKSISTAYLQWNSLQASDTAMYYCARMSL
TSYLATDGWGQGTLVTVSS
   7 VL (i.e., the VL of Scfv7 in CAR005) amino acid sequence (SEQ ID NO: 92) 
QSVVTQPPSVSAAPGQKATISCSGSSSNIGNNDVSWYQQLPGTAPKLLIQDNDKRPSG
IPDRFSGSKSGTSATLGITGLQTGDEADYYCAVWDSSLSAAVFGPGTKVTVLG
   7 VH (i.e., the VH of Scfv7 in CAR005) amino acid sequence (SEQ ID NO: 93) 
MAEVQLVQSGAEVKKPGESLKISCKGSGYSFTSYWIGWVRQMPGKGLEWMGIIYPD
DSDTTYSPSFQGQVTMSVDKSISTAYLQWNSLQASDTAMYYCARMSLTSYLATDGWG
QGTLVTVSS
   ScFv9 amino acid sequence (SEQ ID NO: 94) 
QPVLTQPPSVSGSPGQSVTISCTGTSDDIGNYKRVSWYQQPPGTAPKLMIYEVTNRPP
GVPNRFSGSKSGNTASLTISGLQAEDEADYYCSSYASGDTYVFGTGTKVTVLGSRGGGG
SGGGGSGGGGSLEQVQLVQSGAEVEKPGASVKISCKASTYTFTNYYIHWVRQAPGQGL
EWMGMINPSGDTTSYAQKFQGRVTMTRDTSTSTVYMELSSLRSEDTAVYYCARLSWY
WGGSFDDWGQGTLVTVSS
   9VL (i.e., the VL of Scfv9 in CAR006) amino acid sequence (SEQ ID NO: 95) 
QPVLTQPPSVSGSPGQSVTISCTGTSDDIGNYKRVSWYQQPPGTAPKLMIYEVTNRPP
GVPNRFSGSKSGNTASLTISGLQAEDEADYYCSSYASGDTYVFGTGTKVTVLG
   9VH (i.e., the VH of Scfv9 in CAR006) amino acid sequence (SEQ ID NO: 96) 
QVQLVQSGAEVEKPGASVKISCKASTYTFTNYYIHWVRQAPGQGLEWMGMINPSGD
TTSYAQKFQGRVTMTRDTSTSTVYMELSSLRSEDTAVYYCARLSWYWGGSFDDWGQG
TLVTVSS
   CAR004 nucleic acid sequence (SEQ ID NO: 97) 
ATGGCCCTGCCTGTGACAGCTCTGCTCCTCCCTCTGGCCCTGCTGCTCCATGCCGC
CAGACCCAATTTTATGCTGACTCAGCCCCACTCTGTGTCGGAGTCTCCGGGGAAGAC
GGTAACCATCTCCTGCACCGGCAATAGTGGCAGCATTGCCAGAAACTATGTGCAGTG
GTACCAACAGCGCCCGGGCAGTGCCCCCACCATGCTGATCTATGAGGATAATCGAA
GACCCTCTGGGGTCCCTGATCGGTTCTCTGGCTCCATCGACAGCTCCTCCAACTCTGC
CTCCCTCACCATCTCTGGACTGAAGACTGAGGACGAGGCTGACTATTACTGTCAGTC
TTATGATGACAACACTTCTTGGGTGTTCGGCGGAGGGACCAAGCTGACCGTCCTAGG
TTCTAGAGGTGGTGGTGGTAGCGGCGGCGGCGGCTCTGGTGGTGGTGGATCCCTCGA
GATGGCCGAGGTCCAGCTGGTACAGTCTGGGGCTGAGGTGAAGAAGCCTGGGTCCT
CGGTGAAGGTCTCCTGCAAGGCTTCTGGAGGGACCTTCAGCAGTAATGCTATCACCT
GGGTGCGACAGGCCCCTGGACAAGGCCTTGAGTGGATGGGAGGGATCATCCCTATG
TTTGGTACAACAGACTACGCACAGAAGTTCCAGGGCAGAGTCACGATGACCGCGGA
CGAATCCACGAGCACAGCCTACATGGAACTGCGCAGCCTGAGATCTGAAGACACGG
CCGTGTATTACTGTGCGAGAGATGGGGGGGGACGACACCCCTACTACTACTACGGTA
TGGACGTCTGGGGCCAAGGGACCACGGTCACCGTCTCCTCATTCGTGCCCGTGTTCC
TGCCCGCCAAACCTACTACTACCCCTGCACCTAGGCCTCCCACCCCAGCCCCAACAA
TCGCCAGCCAGCCTCTGTCTCTGCGGCCCGAAGCCTGTAGACCTGCTGCCGGCGGAG
CCGTGCACACCAGAGGCCTGGACTTCGCCTGCGACATCTACATCTGGGCCCCTCTGG
CCGGCACCTGTGGCGTGCTGCTGCTGAGCCTGGTGATCACCCTGTACTGCAACCACC
GGAACAAACGGGGCAGAAAGAAACTCCTGTATATATTCAAACAACCATTTATGAGA
CCAGTACAAACTACTCAAGAGGAAGATGGCTGTAGCTGCCGATTTCCAGAAGAAGA
AGAAGGAGGATGTGAACTGAGAGTGAAGTTCAGCAGATCCGCCGACGCCCCTGCCT
ACCAGCAGGGACAGAACCAGCTGTACAACGAGCTGAACCTGGGCAGACGGGAAGA
GTACGACGTGCTGGACAAGCGGAGAGGCCGGGACCCCGAGATGGGCGGAAAGCCC
AGACGGAAGAACCCCCAGGAAGGCCTGTATAACGAACTGCAGAAAGACAAGATGG
CCGAGGCCTACAGCGAGATCGGCATGAAGGGCGAGCGGAGGCGCGGCAAGGGCCA
CGATGGCCTGTACCAGGGCCTGAGCACCGCCACCAAGGACACCTACGACGCCCTGC
 
ACATGCAGGCCCTGCCCCCCAGAGGATCCGGAGAGGGAAGGGGCAGCTTATTAACA
TGTGGCGATGTGGAAGAGAACCCCGGTCCCATGCTGCTGCTCGTGACCTCTTTACTG
TTATGTGAGCTGCCCCACCCCGCTTTTTTACTGATCCCTCGTAAGGTGTGTAACGGAA
TCGGCATTGGCGAGTTCAAGGACTCTTTAAGCATCAACGCCACAAACATCAAGCACT
TCAAGAATTGTACCTCCATCAGCGGCGATTTACACATTCTCCCCGTGGCTTTTCGTGG
CGATTCCTTCACCCACACCCCCCCTCTGGACCCCCAAGAGCTGGACATTTTAAAAAC
CGTGAAGGAGATCACCGGCTTTCTGCTGATCCAAGCTTGGCCCGAGAATCGTACCGA
CCTCCACGCCTTCGAGAATTTAGAGATTATTCGTGGAAGGACCAAGCAGCACGGCCA
GTTCTCTTTAGCCGTCGTGTCTTTAAACATTACCAGCCTCGGTTTAAGGTCTTTAAAG
GAGATTAGCGACGGCGACGTGATCATCTCCGGCAACAAGAACCTCTGCTACGCCAA
CACCATCAACTGGAAGAAGCTGTTCGGAACCAGCGGCCAAAAGACCAAGATCATCA
GCAATCGTGGAGAGAACTCTTGTAAGGCCACTGGTCAAGTTTGCCACGCCCTCTGTA
GCCCCGAAGGATGTTGGGGCCCCGAGCCTAGGGACTGTGTTAGCTGCAGAAACGTG
AGCAGAGGCAGAGAGTGTGTGGACAAATGCAATTTACTGGAAGGAGAGCCTAGGGA
GTTCGTGGAGAACAGCGAATGTATCCAGTGCCACCCCGAGTGTTTACCTCAAGCCAT
GAACATCACTTGTACCGGAAGGGGCCCCGATAACTGCATCCAATGCGCCCACTACAT
CGACGGACCCCACTGCGTGAAAACTTGTCCCGCCGGAGTGATGGGAGAGAATAACA
CTTTAGTGTGGAAGTACGCCGACGCTGGCCACGTCTGCCATCTGTGCCACCCCAACT
GTACCTACGGCTGCACTGGTCCCGGTTTAGAGGGATGTCCTACCAACGGCCCCAAGA
TCCCCTCCATCGCCACCGGAATGGTGGGCGCTCTGTTATTACTGCTGGTGGTGGCTCT
GGGCATCGGTTTATTCATG
   CAR004 amino acid sequence (SEQ ID NO: 98) 
MALPVTALLLPLALLLHAARPNFMLTQPHSVSESPGKTVTISCTGNSGSIARNYVQW
YQQRPGSAPTMLIYEDNRRPSGVPDRFSGSIDSSSNSASLTISGLKTEDEADYYCQSYDD
NTSWVFGGGTKLTVLGSRGGGGSGGGGSGGGGSLEMAEVQLVQSGAEVKKPGSSVKV
SCKASGGTFSSNAITWVRQAPGQGLEWMGGIIPMFGTTDYAQKFQGRVTMTADESTST
AYMELRSLRSEDTAVYYCARDGGGRHPYYYYGMDVWGQGTTVTVSSFVPVFLPAKPT
 
TTPAPRPPTPAPTIASQPLSLRPEACRPAAGGAVHTRGLDFACDIYIWAPLAGTCGVLLLS
LVITLYCNHRNKRGRKKLLYIFKQPFMRPVQTTQEEDGCSCRFPEEEEGGCELRVKFSRS
ADAPAYQQGQNQLYNELNLGRREEYDVLDKRRGRDPEMGGKPRRKNPQEGLYNELQK
DKMAEAYSEIGMKGERRRGKGHDGLYQGLSTATKDTYDALHMQALPPRGSGEGRGSL
LTCGDVEENPGPMLLLVTSLLLCELPHPAFLLIPRKVCNGIGIGEFKDSLSINATNIKHFKN
CTSISGDLHILPVAFRGDSFTHTPPLDPQELDILKTVKEITGFLLIQAWPENRTDLHAFENL
EIIRGRTKQHGQFSLAVVSLNITSLGLRSLKEISDGDVIISGNKNLCYANTINWKKLFGTSG
QKTKIISNRGENSCKATGQVCHALCSPEGCWGPEPRDCVSCRNVSRGRECVDKCNLLEG
EPREFVENSECIQCHPECLPQAMNITCTGRGPDNCIQCAHYIDGPHCVKTCPAGVMGEN
NTLVWKYADAGHVCHLCHPNCTYGCTGPGLEGCPTNGPKIPSIATGMVGALLLLLVVA
LGIGLFM
   CAR005 nucleic acid sequence (SEQ ID NO: 99) 
ATGGCCCTGCCTGTGACAGCTCTGCTCCTCCCTCTGGCCCTGCTGCTCCATGCCGC
CAGACCCCAGTCTGTCGTGACGCAGCCGCCCTCAGTGTCTGCGGCCCCAGGACAGAA
GGCCACCATCTCCTGCTCTGGAAGCAGCTCCAACATTGGGAATAATGATGTATCCTG
GTACCAGCAGCTTCCAGGAACAGCCCCCAAACTCCTCATTCAGGACAATGATAAGC
GACCCTCAGGTATTCCTGACCGATTCTCTGGCTCCAAGTCTGGCACGTCAGCCACCC
TGGGCATCACCGGACTCCAGACTGGGGACGAGGCCGATTATTACTGCGCAGTATGG
GATAGCAGCCTGAGTGCTGCCGTCTTCGGACCTGGGACCAAGGTCACCGTCCTAGGT
TCTAGAGGTGGTGGTGGTAGCGGCGGCGGCGGCTCTGGTGGTGGATCCCTCGAG
ATGGCCGAAGTGCAGCTGGTGCAGTCTGGAGCAGAGGTGAAAAAGCCCGGGGAGTC
TCTGAAGATCTCCTGTAAGGGTTCTGGATACAGCTTTACCAGCTACTGGATCGGCTG
GGTGCGCCAGATGCCCGGGAAAGGCCTGGAGTGGATGGGAATCATCTATCCTGATG
ACTCTGATACCACGTACAGCCCGTCCTTCCAAGGCCAGGTCACCATGTCAGTCGACA
AGTCCATCAGCACCGCCTACCTGCAGTGGAACAGCCTGCAGGCCTCGGACACCGCC
ATGTATTACTGTGCGCGCATGTCTCTGACCTCTTACCTGGCAACCGATGGTTGGGGTC
AAGGTACTCTGGTGACCGTCTCCTCATTCGTGCCCGTGTTCCTGCCCGCCAAACCTAC
 
TACTACCCCTGCACCTAGGCCTCCCACCCCAGCCCCAACAATCGCCAGCCAGCCTCT
GTCTCTGCGGCCCGAAGCCTGTAGACCTGCTGCCGGCGGAGCCGTGCACACCAGAG
GCCTGGACTTCGCCTGCGACATCTACATCTGGGCCCCTCTGGCCGGCACCTGTGGCG
TGCTGCTGCTGAGCCTGGTGATCACCCTGTACTGCAACCACCGGAACAAACGGGGCA
GAAAGAAACTCCTGTATATATTCAAACAACCATTTATGAGACCAGTACAAACTACTC
AAGAGGAAGATGGCTGTAGCTGCCGATTTCCAGAAGAAGAAGAAGGAGGATGTGAA
CTGAGAGTGAAGTTCAGCAGATCCGCCGACGCCCCTGCCTACCAGCAGGGACAGAA
CCAGCTGTACAACGAGCTGAACCTGGGCAGACGGGAAGAGTACGACGTGCTGGACA
AGCGGAGAGGCCGGGACCCCGAGATGGGCGGAAAGCCCAGACGGAAGAACCCCCA
GGAAGGCCTGTATAACGAACTGCAGAAAGACAAGATGGCCGAGGCCTACAGCGAGA
TCGGCATGAAGGGCGAGCGGAGGCGCGGCAAGGGCCACGATGGCCTGTACCAGGGC
CTGAGCACCGCCACCAAGGACACCTACGACGCCCTGCACATGCAGGCCCTGCCCCCC
AGAGGATCCGGAGAGGGAAGGGGCAGCTTATTAACATGTGGCGATGTGGAAGAGAA
CCCCGGTCCCATGCTGCTGCTCGTGACCTCTTTACTGTTATGTGAGCTGCCCCACCCC
GCTTTTTTACTGATCCCTCGTAAGGTGTGTAACGGAATCGGCATTGGCGAGTTCAAG
GACTCTTTAAGCATCAACGCCACAAACATCAAGCACTTCAAGAATTGTACCTCCATC
AGCGGCGATTTACACATTCTCCCCGTGGCTTTTCGTGGCGATTCCTTCACCCACACCC
CCCCTCTGGACCCCCAAGAGCTGGACATTTTAAAAACCGTGAAGGAGATCACCGGCT
TTCTGCTGATCCAAGCTTGGCCCGAGAATCGTACCGACCTCCACGCCTTCGAGAATT
TAGAGATTATTCGTGGAAGGACCAAGCAGCACGGCCAGTTCTCTTTAGCCGTCGTGT
CTTTAAACATTACCAGCCTCGGTTTAAGGTCTTTAAAGGAGATTAGCGACGGCGACG
TGATCATCTCCGGCAACAAGAACCTCTGCTACGCCAACACCATCAACTGGAAGAAG
CTGTTCGGAACCAGCGGCCAAAAGACCAAGATCATCAGCAATCGTGGAGAGAACTC
TTGTAAGGCCACTGGTCAAGTTTGCCACGCCCTCTGTAGCCCCGAAGGATGTTGGGG
CCCCGAGCCTAGGGACTGTGTTAGCTGCAGAAACGTGAGCAGAGGCAGAGAGTGTG
TGGACAAATGCAATTTACTGGAAGGAGAGCCTAGGGAGTTCGTGGAGAACAGCGAA
TGTATCCAGTGCCACCCCGAGTGTTTACCTCAAGCCATGAACATCACTTGTACCGGA
 
AGGGGCCCCGATAACTGCATCCAATGCGCCCACTACATCGACGGACCCCACTGCGTG

AAAACTTGTCCCGCCGGAGTGATGGGAGAGAATAACACTTTAGTGTGGAAGTACGC

CGACGCTGGCCACGTCTGCCATCTGTGCCACCCCAACTGTACCTACGGCTGCACTGG

TCCCGGTTTAGAGGGATGTCCTACCAACGGCCCCAAGATCCCCTCCATCGCCACCGG

AATGGTGGGCGCTCTGTTATTACTGCTGGTGGTGGCTCTGGGCATCGGTTTATTCATG
   CAR005 amino acid sequence (SEQ ID NO: 100) 
MALPVTALLLPLALLLHAARPQSVVTQPPSVSAAPGQKATISCSGSSSNIGNNDVSW
YQQLPGTAPKLLIQDNDKRPSGIPDRFSGSKSGTSATLGITGLQTGDEADYYCAVWDSSL
SAAVFGPGTKVTVLGSRGGGGSGGGGSGGGGSLEMAEVQLVQSGAEVKKPGESLKISC
KGSGYSFTSYWIGWVRQMPGKGLEWMGIIYPDDSDTTYSPSFQGQVTMSVDKSISTAYL
QWNSLQASDTAMYYCARMSLTSYLATDGWGQGTLVTVSSFVPVFLPAKPTTTPAPRPP
TPAPTIASQPLSLRPEACRPAAGGAVHTRGLDFACDIYIWAPLAGTCGVLLLSLVITLYCN
HRNKRGRKKLLYIFKQPFMRPVQTTQEEDGCSCRFPEEEEGGCELRVKFSRSADAPAYQ
QGQNQLYNELNLGRREEYDVLDKRRGRDPEMGGKPRRKNPQEGLYNELQKDKMAEA
YSEIGMKGERRRGKGHDGLYQGLSTATKDTYDALHMQALPPRGSGEGRGSLLTCGDVE
ENPGPMLLLVTSLLLCELPHPAFLLIPRKVCNGIGIGEFKDSLSINATNIKHFKNCTSISGDL
HILPVAFRGDSFTHTPPLDPQELDILKTVKEITGFLLIQAWPENRTDLHAFENLEIIRGRTK
QHGQFSLAVVSLNITSLGLRSLKEISDGDVIISGNKNLCYANTINWKKLFGTSGQKTKIIS
NRGENSCKATGQVCHALCSPEGCWGPEPRDCVSCRNVSRGRECVDKCNLLEGEPREFV
ENSECIQCHPECLPQAMNITCTGRGPDNCIQCAHYIDGPHCVKTCPAGVMGENNTLVWK
YADAGHVCHLCHPNCTYGCTGPGLEGCPTNGPKIPSIATGMVGALLLLLVVALGIGLFM
   CAR006 nucleic acid sequence (SEQ ID NO: 101) 
ATGGCCCTGCCTGTGACAGCTCTGCTCCTCCCTCTGGCCCTGCTGCTCCATGCCGC
CAGACCCCAGCCTGTGCTGACTCAGCCTCCCTCCGTGTCCGGGTCTCCTGGACAGTC
AGTCACCATCTCCTGCACTGGAACCAGCGATGACATTGGAAATTATAAGCGTGTCTC
CTGGTACCAGCAGCCCCCTGGCACAGCCCCCAAACTCATGATTTATGAGGTCACTAA
TCGGCCCCCAGGGGTCCCTAATCGCTTCTCTGGGTCCAAGTCTGGCAACACGGCCTC
 
CCTGACCATCTCTGGGCTCCAGGCTGAGGACGAGGCTGATTATTACTGCAGCTCATA
TGCAAGCGGCGACACTTATGTCTTCGGAACTGGGACCAAGGTCACCGTCCTAGGTTC
TAGAGGTGGTGGTGGTAGCGGCGGCGGCTCTGGTGGTGGATCCCTCGAGC
AGGTGCAGCTGGTGCAGTCTGGGGCTGAGGTGGAGAAGCCTGGGGCCTCAGTGAAG
ATTTCCTGCAAGGCATCTACATACACCTTCACCAACTACTATATACACTGGGTACGA
CAGGCCCCTGGACAAGGGCTTGAGTGGATGGGAATGATCAACCCTAGTGGTGATAC
CACAAGCTACGCACAGAAGTTCCAGGGCAGAGTCACCATGACCAGGGACACGTCCA
CGAGCACTGTCTACATGGAGTTGAGCAGCCTGAGATCTGAGGACACGGCCGTGTATT
ACTGTGCGCGCCTGTCTTGGTACTGGGGTGGTTCTTTCGATGACTGGGGTCAAGGTA
CTCTGGTGACCGTCTCCTCATTCGTGCCCGTGTTCCTGCCCGCCAAACCTACTACTAC
CCCTGCACCTAGGCCTCCCACCCCAGCCCCAACAATCGCCAGCCAGCCTCTGTCTCT
GCGGCCCGAAGCCTGTAGACCTGCTGCCGGCGGAGCCGTGCACACCAGAGGCCTGG
ACTTCGCCTGCGACATCTACATCTGGGCCCCTCTGGCCGGCACCTGTGGCGTGCTGC
TGCTGAGCCTGGTGATCACCCTGTACTGCAACCACCGGAACAAACGGGGCAGAAAG
AAACTCCTGTATATATTCAAACAACCATTTATGAGACCAGTACAAACTACTCAAGAG
GAAGATGGCTGTAGCTGCCGATTTCCAGAAGAAGAAGAAGGAGGATGTGAACTGAG
AGTGAAGTTCAGCAGATCCGCCGACGCCCCTGCCTACCAGCAGGGACAGAACCAGC
TGTACAACGAGCTGAACCTGGGCAGACGGGAAGAGTACGACGTGCTGGACAAGCGG
AGAGGCCGGGACCCCGAGATGGGCGGAAAGCCCAGACGGAAGAACCCCCAGGAAG
GCCTGTATAACGAACTGCAGAAAGACAAGATGGCCGAGGCCTACAGCGAGATCGGC
ATGAAGGGCGAGCGGAGGCGCGGCAAGGGCCACGATGGCCTGTACCAGGGCCTGAG
CACCGCCACCAAGGACACCTACGACGCCCTGCACATGCAGGCCCTGCCCCCCAGAG
GATCCGGAGAGGGAAGGGGCAGCTTATTAACATGTGGCGATGTGGAAGAGAACCCC
GGTCCCATGCTGCTGCTCGTGACCTCTTTACTGTTATGTGAGCTGCCCCACCCCGCTT
TTTTACTGATCCCTCGTAAGGTGTGTAACGGAATCGGCATTGGCGAGTTCAAGGACT
CTTTAAGCATCAACGCCACAAACATCAAGCACTTCAAGAATTGTACCTCCATCAGCG
GCGATTTACACATTCTCCCCGTGGCTTTTCGTGGCGATTCCTTCACCCACACCCCCCC
 
TCTGGACCCCCAAGAGCTGGACATTTTAAAAACCGTGAAGGAGATCACCGGCTTTCT
GCTGATCCAAGCTTGGCCCGAGAATCGTACCGACCTCCACGCCTTCGAGAATTTAGA
GATTATTCGTGGAAGGACCAAGCAGCACGGCCAGTTCTCTTTAGCCGTCGTGTCTTT
AAACATTACCAGCCTCGGTTTAAGGTCTTTAAAGGAGATTAGCGACGGCGACGTGAT
CATCTCCGGCAACAAGAACCTCTGCTACGCCAACACCATCAACTGGAAGAAGCTGTT
CGGAACCAGCGGCCAAAAGACCAAGATCATCAGCAATCGTGGAGAGAACTCTTGTA
AGGCCACTGGTCAAGTTTGCCACGCCCTCTGTAGCCCCGAAGGATGTTGGGGCCCCG
AGCCTAGGGACTGTGTTAGCTGCAGAAACGTGAGCAGAGGCAGAGAGTGTGTGGAC
AAATGCAATTTACTGGAAGGAGAGCCTAGGGAGTTCGTGGAGAACAGCGAATGTAT
CCAGTGCCACCCCGAGTGTTTACCTCAAGCCATGAACATCACTTGTACCGGAAGGGG
CCCCGATAACTGCATCCAATGCGCCCACTACATCGACGGACCCCACTGCGTGAAAAC
TTGTCCCGCCGGAGTGATGGGAGAGAATAACACTTTAGTGTGGAAGTACGCCGACG
CTGGCCACGTCTGCCATCTGTGCCACCCCAACTGTACCTACGGCTGCACTGGTCCCG
GTTTAGAGGGATGTCCTACCAACGGCCCCAAGATCCCCTCCATCGCCACCGGAATGG
TGGGCGCTCTGTTATTACTGCTGGTGGTGGCTCTGGGCATCGGTTTATTCATG
   CAR006 amino acid sequence (SEQ ID NO: 102)

MALPVTALLLPLALLLHAARPQPVLTQPPSVSGSPGQSVTISCTGTSDDIGNYKRVSW
YQQPPGTAPKLMIYEVTNRPPGVPNRFSGSKSGNTASLTISGLQAEDEADYYCSSYASGD
TYVFGTGTKVTVLGSRGGGGSGGGGSGGGGSLEQVQLVQSGAEVEKPGASVKISCKAS
TYTFTNYYIHWVRQAPGQGLEWMGMINPSGDTTSYAQKFQGRVTMTRDTSTSTVYME
LSSLRSEDTAVYYCARLSWYWGGSFDDWGQGTLVTVSSFVPVFLPAKPTTTPAPRPPTP
APTIASQPLSLRPEACRPAAGGAVHTRGLDFACDIYIWAPLAGTCGVLLLSLVITLYCNH
RNKRGRKKLLYIFKQPFMRPVQTTQEEDGCSCRFPEEEEGGCELRVKFSRSADAPAYQQ
GQNQLYNELNLGRREEYDVLDKRRGRDPEMGGKPRRKNPQEGLYNELQKDKMAEAYS
EIGMKGERRRGKGHDGLYQGLSTATKDTYDALHMQALPPRGSGEGRGSLLTCGDVEEN
PGPMLLLVTSLLLCELPHPAFLLIPRKVCNGIGIGEFKDSLSINATNIKHFKNCTSISGDLHI
LPVAFRGDSFTHTPPLDPQELDILKTVKEITGFLLIQAWPENRTDLHAFENLEIIRGRTKQH

GQFSLAVVSLNITSLGLRSLKEISDGDVIISGNKNLCYANTINWKKLFGTSGQKTKIISNRG
ENSCKATGQVCHALCSPEGCWGPEPRDCVSCRNVSRGRECVDKCNLLEGEPREFVENSE
CIQCHPECLPQAMNITCTGRGPDNCIQCAHYIDGPHCVKTCPAGVMGENNTLVWKYAD
AGHVCHLCHPNCTYGCTGPGLEGCPTNGPKIPSIATGMVGALLLLLVVALGIGLFM

## Claims

1. A chimeric antigen receptor (CAR) comprising a CD5 binding domain, a transmembrane domain, a co-stimulatory domain and an intracellular signaling domain, wherein the CD5 binding domain comprises an antibody specifically binding to CD5 or a fragment thereof; the antibody comprises a heavy chain complementarity determining region 1 (HCDR1), a heavy chain complementarity determining region 2 (HCDR2) and a heavy chain complementarity determining region 3 (HCDR3); and the amino acid sequences of the HCDR1, HCDR2, and HCDR3 are selected from the following combinations:
(1) the HCDR1 having an amino acid sequence as set forth in SEQ ID NO: 43, the HCDR2 having an amino acid sequence as set forth in SEQ ID NO: 44, and the HCDR3 having an amino acid sequence as set forth in SEQ ID NO: 45;
(2) the HCDR1 having an amino acid sequence as set forth in SEQ ID NO: 49, the HCDR2 having an amino acid sequence as set forth in SEQ ID NO: 50, and the HCDR3 having an amino acid sequence as set forth in SEQ ID NO: 51;
(3) the HCDR1 having an amino acid sequence as set forth in SEQ ID NO: 55, the HCDR2 having an amino acid sequence as set forth in SEQ ID NO: 56, and the HCDR3 having an amino acid sequence as set forth in SEQ ID NO: 57;
(4) the HCDR1 having an amino acid sequence as set forth in SEQ ID NO: 61, the HCDR2 having an amino acid sequence as set forth in SEQ ID NO: 62, and the HCDR3 having an amino acid sequence as set forth in SEQ ID NO: 63;
(5) the HCDR1 having an amino acid sequence as set forth in SEQ ID NO: 67, the HCDR2 having an amino acid sequence as set forth in SEQ ID NO: 68, and the HCDR3 having an amino acid sequence as set forth in SEQ ID NO: 69; or
(6) the HCDR1 having an amino acid sequence as set forth in SEQ ID NO: 73, the HCDR2 having an amino acid sequence as set forth in SEQ ID NO: 74, and the HCDR3 having an amino acid sequence as set forth in SEQ ID NO: 75.

2. The CAR of claim 1, wherein the antibody further comprises a light chain complementarity determining region 1 (LCDR1), a light chain complementarity determining region 2 (LCDR2) and a light chain complementarity determining region 3 (LCDR3), and the amino acid sequences of the LCDR1, LCDR2, LCDR3 are selected from the following combinations:
(1) the LCDR1 having an amino acid sequence as set forth in SEQ ID NO: 46, the LCDR2 having an amino acid sequence as set forth in SEQ ID NO: 47, and the LCDR3 having an amino acid sequence as set forth in SEQ ID NO: 48;
(2) the LCDR1 having an amino acid sequence as set forth in SEQ ID NO: 52, the LCDR2 having an amino acid sequence as set forth in SEQ ID NO: 53, and the LCDR3 having an amino acid sequence as set forth in SEQ ID NO: 54;
(3) the LCDR1 having an amino acid sequence as set forth in SEQ ID NO: 58, the LCDR2 having an amino acid sequence as set forth in SEQ ID NO: 59, and the LCDR3 having an amino acid sequence as set forth in SEQ ID NO: 60;
(4) the LCDR1 having an amino acid sequence as set forth in SEQ ID NO: 64, the LCDR2 having an amino acid sequence as set forth in SEQ ID NO: 65, and the LCDR3 having an amino acid sequence as set forth in SEQ ID NO: 66;
(5) the LCDR1 having an amino acid sequence as set forth in SEQ ID NO: 70, the LCDR2 having an amino acid sequence as set forth in SEQ ID NO: 71, and the LCDR3 having an amino acid sequence as set forth in SEQ ID NO: 72; or
(6) the LCDR1 having an amino acid sequence as set forth in SEQ ID NO: 76, the LCDR2 having an amino acid sequence as set forth in SEQ ID NO: 77, and the LCDR3 having an amino acid sequence as set forth in SEQ ID NO: 78.

3. The CAR of any one of claims 1-2, wherein the HCDR1, HCDR2, HCDR3 and LCDR1, LCDR2, LCDR3 comprised in the antibody are selected from any one of the following combinations:
(1) the HCDR1 having an amino acid sequence as set forth in SEQ ID NO: 43, the HCDR2 having an amino acid sequence as set forth in SEQ ID NO: 44, and the HCDR3 having an amino acid sequence as set forth in SEQ ID NO: 45; the LCDR1 having an amino acid sequence as set forth in SEQ ID NO: 46, the LCDR2 having an amino acid sequence as set forth in SEQ ID NO: 47, and the LCDR3 having an amino acid sequence as set forth in SEQ ID NO: 48;
(2) the HCDR1 having an amino acid sequence as set forth in SEQ ID NO: 49, the HCDR2 having an amino acid sequence as set forth in SEQ ID NO: 50, and the HCDR3 having an amino acid sequence as set forth in SEQ ID NO: 51; the LCDR1 having an amino acid sequence as set forth in SEQ ID NO: 52, the LCDR2 having an amino acid sequence as set forth in SEQ ID NO: 53, and the LCDR3 having an amino acid sequence as set forth in SEQ ID NO: 54;
(3) the HCDR1 having an amino acid sequence as set forth in SEQ ID NO: 55, the HCDR2 having an amino acid sequence as set forth in SEQ ID NO: 56, and the HCDR3 having an amino acid sequence as set forth in SEQ ID NO: 57; the LCDR1 having an amino acid sequence as set forth in SEQ ID NO: 58, the LCDR2 having an amino acid sequence as set forth in SEQ ID NO: 59, and the LCDR3 having an amino acid sequence as set forth in SEQ ID NO: 60;
(4) the HCDR1 having an amino acid sequence as set forth in SEQ ID NO: 61, the HCDR2 having an amino acid sequence as set forth in SEQ ID NO: 62, and the HCDR3 having an amino acid sequence as set forth in SEQ ID NO: 63; the LCDR1 having an amino acid sequence as set forth in SEQ ID NO: 64, the LCDR2 having an amino acid sequence as set forth in SEQ ID NO: 65, and the LCDR3 having an amino acid sequence as set forth in SEQ ID NO: 66;
(5) the HCDR1 having an amino acid sequence as set forth in SEQ ID NO: 67, the HCDR2 having an amino acid sequence as set forth in SEQ ID NO: 68, and the HCDR3 having an amino acid sequence as set forth in SEQ ID NO: 69; the LCDR1 having an amino acid sequence as set forth in SEQ ID NO: 70, the LCDR2 having an amino acid sequence as set forth in SEQ ID NO: 71, and the LCDR3 having an amino acid sequence as set forth in SEQ ID NO: 72; or
(6) the HCDR1 having an amino acid sequence as set forth in SEQ ID NO: 73, the HCDR2 having an amino acid sequence as set forth in SEQ ID NO: 74, and the HCDR3 having an amino acid sequence as set forth in SEQ ID NO: 75; the LCDR1 having an amino acid sequence as set forth in SEQ ID NO: 76, the LCDR2 having an amino acid sequence as set forth in SEQ ID NO: 77, and the LCDR3 having an amino acid sequence as set forth in SEQ ID NO: 78.

4. The CAR of any one of claims 1-3, wherein the antibody comprises a heavy chain variable region, and the amino acid sequence of the heavy chain variable region is set forth in SEQ ID NO: 30, 36, 42, 90, 93 or 96.

5. The CAR of any one of claims 1-4, wherein the antibody comprises a light chain variable region, and the amino acid sequence of the light chain variable region is set forth in SEQ ID NO: 29, 35, 41, 89, 92 or 95.

6. The CAR of any one of claims 1-5, wherein the antibody is a single chain antibody.

7. The CAR of any one of claims 1-6, wherein the antibody comprises an amino acid sequence as set forth in SEQ ID NO: 28, 34, 40, 88, 91 or 94 or a functional variant thereof.

8. The CAR of any one of claims 1-7, wherein the transmembrane domain comprises a polypeptide selected from the following proteins: α, β or ζ chains of T-cell receptors, CD28, CD3e, CD45, CD4, CD5, CD8a, CD9, CD16, CD22, CD33, CD37, CD64, CD80, CD86, CD134, CD137, and CD154.

9. The CAR of any one of claims 1-8, wherein the transmembrane domain comprises an amino acid sequence as set forth in SEQ ID NO: 6 or a functional variant thereof.

10. The CAR of any one of claims 1-9, wherein the co-stimulatory domain comprises a polypeptide selected from the following proteins: CD28, 4-1BB, OX-40 and ICOS.

11. The CAR of any one of claims 1-10, wherein the co-stimulatory domain comprises an amino acid sequence as set forth in SEQ ID NO: 8 or a functional variant thereof.

12. The CAR of any one of claims 1-11, wherein the intracellular signaling domain comprises a signaling domain from CD3ζ.

13. The CAR of any one of claims 1-12, wherein the intracellular signaling domain comprises an amino acid sequence as set forth in SEQ ID NO: 10 or a functional variant thereof.

14. The CAR of any one of claims 1-13, wherein the CAR further comprises a hinge region connecting the antibody and the transmembrane domain.

15. The CAR of claims 14, wherein the hinge region comprises an amino acid sequence as set forth in SEQ ID NO: 4 or a functional variant thereof.

16. The CAR of any one of claims 1-15, wherein the CAR is further linked to a CD8α signal peptide.

17. The CAR of claims 16, wherein the signal peptide comprises an amino acid sequence as set forth in SEQ ID NO: 2 or a functional variant thereof.

18. The CAR of any one of claims 1-17, wherein the CAR is further linked to a cleaving peptide.

19. The CAR of claim 18, wherein the cleaving peptide comprises an amino acid sequence from a T2A peptide.

20. The CAR of any one of claims 18-19, wherein the cleaving peptide comprises an amino acid sequence as set forth in SEQ ID NO: 12 or a functional variant thereof.

21. The CAR of any one of claims 1-20, wherein the CAR is further linked to a truncated EGFR molecule (tEGFR) by linking to the cleaving peptide and a CSF2RA signal peptide.

22. The CAR of claim 21, wherein the CSF2RA signal peptide comprises an amino acid sequence as set forth in SEQ ID NO: 14 or a functional variant thereof, and the truncated EGFR molecule comprises an amino acid sequence as set forth in SEQ ID NO: 16 or a functional variant thereof.

23. The CAR of any one of claims 1-22, comprising an amino acid sequence selected from the group consisting of SEQ ID NO: 22-24, 98, 100 or 102 or a functional variant thereof.

24. An isolated nucleic acid molecule encoding the CAR of any one of claims 1-23.

25. The isolated nucleic acid molecule encoding CAR of claim 24, comprising a nucleic acid sequence selected from the group consisting of SEQ ID NO: 1, 3, 5, 7, 9, 11, 13, 15, 17, 19-21, 25-27, 31-33, 37-39, 79-87, 97, 99 or 101 or a functional variant thereof.

26. A vector comprising the nucleic acid molecule of any one of claims 24-25.

27. The vector of claim 26, wherein the vector is selected from the group consisting of a plasmid, a retroviral vector and a lentiviral vector.

28. An immune effector cell comprising the CAR of any one of claims 1-23, the nucleic acid molecule of any one of claims 24-25, or the vector of any one of claims 26-27.

29. The cell of claim 28, wherein CD5 on the immune effector cell is not expressed.

30. The cell of any one of claims 28-29, wherein the immune effector cell is selected from a T lymphocyte and a natural killer (NK) cell.

31. A method for preparing an immune effector cell, comprising knocking out the CD5 gene of the immune effector cell, and introducing the vector of claim 26 or 27 into the immune effector cell; preferably, the knocking out is carried out using an sgRNA as set forth in any one of SEQ ID NOs: 103-106.

32. A pharmaceutical composition comprising the immune effector cell of any one of claims 28-30 and a pharmaceutically acceptable adjuvant.

33. Use of the CAR of any one of claims 1-23, the nucleic acid molecule of any one of claims 24-25, the vector of any one of claims 26-27, or the immune effector cell of any one of claims 28-30 in the preparation of a drug, wherein the drug is used for treating diseases or conditions associated with the expression of CD5.

34. A method for treating a disease or condition associated with the expression of CD5, comprising administering a therapeutically effective amount of the immune effector cell of any one of claims 28-30 or the pharmaceutical composition of claim 32 to a patient in need thereof.

35. The method of claim 34, further comprising further administering an anti-EGFR antibody to the patient in need thereof to suppress the effect of the immune effector cell or the pharmaceutical composition.

36. The use of claim 33 or the method of any one of claims 34-35, wherein the disease or condition associated with the expression of CD5 is a cancer or malignancy.

37. A CD5-targeting fully human antibody or a single chain antibody or fragment thereof, wherein the fully human antibody comprises a heavy chain variable region (HCVR) and/or a light chain variable region (LCVR), and the heavy chain variable region comprises a HCDR1, a HCDR2 and a HCDR3, the light chain variable region comprises a LCDR1, a LCDR2 and a LCDR3, and the HCDR1, HCDR2, HCDR3 and/or the LCDR1, LCDR2 and LCDR3 are selected from one of the following combinations:
(1) the LCDR1 having an amino acid sequence of SGSIARNY (SEQ ID NO: 64);
the LCDR2 having an amino acid sequence of EDN (SEQ ID NO: 65);
the LCDR3 having an amino acid sequence of QSYDDNTSWV (SEQ ID NO: 66);
the HCDR1 having an amino acid sequence of GGTFSSNA (SEQ ID NO: 61);
the HCDR2 having an amino acid sequence of IIPMFGTT (SEQ ID NO: 62);
the HCDR3 having an amino acid sequence of ARDGGGRHPYYYYGMDV (SEQ ID NO: 63);
(2) the LCDR1 having an amino acid sequence of SSNIGNND (SEQ ID NO: 71);
the LCDR2 having an amino acid sequence of DND (SEQ ID NO: 72);
the LCDR3 having an amino acid sequence of AVWDSSLSAAV (SEQ ID NO: 73);
the HCDR1 having an amino acid sequence of GYSFTSYW (SEQ ID NO: 67);
the HCDR2 having an amino acid sequence of IYPDDSDT (SEQ ID NO: 68);
the HCDR3 having an amino acid sequence of ARMSLTSYLATDG (SEQ ID NO: 69);
(3) the LCDR1 having an amino acid sequence of SDDIGNYKR (SEQ ID NO: 76);
the LCDR2 having an amino acid sequence of EVT (SEQ ID NO: 77);
the LCDR3 having an amino acid sequence of SSYASGDTYV (SEQ ID NO: 78);
the HCDR1 having an amino acid sequence of TYTFTNYY (SEQ ID NO: 73);
the HCDR2 having an amino acid sequence of INPSGDTT (SEQ ID NO: 74);
the HCDR3 having an amino acid sequence of ARLSWYWGGSFDD (SEQ ID NO: 75);
(4) the LCDR1 having an amino acid sequence of SSNIGAGYD (SEQ ID NO: 46);
the LCDR2 having an amino acid sequence of GNI (SEQ ID NO: 47);
the LCDR3 having an amino acid sequence of GTWDNSLSAHYV (SEQ ID NO: 48);
the HCDR1 having an amino acid sequence of GFTFNNYT (SEQ ID NO: 43);
the HCDR2 having an amino acid sequence of ISSSSSYI (SEQ ID NO: 44);
the HCDR3 having an amino acid sequence of ARYFSGSAGDY (SEQ ID NO: 45);
(5) the LCDR1 having an amino acid sequence of NSNIGNNY (SEQ ID NO: 52);
the LCDR2 having an amino acid sequence of DNN (SEQ ID NO: 53);
the LCDR3 having an amino acid sequence of GIWDSSLDAYV (SEQ ID NO: 54);
the HCDR1 having an amino acid sequence of GFTFSSYS (SEQ ID NO: 49);
the HCDR2 having an amino acid sequence of ISSSSSYI (SEQ ID NO: 50);
the HCDR3 having an amino acid sequence of ARGNPWYGVDY (SEQ ID NO: 51);
(6) the LCDR1 having an amino acid sequence of SSNIGNNY (SEQ ID NO: 58);
the LCDR2 having an amino acid sequence of DNN (SEQ ID NO: 59);
the LCDR3 having an amino acid sequence of GTWDSSLSAVV (SEQ ID NO: 60);
the HCDR1 having an amino acid sequence of GFTFSSYA (SEQ ID NO: 55);
the HCDR2 having an amino acid sequence of ITDSGDIT (SEQ ID NO: 56);
the HCDR3 having an amino acid sequence of ARMSSHWYFSADY (SEQ ID NO: 57); or
the fully human antibody comprises a variant of the CDR sequence combination in any one of (1)-(6), wherein compared with the CDR sequence in any one of (1)-(6), the variant has at least 90% sequence identity, or comprises at least 1 and no more than 10 amino acid changes, or no more than 5, 4, 3, 2 or 1 amino acid changes in total in the CDR sequences.

38. The fully human antibody or single-chain antibody or fragment thereof of claim 37, wherein the amino acid sequence of the heavy chain variable region and/or light chain variable region is selected from any one of the following combinations:
(1) a sequence as set forth in SEQ ID NO: 30 or a heavy chain variable region sequence having at least 90% sequence identity therewith, and a sequence as set forth in SEQ ID NO: 29 or a light chain variable region sequence having at least 90% sequence identity therewith;
(2) a sequence as set forth in SEQ ID NO: 36 or a heavy chain variable region sequence having at least 90% sequence identity therewith, and a sequence as set forth in SEQ ID NO: 35 or a light chain variable region sequence having at least 90% sequence identity therewith;
(3) a sequence as set forth in SEQ ID NO: 42 or a heavy chain variable region sequence having at least 90% sequence identity therewith, and a sequence as set forth in SEQ ID NO: 41 or a light chain variable region sequence having at least 90% sequence identity therewith;
(4) a sequence as set forth in SEQ ID NO: 90 or a heavy chain variable region sequence having at least 90% sequence identity therewith, and a sequence as set forth in SEQ ID NO: 89 or a light chain variable region sequence having at least 90% sequence identity therewith;
(5) a sequence as set forth in SEQ ID NO: 93 or a heavy chain variable region sequence having at least 90% sequence identity therewith, and a sequence as set forth in SEQ ID NO: 92 or a light chain variable region sequence having at least 90% sequence identity therewith;
(6) a sequence as set forth in SEQ ID NO: 96 or a heavy chain variable region sequence having at least 90% sequence identity therewith, and a sequence as set forth in SEQ ID NO: 95 or a light chain variable region sequence having at least 90% sequence identity therewith.

39. The fully human antibody or single-chain antibody or fragment thereof of claim 37 or 38, wherein the amino acid sequence of the heavy chain variable region and/or light chain variable region is selected from any one of the following combinations:
(1) a heavy chain variable region sequence as set forth in SEQ ID NO: 30, and a light chain variable region sequence as set forth in SEQ ID NO: 29;
(2) a heavy chain variable region sequence as set forth in SEQ ID NO: 36, and a light chain variable region sequence as set forth in SEQ ID NO: 35;
(3) a heavy chain variable region sequence as set forth in SEQ ID NO: 42, and a light chain variable region sequence as set forth in SEQ ID NO: 41;
(4) a heavy chain variable region sequence as set forth in SEQ ID NO: 90, and a light chain variable region sequence as set forth in SEQ ID NO: 89;
(5) a heavy chain variable region sequence as set forth in SEQ ID NO: 93, and a light chain variable region sequence as set forth in SEQ ID NO: 92;
(6) a heavy chain variable region sequence as set forth in SEQ ID NO: 96, and a light chain variable region sequence as set forth in SEQ ID NO: 95.

40. The fully human antibody or single chain antibody or fragment thereof of any one of claims 37-39, wherein the fully human antibody comprises an amino acid sequence as set forth in SEQ ID NO: 28, 34, 40, 88, 91 or 94.

41. A nucleic acid molecule encoding the fully human antibody or single chain antibody or fragment thereof of any one of claims 37-40.

42. The nucleic acid molecule of claim 41, comprising a nucleotide sequence as set forth in any one of SEQ ID NOs: 25-27, 31-33, 37-39, and 79-87.

43. An expression vector comprising the nucleic acid molecule of claim 41 or 42.

44. A host cell comprising the expression vector of claim 43.

45. A pharmaceutical composition comprising the fully human antibody or single chain antibody or fragment thereof of any one of claims 37-40, and a pharmaceutically acceptable carrier or diluent.

46. A method of treating a disease or condition, comprising administering a therapeutically effective amount of the fully human antibody or single chain antibody or fragment thereof of any one of claims 37-40, the host cell of claim 44 or the pharmaceutical composition of claim 45 to a patient in need thereof to eliminate, inhibit or reduce CD5 activity, thereby preventing, alleviating, improving or inhibiting the disease or condition.

47. The method of claim 46, wherein the disease or condition is a cancer.

48. The method of claim 47, wherein the cancer is a T-cell malignancy.

49. An antibody or a fragment competing for the same epitope with the fully human antibody or single chain antibody or fragment thereof of any one of claims 37-40.

50. A kit for detecting CD5 protein in a sample, wherein the kit includes the fully human antibody or single-chain antibody or fragment thereof of any one of claims 37-40.

51. Use of the fully human antibody or single chain antibody or fragment thereof of any one of claims 37-40 or the host cell of claim 44 in the preparation of a drug for eliminating, inhibiting or reducing CD5 activity, thereby preventing, alleviating, improving or inhibiting a disease or condition.

52. The use of claim 51, wherein the disease or condition is a cancer.

53. The use of claim 52, wherein the cancer is a T-cell malignancy or a B-cell malignancy.

54. The method of claim 53, wherein the T-cell malignancy is selected from T-cell acute lymphocytic leukemia (T-ALL) and T-cell lymphoma (TCL), and the B-cell malignancy is selected from chronic lymphocytic leukemia (B-CLL) and mantle cell lymphoma (B-MCL).
